# EUROPEAN PATENT SPECIFICATION

(11) **EP 1 140 027 B1**
(45) Date of publication and mention of the grant of the patent: **12.10.2005**
(21) Application number: 99962459.6
(22) Date of filing: 23.12.1999
(51) Int. Cl.: A61K 9/16, A61K 9/26, A61K 9/48, A61K 9/52

(54) **DOSAGE FORMS COMPRISING POROUS PARTICLES**
DOSIERUNGSFORMEN, DIE PORÖSE PARTIKEL ENTHALTEN
FORMES POSOLOGIQUES COMPRENANT DES PARTICULES POREUSES

(30) Priority: 23.12.1998 US 113559 P; 23.12.1998 US 113615 P; 23.12.1998 US 113750 P
(43) Date of publication of application: 10.10.2001
(73) Proprietor: ALZA CORPORATION, Mountain View, CA 94039-7210 (US)
(72) Inventor: WONG, Patrick, Burlingame, CA 94010 (US); EDGREN, David, El Granada, CA 94018 (US); DONG, Liang-Chang, Sunnyvale, CA 94086 (US); POLLOCK-DOVE, Crystal, 15 Mountain View, CA 94043 (US)
(74) Representative: Allan, James Stewart
(86) International application number: PCT/GB1999/004426
(87) International publication number: WO 2000/038655

(56) References cited:
- EP-A- 0 163 178
- EP-A- 0 448 091
- EP-A- 0 985 411
- WO-A-98/38987
- GB-A- 2 155 889
- US-A- 5 486 365
- US-A- 5 800 834
- US-A- 5 955 086

## Description

The present invention relates to various dosage forms for the delivery of liquid active pharmaceutical agents using a porous carrier which is compactable, but which still retains substantially all the liquid agent in a liquid form. The present invention is based on the use of certain absorbent materials having prescribed physical characteristics, and useable for various different types of dosage forms.

From one view, it is desired to have dosage forms that permit rapid release of liquid, active agent formulations to facilitate absorption of the active agent by the gastrointestinal tract and to minimize delay in the onset of the intended beneficial effect of the active agent.

Delay of onset of the beneficial effect of an active agent orally administered to a subject may be attributed, inter alia, to three basic factors: firstly, the time that it takes for the active agent to come into contact with the fluid environment in which the active agent is to be utilized; secondly, the time for the active agent to dissolve in the fluid environment; and thirdly, the time for the active agent to be absorbed from the gastrointestinal tract. For active agents that are soluble, all three of the above considerations may be addressed by the administration of a solution of the liquid, active agent formulation. Typically, solutions of liquid, active agent formulations for pharmaceutical applications are administered either from a bulk solution with the aid of a device, e.g., spoon, volumetric measuring thimble, or the like, that provides the desired dose of the active agent to the subject, or in liquid-filled gelatin capsules which provide a pre-determined dose of active agent in each capsule. Dispensing from a bulk solution is not always satisfactory because of the difficulty of accurately measuring the dose of active agent to be administered. While that usually is not the case with capsules, filling of capsules is often expensive and the onset of the beneficial effect of the active agent may be delayed for an undesirable length of time to allow for the capsule wall to dissolve and release the liquid, active agent formulation to the environment of use.

When the active agent is insoluble or poorly soluble, the second and third considerations may be particularly troublesome. Various approaches have been put forth to address such problems, including the use of water-soluble salts, self-emulsifying compositions, polymorphic forms, powdered solutions, molecular complexes, micronization, eutectics, and solid solutions.

The prior art systems are primarily based on incorporating drug liquid in polymeric powders, which polymeric powders, such as cellulose, are insoluble in aqueous media. The insoluble polymeric powders, while serving to carry the drug liquid, can themselves impede the rapid and complete delivery of the drug to the environment of use. A critical need therefore exists for dosage forms having a liquid drug carrier that is soluble in the environment of use.

US Patent No. 5,846,365, describes a spheronized material formed from a scale-like calcium hydrogen phosphate particulate material having a high specific surface area, good compressibility and low friability. That patent indicates that the material has the characteristic of high liquid absorption. However, the patent does not suggest that the material may be used as a carrier for a liquid medicament formulation without forming a dried, final dosage form, as for example by spray drying. The patent describes the use of a suspension containing medicines and binders during the spray-drying granulation process to form a spherical particle containing the medicine. As an example, ascorbic acid in an amount equivalent to 10% of the scale-like calcium hydrogen phosphate was dissolved into a slurry of 20 weight percent of calcium hydrogen phosphate in water, and the resulting slurry was spray dried to form dried, spherical calcium hydrogen phosphate containing ascorbic acid. That material was then tableted under loads of 500-2000 kgf/cm². The calcium hydrogen phosphate is a non-polymeric material which dissolves in the low pH environment of the stomach.

It has been surprisingly discovered that certain absorbent materials having prescribed physical characteristics, as exemplified by, for example, by particular porous calcium hydrogen phosphate powders described in US Patent 5,486,365, sold under the trademark FujiCalin® and magnesium aluminometasilicate sold under the Trade Mark Neusilin may be used to prepare dosage forms in which liquid, active agent formulations may be adsorbed into the interior pores of the aforementioned materials in significant amounts and delivered to the site of administration in the liquid state. Wet granulated materials may be suitably tableted by conventional methods without removal of the solvent or liquid carrier of the active agent prior to tableting and without deleterious exudation of the liquid, active agent formulation during tableting. The completed dosage forms permit the delivery of the active agent to the delivery site in the liquid state, thus providing minimal delay in the onset of the desired beneficial effect of the active agent, since the active agent does not have to be initially dissolved or dispersed in the form of microparticles at the site of action. Unlike powder carriers of the prior art which are organic and which are insoluble in aqueous media, the calcium hydrogen phosphate carrier is an inorganic carrier that is soluble in gastric fluids. Certain other particulates or powders, for example, magnesium aluminometasilicate powders, sold under the trademark Neusilin™, may also be utilized, as may blends of the calcium hydrogen phosphate particles and the magnesium aluminometasilicate powders.

From another view, it is desired to have a dosage form being adapted to be retained in the stomach for a prolonged period of time, during which a liquid, active agent formulation is released to the environment of use.

Controlled release dosage forms that provide for prolonged delivery of active agent formulations to the environment of use have found application for increasing numbers of active agents. However, it has generally been a problem to deliver liquid, active agent formulations to the stomach of a subject continuously or intermittently over a prolonged period of time. Particularly when the active agent is absorbed only in the upper gastrointestinal tract, the bioavailability of the active agent may be greatly reduced if it is rapidly released from the stomach and passes quickly through the upper gastrointestinal tract. The period of time for absorption may be too short for an effective amount of active agent to be absorbed over a reasonable period of time, without frequent, subsequent dosing. This is particularly a problem with liquid forms of active agents, since they tend not to be retained within the stomach for more than a short period of time. Instead they tend to pass quickly from the stomach, through the upper gastrointestinal tract and into the lower gastrointestinal tract.

Generally, the time of passage of different particles through the small intestine does not vary significantly, and passage is generally independent of food intake and particle size. Thus, active agent dissolved in liquid, solid active agent dispersed in liquid and relatively larger delivery units of active agent, such as microcapsules and the like, will traverse the length of the small intestine in substantially the same time frame, usually about 3-5 hours. However, if liquid active agents can be retained in the stomach and released over a prolonged period of time, the active agent can be delivered to the small intestine over a time much longer than the 3-5 hour window, increasing the likelihood of increased absorption.

Most active agents are not well absorbed in the stomach, but even in those instances where the active agent is not well absorbed, the continuous release of active agent in the stomach over a prolonged time period will dispense active agent over that same period of time to the small intestine where it can be absorbed.

The physiological behavior of the stomach is usually determined by whether it contains food or is empty. Food is mixed and partially digested in the distal stomach (antrum). As the stomach undergoes contractions, partially digested material is discharged into the small intestine and non-digested material is retropelled into the main part of the stomach for further digestion. In the fed state, non-digestible material is not generally able to leave the stomach. At the end of a digestive period, the stomach enters the fasting stage and begins a cycle called the interdigestive myoelectric motor cycle or IMMC.

The IMMC can be considered to be divided into four phases: (1) phase 1 is an approximately one hour period with no contractions; (2) phase 2 is about a forty minute period of intermittent potentials and contractions that increase in intensity over time; (3) phase 3 is a relatively short period, generally between about five to fifteen minutes, of intense contractions (commonly called the "housekeeper wave") that completely empties the stomach; and (4) phase 4 is a short transitory period between the intense activity of phase 3 and the quiescence of phase 1. The different phases move distally from the stomach to the terminal ileum over an approximately two hour period as the cycle is repeated. Since the cycle is interrupted by the receipt of food by the stomach, it is possible to delay the emptying phase, phase 3, by maintaining a fed state. However, it is not practical to regularly maintain the fed state over a long period of time. Consequently, a need exists for a delivery device that can remain in the stomach for a significant period, whether in the fed or fasted state, and deliver active agent to the stomach over a prolonged period of time.

A variety of studies have been conducted in dog and in man to determine sizes of objects that would be retained in the stomach during the fed stage and also in the fasting stage when IMMC is present. Khosla and Davis, International Journal of Pharmaceutics, Vol. 62 (1990), pages R9-R11 have reported that a particle size less that 2 mm generally results in emptying from the stomach of the dog. Non-disintegrating tablets having sizes of 7, 11 and 13 mm in diameter were emptied from the human stomach, but the larger sized tablets tended to remain in the stomach longer than the small sized tablets. Tablets larger than 11 mm tended to be emptied only during the IMMC. Davis et al., Pharmaceutical Research , Vol. 8, No. 10 (1991) has described retention of radio-telemetry capsules having a size of 25 x 8 mm in the stomach of human subjects past phase 3 of the IMMC. Timmermans et al., Journal of Pharmaceutical Sciences, Vol. 82, No. 8 (1993) has reported the mean resting pyloric diameter in humans as 12.8 ± 7.0 mm. Accordingly, it is important that gastric retentive delivery vehicles are adapted to disintegrate, dissolve or erode to sizes that permit eventual elimination of the vehicle without causing gastric obstruction.

The influence of food on gastric retention time and the absorption of acyclovir has been reported in International Journal of Pharmaceutics, Vol. 38 (1987), pages 221-225. As reported there, compared to a lighter meal, the heavier meal slowed the rate of gastric emptying, prolonged small intestinal transit time and decreased absorption of the active agent.

The use of albumin-cross-linked polyvinylpyrrolidone hydrogels to deliver flavin mononucleotide to dogs has been described by Park et al. in Journal of Controlled Release, Vol.19 (1992) pages 131-134. The hydrogels were maintained in the stomachs of dogs for extended periods, even in the fasted state. Gels with a glassy core tended to remain in the stomach longer than hydrogels without the glassy core. Control of the size of the core was attempted by administration of water in the stomach. While it is possible to control the dimensions of the hydrogel in the dry state, controlling the size of the glassy core within the hydrogel after administration to a subject by addition of water is not suitable for fabrication of a dosage form that can routinely and controllably be retained in the stomach of a subject over a prolonged period of time.

For many applications it may be important that the delivery device is adapted to remain in the stomach for a prolonged period. For certain applications it may also be important that the device deliver active agent in a controlled manner. Delivery systems, such as those described below, are representative of the many different systems have been suggested for such controlled delivery of active agents over a prolonged period of time.

U.S. Patent No. 5,534,263, describes a dosage form useful for the prolonged delivery of an active agent formulation in the form of a matrix having two or more insoluble bands on the surface of the matrix. The exposed surfaces of the matrix erode in a manner that creates additional surface areas to provide for prolonged release of an active agent formulation with determined release profiles.

Generally the previous systems have been directed to the delivery of active agents which are in the dosage forms initially in the dry state. Little effort appears to have been made to deliver liquid active agent formulations that would be retained in the stomach for a sustained period of time. Administration of acyclovir by sipped solution over a four-hour period has been described in Br. J. clin. Pharmac., 21, 459-462 (1986) to achieve an increased contact time with the human stomach and the gastrointestinal tract. The total amount of acyclovir absorbed was increased over that observed with administration of acyclovir tablets. However, no attempt was made to maintain the acyclovir solution in the stomach for a sustained period except by continuous oral administration.

Furthermore, when the active agent is insoluble or poorly soluble, prior art systems may not provide suitable delivery of active agent or concentration gradients at the site of absorption for that period of time that the active agent sees the absorption site. Various attempts have been made to address such problems, including the use of water-soluble salts, self-emulsifying compositions, polymorphic forms, powdered solutions, molecular complexes, micronization, eutectics, and solid solutions, in the context of immediate release delivery. An example of the use of a powdered solution is described by Sheth, et al., in "Use of Powdered Solutions to Improve the Dissolution Rate of Polythiazide Tablets," Drug Development and Industrial Pharmacy, 16(5), 769-777 (1990). References to certain of the other approaches are cited therein. Additional examples of powdered solutions are described in US Patent 5,800,834. The patent describes methodology for calculating the amount of liquid that may be optimally sorbed into materials to prevent the drug solution from being exuded from the granular composition during compression.

As can be observed in the above-referenced patents and publications, devices have been described that provide for prolonged delivery of an active agent and retention in the gastric environment. However, there remains a continuing need for improved systems for delivering a liquid, active agent formulation to the gastric environment over a prolonged period of time and in a reliable, controllable and reproducible manner. In particular, there is a need for controlled release delivery devices that are to remain in the stomach, even during a fasting state in which IMMC is present, for a prolonged period, for example from about 3 hours to up to about 20-24 hours, and deliver a liquid, active agent formulation. Such devices should exhibit a combination of flexibility and rigidity so as not to be expelled from the stomach into the pyloric sphincter under fed or fasting conditions, and deliver active agent in a reproducible, controlled manner, over a prolonged period of time.

From another view, it is desired to have improved methods, dosage forms and devices for the controlled delivery of liquid active agent formulations to an environment of use.

Administration of liquid, active agent formulations is often preferred over solid active agent formulations in order to facilitate absorption of the active agent and obtain a beneficial effect for the intended use in the shortest possible time after the formulation is exposed to the environment of use. Examples of prior art devices to deliver liquid, active agent formulations are soft gelatin capsules that contain a liquid active agent formulation or liquid formulations of the active agent that are bottled and dispensed in measured dosage amounts by the spoonful, or the like. Those systems are not generally amenable to controlled delivery of the active agent over time. While it is desired to have the active agent exhibit its effect as soon as it is released to the environment of use, it also often is desirable to have controlled release of the active agent to the environment of use over time. Such controlled release may be sustained delivery over time, such as zero order, or patterned delivery, such as pulsatile for example. Prior art systems have not generally been suitable for such delivery.

Various devices and methods have been described for the continuous delivery of active agents over time.

Typically, such prior art systems have been used to deliver active agents initially in the dry state prior to administration. For example, US Patent Nos. 4,892,778 and 4,940,465, describe dispensers for delivering a beneficial agent to an environment of use that include a semipermeable wall defining a compartment containing a layer of expandable material that pushes a drug layer out of the compartment formed by the wall. The exit orifice in the device is substantially the same diameter as the inner diameter of the compartment formed by the wall.

US Patent No. 4,915,949, describes a dispenser for delivering a beneficial agent to an environment of use that includes a semipermeable wall containing a layer of expandable material that pushes a drug layer out of the compartment formed by the wall. The drug layer contains discrete tiny pills dispersed in a carrier. The exit orifice in the device is substantially the same diameter as the inner diameter of the compartment formed by the wall.

US Patent No. 5,126,142, describes a device for delivering an ionophore to livestock that includes a semipermeable housing in which a composition containing the ionophore and a carrier and an expandable hydrophilic layer is located, along with an additional element that imparts sufficient density to the device to retain it in the rumen-reticular sac of a ruminant animal. The ionophore and carrier are present in a dry state during storage and the composition changes to a dispensable, fluid-like state when it is in contact with the fluid environment of use. A number of different exit arrangements are described, including a plurality of holes in the end of the device and a single exit of varying diameter to control the amount of drug released per unit time due to diffusion and osmotic pumping.

It is often preferable that a large orifice, from about 50%-100% of the inner diameter of the drug compartment, be provided in the dispensing device containing the active agent and a bioerodible or degradable active agent carrier. When exposed to the environment of use, drug is released from the drug layer by erosion and diffusion. In those cases where the drug is present in the solid state, the realization of the beneficial effect is delayed until the drug is dissolved in the fluids of the environment of use and absorbed by the tissues or mucosal environment of the gastrointestinal tract. Such delay often is not tolerable. Also, for drugs that are poorly soluble in gastric or intestinal fluids, the delay may be further exacerbated.

Devices in which the drug composition initially is dry but in the environment of use is delivered as a slurry, suspension or solution from a small exit orifice by the action of an expandable layer are described in U. S. Patents Nos. 5,660,861, 5,633,011; 5,190,765; 5,252,338; 5,620,705; 4,931,285; 5,006,346; 5,024,842; and 5,160,743. Typical devices include an expandable push layer and a drug layer surrounded by a semipermeable membrane. In certain instances, the drug layer is provided with a subcoat to protect the drug composition in those portions of the gastrointestinal tract having acidic pH, to delay release of the drug composition to the environment of use or to form an annealed coating in conjunction with the semipermeable membrane. However, such devices often are not well suited for the delivery of active agents that demonstrate instability over time in the fluids with which they come in contact in the environment of use. Attempts have been made to protect the active agent from the environment of use by stabilizing agents, enteric coatings and the like. However, stabilization methods and coatings may delay the absorption of the active agent with concomitant delay in realized beneficial effect. Also, such systems may not generally be amenable to controlled delivery of active agent in the liquid state.

Furthermore, when the active agent is insoluble or poorly soluble, prior art systems may not provide rapid delivery of active agent or concentration gradients at the site of absorption that facilitate absorption through the gastrointestinal tract. Various approaches have been put forth to address such problems, including the use of water-soluble salts, self-emulsifying compositions, polymorphic forms, powdered solutions, molecular complexes, micronization, eutectics, and solid solutions. An example of the use of a powdered solution is described by Sheth, et al., in "Use of Powdered Solutions to Improve the Dissolution Rate of Polythiazide Tablets," Drug Development and Industrial Pharmacy, 16(5), 769-777 (1990). References to certain of the other approaches are cited therein. Additional examples of powdered solutions are described in US Patent 5,800,834. The patent describes methodology for calculating the amount of liquid that may be optimally sorbed into materials to prevent the drug solution from being exuded from the granular composition during compression.

It has been surprisingly discovered that certain absorbent materials having prescribed physical characteristics, as exemplified by, for example, particular porous calcium hydrogen phosphate powders described in US Patent 5,486,365, already discussed herein, and sold under the trademark FujiCalin®, may be used to prepare dosage forms in which liquid, active agent formulations may be adsorbed into the interior pores of the aforementioned materials in significant amounts and delivered to the site of administration in the liquid state. It has further been surprisingly discovered that such types of porous particles with liquid, active agent formulations sorbed into the particles may be fabricated into controlled release dosage forms without exuding the liquid, active agent formulation out of the particles during the manufacturing process. That discovery has permitted the fabrication of controlled release dosage forms that provided for the delivery of the active agent to the delivery site in the liquid state, thus providing minimal delay in the onset of the desired beneficial effect of the active agent, since the active agent does not have to be initially dissolved or dispersed in the form of microparticles at the site of action. Furthermore, such dosage forms permit the delivery of high concentrations of active agent, and optionally absorption enhancers, to the absorption site. Other particles having the characteristics of the calcium hydrogen phosphate as described herein and sold under the trademarks of FujiCalin, such as, for example, magnesium aluminometasilicate powders, sold under the trademark Neusilin™, may also be utilized.

According to one aspect of the present invention, there is provided a dosage form as described in claim 1. The drug layer comprise a plurality of particles having interior pores and a liquid, active agent formulation in the pores, the particles being compactable and adapted to retain substantially all of the liquid active agent formulation within the pores during the compacting process.

Preferably, the particles are formed from calcium hydrogen phosphate, microcrystaline cellulose, silicon dioxide, or magnesium aluminosilicate, or blends thereof.

In one embodiment of the present invention, the particles formed from calcium hydrogen phosphate may have the following general formula

CaHPO₄•mH₂O

wherein m satisfies the relationship 0≦m≦2.0.

In one form, the particles are formed by spray drying a scale-like calcium hydrogen phosphate with a specific surface area of 20 m²/g to 60 m²/g, an apparent specific volume of 1.5 ml/g or more, an oil absorption capacity of 0.7 ml/g or more, a primary particle size of 0.1µ to 5µ, and an average particle size of 2µ to 10µ among secondary particles that are aggregates of the primary particles.

In another form, the particles are calcium hydrogen phosphate having a specific volume of at least 1.5 ml/g, a BET specific area of at least 20 m²/g, and a water absorption capacity of at least 0.7 ml/g.

In another form, the particles have a bulk density of 0.4-0.6 g/ml, a BET surface area of 30-50 m²/g, a specific volume of greater than 1.5 ml/g, and a mean pore size of at least 50 Angstroms.

In another form, the particles are calcium hydrogen phosphate having a bulk specific volume of 1.5 ml/g-5 ml/g, a BET specific area of 20 m²/g-60 m²/g, a water absorption capacity of at least 0.7 ml/g, and a mean particle size of at least 70 microns.

Preferably, the porous particles have a size distribution of 100% less than 40 mesh, 50%-100% less than 100 mesh and 10%-60% less than 200 mesh, more preferably 60%-90% are less than 100 mesh and 20%-60% are less than 200 mesh.

In another embodiment of the present invention, the particles are magnesium aluminometasilicate represented by the general formula

Al₂O₃MgO•2SiO₂•nH₂O

wherein n satisfies the relationship 0≤n≤10. The particles may comprise magnesium aluminometasilicate powder.

The dosage form may include a pH regulating agent selected from one or more of the group comprising organic acids, inorganic acids and bases, and/or include a chelating agent. In particular, it has been found that the use of organic acid(s) and/or chelating agent(s) facilitate the dissolution of FujiCalin particles upon contact with gastric acid.

Preferably, the weight percent of a liquid, active agent formulation is at least 5% of the total weight of the dosage form.

These absorbent materials have been found to be useful for various types of dosage forms.

In one embodiment of the present invention, the dosage form is adapted for rapid, possibly immediate, release of the active agent. The active agent for this could be selected from active agents that have low water solubility, such as for example sildenafil citrate, acetaminophen, ibuprofen or ketoprofen.

In the capsule form, the particles are preferably to bind themselves in a dosage form such a gelatin capsule or a tablet. The particles could be dispersed in a liquid to form a paste adapted for loading into a gelatin capsule, and be calcium hydrogen phosphate having a specific volume of at least 1.5 ml/g, a BET specific area of at least 20 m²/g, and a water absorption capacity of at least 0.7 ml/g. The liquid forming the paste with the particles could be the same liquid as the liquid of the liquid, active agent formulation.

The completed dosage forms permit the delivery of the active agent to the site of action in the liquid state, thus providing minimal delay in the onset of the desired beneficial effect of the active agent since the active agent does not have to be initially dissolved or dispersed in the form of microparticles at the site of action. Certain magnesium aluminometasilicate powders, sold under the trademark Neusilin™, or blends of FujiCalin and Neusilin, may also be utilized to afford dosage forms of the present invention.

In a second embodiment of the present invention, the dosage form is such that the porous particles can be dispersed in a bioerodible carrier. The bioerodible carrier preferably swells upon imbibing fluid from stomach so as to be retained within the stomach of a subject for a prolonged period of time.

The bioerodible carrier preferably comprises a polymer matrix formed of a mixture of a swellable, water soluble polymer that expands when in contact with fluids in the gastric environment and a hydroattractant.

The matrix could be formed with a rigid or semi-rigid segment in which swelling of the matrix is constrained to provide a rigid or semi-rigid section in the dosage form that facilitates the dosage form remaining in the stomach of a subject over a prolonged period of time. The rigid or semi-rigid section of the dosage form preferably comprises one or more insoluble materials, having low water permeability and formed as a band circumscribing a portion of the surface of the matrix, that along with the banded portion of the polymer matrix forms the rigid or semi-rigid segment of the dosage form.

In one form, the dosage form comprises (a) a therapeutically-effective amount of a liquid, active agent formulation sorbed into porous particles, (b) a polymer matrix in which the porous particles are dispersed, the polymer matrix including a high molecular weight, water-soluble polymer and a hydroattractant, the polymer matrix having an outer surface for exposure to the environment of use, and (c) a band of insoluble material circumscribing a portion of the outer surface of the polymer matrix.

The hydroattractant is preferably a water-insoluble polymer, and the polymer matrix could further include non-polymeric water-soluble excipients and polymers of molecular weight of less than 10,000 grams per mole. The weight percent of the water soluble, high molecular weight polymer could be about 10 to 50 weight percent and the weight percent of the hydroattractant could be about 5 to 70 weight percent.

In another form, the dosage form of this embodiment comprises a unitary compressed dispersion of a liquid, active agent formulation in a plurality of porous particles in a gel-forming, erodible polymer matrix having a first portion that swells in the stomach while maintaining its physical integrity for a prolonged period of time and a second, non-erodible, non-gel-forming portion for promoting retention of the dosage form in the stomach over a prolonged period of time.

In general, the number average molecular weight of the water-soluble polymer can be between about 100,000 and 20,000,000 grams per mole, such as for example one or more of the group comprising polyethylene oxide, hydroxypropyl cellulose, hydroxypropyl methyl cellulose, hydroxyethyl cellulose, sodium carboxyl methylcellulose, calcium carboxymethyl cellulose, methyl cellulose, polyacrylic acid, maltodextrin, pre-gelatinised starch or polyvinyl alcohol.

The hydroattractant is preferably one or more of the group comprising low-substituted hydroxypropyl cellulose, microcrystalline cellulose, cross-linked sodium or calcium carboxymethyl cellulose, cellulose fiber, cross-linked polyvinyl pyrrolidone, cross-linked polyacrylic acid, cross-linked Amberlite resin, alginates, colloidal magnesium-aluminum silicate, corn starch granules, rice starch granules, potato starch granules or sodium carboxymethyl starch.

This form of dosage form is adapted for gastric retention, and could be used wherein the active agent is one or more of the group comprising an antiviral, antimicrobial, antidiabetic, antihperglycemic, hypoglycemic, antidepressant, antiobesity, immunosuppresive, antiidiabetic or antifungal active agent, such as for example acyclovir, ganciclovir, cimetidine, ranitidine, captopril, methyldopa, selegiline, minocycline, metformin, bupropion, orlistat, cyclosporin, cylcosporine metasporin or fexofenadine or a pharmaceutically acceptable salt thereof.

This dosage form could also release the active agent from the porous particles in a liquid formulation to the gastrointestinal tract over a time period of at least 3 hours, and/or act a gastric-emptying delaying agent. Suitable gastric-emptying delaying agents include anticholonergic agents, methylcellulose, guar gum, fats such as triglyceride esters, and fatty acids of 10-15 carbon atoms.

This embodiment of the present invention provides a dosage form that is retained in the stomach for a prolonged period of time and that is useful for the prolonged delivery of a liquid, active agent formulation to a fluid environment of use. Certain embodiments of the invention provide for initial and substantially complete delivery of a liquid, active agent formulation in the stomach of a user, where the active agent may be absorbed or released from the stomach to be absorbed in the gastrointestinal tract.

In particular applications the gastric retentive dosage forms of the invention may allow for less frequent dosing of the active agent than with immediate release formulations or sustained release formulations that are not gastric retentive dosage forms. In other applications the frequency of dosing may be the same, but the gastric retentive dosage forms will beneficially alter the absorption profile of the active agent from that available with immediate release formulations. This may result in increased bioavailability of the active agent or reduced side effects, for example.

Microcrystalline cellulose and silicon dioxide having high surface area and good absorption properties may especially be used in these dosage forms.

For this embodiment of the present invention, the following definitions are used.

The phrase "prolonged period" or "prolonged period of time" intends a time period that lasts for several hours to about 24 hours, usually up to about 12 hours, and often between about 3 and 14 hours, and most often at least 6 hours.

The phrase "prolonged delivery" intends a duration of delivery extending over a time period that lasts for several hours to about 24 hours, usually up to about 12 hours, and often between about 3 and 14 hours, and most often at least 6 hours.

By "insoluble" is intended a material that will not substantially dissolve in the environment of use during the delivery period.

The term "active agent" refers to an agent, drug, compound or other substance, or compositions and mixtures thereof, that provide some pharmacologic, often beneficial, effect. Reference to a specific active agent shall include where appropriate the active agent and its pharmaceutically acceptable salts and may include mixtures of active agents.

The term "polymer matrix" as used herein means a mixture of a water soluble, high molecular weight polymer and a hydroattractant.

The term "liquid, active agent formulation" intends a solution, suspension or dispersion of the active agent or the active agent optionally in combination with pharmaceutically acceptable carriers and additional inert ingredients, in a liquid.

The terms "adapted for gastric retention" or "gastric retentive" mean, with respect to the dosage form of this invention, that the dosage form will remain in the stomach of a subject for a prolonged period of time.

The terms "rigid" and "semi-rigid" mean, with respect to a portion of the active agent formulation matrix or polymer matrix as defined above, that such portion will not swell and form a gel when initially contacted with gastric fluid.

The term "bioerodible" intends a material that will, at least in part, dissolve, degrade or erode in the fluid environment of use.

The term "bioequivalent" intends, with respect to an active agent dosage form of this invention, that there is greater than a 90% probability that the bioavailability of the active agent as determined by standard methods is 80-125% of the defined dosage form and that there is greater than a 90% probability that the maximum blood plasma concentration and the minimum blood plasma concentration of the active agent as measured by standard methods is 80-125% of the defined dosage form.

The term "polymer" means a material formed from a single polymer or a mixture of polymers.

The term "swellable" means, with respect to a polymer or a polymer matrix, that the polymer or polymer matrix is capable of imbibing fluid and expanding when in contact with fluid present in the environment of use.

The terms "therapeutically effective" amount or rate refer to the amount or rate of the active agent needed to effect the desired pharmacologic, often beneficial, result.

The dosage forms of this form of the invention find use, for example, in humans or other animals. The environment of use is a fluid environment and for the purposes of this invention primarily includes the fluid environment of the stomach and the upper intestinal tract or small intestine. A single dosage form or several dosage forms can be administered to a subject during a therapeutic program.

In a third embodiment of the present invention, there is a dosage form for sustained or pulsatle release of active agent. In general there is a dosage form for an active agent comprising a wall defining a cavity, the wall having an exit orifice formed or formable therein and at least a portion of the wall being semipermeable; an expandable layer located within the cavity remote from the exit orifice and in fluid communication with the semipermeable portion of the wall; a drug layer located within the cavity adjacent the exit orifice and in direct or indirect contacting relationship with the expandable layer, wherein the drug layer is a form defined by the dosage forms defined herein above

In certain embodiments, there is a placebo layer between the exit orifice and the drug layer, and/or a flow-promoting layer interposed between the inner surface of the wall and at least the external surface of the drug layer located within the cavity. There may be two drug layers separated by at least one inert layer, possibly with each of said drug layers containing a different active agent.

For this form of dosage form, the liquid, active agent formulation of the drug layer preferably comprises a self-emulsifying formulation, and has low water solubility. The liquid active agent of the drug layer may also comprise an absorption enhancer, and the liquid, active agent formulation preferably comprises at least 30% by weight of the drug layer.

This form of dosage form may be adapted for sustained or pulsatile release of the liquid, active agent formulation upon administration to a subject. In general, the dosage forms of the present invention are in a unitary and oral dosage form.

The present invention also covers a composition comprising from about 1 to 50 weight percent of porous calcium hydrogen phosphate particles having sorbed therein a liquid, active agent formulation, about 5 weight percent to about 50 weight percent of a polyethylene oxide polymer having a number average molecular weight of between about 100,000 and 20,000,000 grams per mole and about 5 weight percent to about 60 weight percent of a hydroxypropyl cellulose polymer having a hydroxypropyl content of between about 10 weight percent and about 13 weight percent of the hydroxypropyl cellulose polymer the porous particles comprising calcium hydrogen phosphate with a specific surface area of 20 m²/g to 60 m²/g, an apparent specific volume of 1.5 ml/g or more, an oil absorption capacity of 0.7 ml/g or more, and a mean particle size of greater than 70 microns, the calcium hydrogen phosphate being represented by the following general formula:

CaHPO₄•mH₂O

wherein m satisfies the relationship 0≦m≦2.0, or a composition comprising a liquid formulation of the active agent sorbed into a plurality of porous particles, the particles being formed by spray drying a scale-like calcium hydrogen phosphate with a specific surface area of 20 m²/g to 60 m²/g, an apparent specific volume of 1.5 ml/g or more, an oil absorption capacity of 0.7 ml/g or more, a primary particle size of 0.1µ to 5µ, and an average particle size of 2µ to 10µ among secondary particles that are aggregates of the primary particles, the scale-like calcium hydrogen phosphate being represented by the following general formula:

CaHPO₄•mH₂O

wherein m satisfies the relationship 0≦m≦2.0, and dispersed throughout a bioerodible carrier, the particles being released in the environment of use over a prolonged period of time.

In further aspects of the present invention, there are provided the following methods;

A method of manufacturing a dosage form comprising contacting a plurality of particles having interior pores as defined hereinbefore with a liquid, active agent formulation, and compacting the particles into a dosage form without removing all of the liquid from the liquid, active agent formulation.

Preferably, the particles are spherical calcium hydrogen phosphate particles obtained by spray drying a scale-like calcium hydrogen phosphate or magnesium aluminometasilicate particles as hereinbefore defined.

Also preferably, less than 80% of the liquid of the active agent formulation is removed prior to the compacting step.

A method of facilitating the release of an active agent from a dosage form comprising sorbing a liquid formulation of the active agent into a plurality of porous particles, the particles being formed as defined hereinbefore, and dispersing the particles throughout a bioerodible carrier.

A method for facilitating the immediate release of an active agent from a dosage form containing a liquid, active agent formulation sorbed into a porous particle, wherein the dissolution rate of the porous particle is pH sensitive, comprising incorporating a pH regulating agent into the dosage form to bias the pH of the microenvironment of the porous particle after administration toward a pH increasing the rate of dissolution of the porous particle.

Preferably, the pH regulating agent is an organic acid, and inorganic acid or a base, more preferably the particle is a calcium hydrogen phosphate and the pH regulating agent is an organic acid.

Embodiments of the present invention will now be described by way of example only and with reference to the accompanying drawings in which:
Figure 1 is a dosage form according to one embodiment of the present invention.
Figures 2A, 2B and 2C illustrate a second embodiment of the delivery device of the present invention; the device in Figure 2A representing the active agent formulation matrix not including the insoluble material or band, the device in Figure 2B representing the banded device in prepared form prior to placement in the stomach; and Figure 2C illustrating a porous particle having liquid, active agent sorbed therein.
Figure 3 illustrates the device of Figure 2B in its initially-swollen state after having expanded in the stomach;
Figures 4A and 4B illustrate the device of Figure 3 at later stages where the device has eroded in the fluid environment of use;
Figures 5A-5D illustrate an embodiment of the invention having multiple, insoluble bands on the surface of the dosage form.
Figure 6 illustrates a dosage form of one design of third embodiment of the present invention adapted for zero order release of active agent;
Figure 7 illustrates a dosage form of another design of the third embodiment of this invention adapted to deliver a delayed pulse of the active agent;
Figure 8 illustrates the release profile (release rate as a function of time) of the active agent progesterone from a representative dosage form of the invention having zero order release;
Figures 9-13 illustrate the release profiles (percent of active agent released as a function of time) of the active agent progesterone for representative dosage forms of the invention having a delayed pulse release, wherein the initial delay is 2 hours, 3 hours, 4-5 hours, 6-7 hours and about 10 hours for the dosage forms described in Examples 12-16 respectively; and
Figures 14-17 illustrate various dissolution and release profiles relating to dosage forms described in Examples 17 and 18.

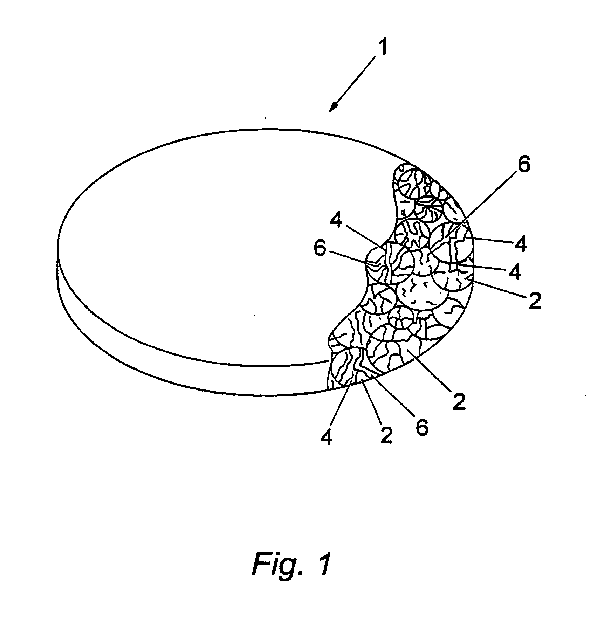

An embodiment of a rapid, possibly immediate release dosage form of the invention is illustrated in FIG. 1. In FIG. 1, dosage form 1 comprises a plurality of particles 2 having a plurality of interior and surface pores 4. Absorbed into the interior of pores 4 is a liquid, active agent formulation 6. Particles 2 are compacted to form a tableted, unitary dosage form 1 from which the active agent formulation 6 may be delivered to the site of action in the liquid form, thus avoiding delayed onset of activity of the active agent.

Materials useful as carriers for the liquid, active agent formulations for all forms of the present invention are porous particulates that are characterized by high compressibility or tensile strength to withstand compacting forces applied during compacting steps and minimize exudation of liquid, active agent formulation from the pores; particle flow characteristics that allow for the porous particles to be directly compacted without the use of a binder or with minimal use of a binder; low friability so as to preclude or minimize exudation of the liquid, active agent formulation from the particles during compacting steps; and high porosity so as to absorb an adequate of amount of a liquid, active agent formulation to provide an effective amount of active agent in a dosage form. The particles should be adapted to absorb an amount of liquid, active agent formulation such that a therapeutically effective amount of the active agent may be delivered in a unitary dosage form that is of a size that can be conveniently swallowed by a subject and, preferably provided in four or fewer tablets or capsules for ingestion at the same time. The porosity of the particles should be such that at least 5% of the liquid, active agent is sorbed into the pores of the particles. Typically, up to 70% by weight, and usually in the range 20-70%, more preferably 30-60%, and most preferably 40-60% of the liquid, active agent formulation, based on the weight of the particles, may be sorbed into the pores of the particles, while the particles exhibit sufficient strength at such degree of liquid, active agent loading so as not to significantly be crushed or pulverized by compacting forces to which the particles will be subjected during manufacturing operations. Preferably, the loading of the liquid, active agent formulation will be on the order of at least 30-40 weight percent when the particles are crystalline, such as calcium hydrogen phosphate. When the particles are amorphous, such as with magnesium aluminometasilicate, greater loading burdens may usually be achieved, e.g. up to 60% by weight. At high loadings, it may be advantageous to use blends of calcium hydrogen phosphate particles and the amorphous magnesium aluminometasilicate powders.

Preferred materials are those having a strength to resist compression forces of greater than 1500 kg/cm² without substantial exudation of the liquid, active agent formulation, and most preferably without the tablet hardness plateauing.

A particularly suitable carrier is exemplified by the particular form of calcium hydrogen phosphate described in U.S. Patent No. 5,486,365. As described therein, calcium hydrogen phosphate is prepared by a process yielding a scale-like calcium hydrogen phosphate that can be represented by the formula CaHPO₄•mH₂O wherein m satisfies the expression 0 ≤ m ≤ 2.0. The scale-like calcium hydrogen phosphate produced has characteristic physical properties that make it particularly suitable for use in the present invention. The scale-like material provides high specific surface area, high specific volume, high capacity for water and oil absorption, and the ability to readily form into spheres upon spray drying. The spherical particulates have excellent flow properties and permit direct compaction into tablets with minimal or no use of binders and without significant crushing or pulverizing of the particles during the compaction step.

The scale-like calcium hydrogen phosphate particles generally have a BET specific surface area of at least 20 m²/g, typically 20 m²/g - 60 m²/g, a specific volume of at least 1.5 ml/g, typically 2-5 ml/g or more, and an oil and water absorption capacity of at least 0.7 ml/g, typically 0.8-1.5 ml/g. When formed into spheres the spherical particulates may have a mean particle size of at least 70 microns, usually about 70-130 microns, and often about 90-120 microns. The particle size distribution may be 100% through 40 mesh, 50%-100% through 100 mesh, and 20%-60% through 200 mesh. The bulk density may be from about 0.4 g/ml-0.6 g/ml.

A most preferred form of calcium hydrogen phosphate is that sold under the trademark FujiCalin by Fuji Chemical Industries (U.S.A.) Inc., Englewood, New Jersey, in types SG and S. Typical parameters for that material include a mean pore size on the order of 70 Angstroms, a mean particle size of about 110 microns, a specific volume of about 2 ml/g, a BET specific surface area of about 30-40 m²/g, and an oil and water absorption capacity of about 0.8 ml/g. Type SG typically will have a particle size distribution of 100% through 40 mesh, 60% through 100 mesh and 20 through 200 mesh. Type S typically will have a particle size distribution of 100% through 40 mesh, 90% through 100 mesh and 60% through 200 mesh. Mixtures of the two types may be conveniently employed to provide particulates having physical characteristics that are suitable for various applications, as may be determined by those skilled in the art of pharmaceutical formulation, tableting and manufacturing.

The calcium hydrogen phosphate has low friability, demonstrating a tensile strength of up to about 130 Kg/cm² when subjected to compressive forces of up to 3000 Kg/cm². The hardness of the tableted material tends not to plateau at compression forces to that limit, while materials such as microcrystalline cellulose (Avicel PH 301), lactose, DI-TAB and Kyowa GS tend to plateau at or about 700-1500 Kgf/cm². The angle of repose for the preferred materials typically is on the order of 32-35 degrees.

Another material that may be utilized is that formed of magnesium aluminometasilicate which may be represented by the general formula

Al₂O₃MgO•2SiO₂•nH₂O

wherein n satisfies the relationship 0≦n≦10. Commercially available magnesium aluminometasilicates are sold as Grades S₁, SG₁, UFL₂, US₂, FH₁, FH₂, FL₁, FL₂, S₂, SG₂, NFL₂N, and NS₂N, under the trademark Neusilin™ by Fuji Chemical Industries (U.S.A.) Inc., Englewood, New Jersey. Especially preferred grades are S₁, SG₁, US₂ and UFL₂. The most preferred for many applications is grade US₂. Those materials, which are amorphous, typically have a specific surface area (arca) of about 100-300 m²/g, an oil absorption capacity of about 1.3-3.4 ml/g, a mean particle size of about 1-2 microns, an angle of repose about 25° -45°, a specific gravity of about 2 g/ml and a specific volume of about 2.1-12 ml/g.

Other materials having similar physical characteristics to the foregoing ranges for FujiCalin and Neusilin may be equivalently substituted for the foregoing, as may blends of the various materials described.

The liquid, active agent formulation may be in any form that can be dispensed from the inside of the pores 4 as the tablet disintegrates in the environment of use.
The formulation, for example, may be neat, liquid active agent, liquid active agent in a solution, suspension, emulsion or self-emulsifying composition, or the like, or a liposomal solution or solid formulation, or solid active agent in solution, suspension or slurry. Optionally other dosage-forming ingredients, such as an anti-oxidant, a suspending agent, a surface active agent, and the like may be present in the liquid, active agent formulation. The liquid, active agent formulation will be released in a form most suitable to provide active agent to the site of delivery in a state in which it may be rapidly absorbed in the environment of use to provide its beneficial action with minimum delay. An example of the usefulness of the rapid release form of the present invention is demonstrated by its use for the popular drug sildenafil citrate, sold under the trademark Viagra®. The marketed dosage form is indicated to provide maximum plasma concentrations in a subject at ½ to 3 hours after administration. More rapid onset of the beneficial effect of the active agent is desirable and may be provided with the dosage forms of this invention. That aspect of the inventions is advantageous, also, for the preparation of dosage forms that contain active agents which are poorly soluble in water, such as for example the pain relievers acetaminophen and non-steroidal antiinflammatory agents such as ketoprofen, ibuprofen and the like.

The tableted dosage form 1 may be manufactured in accordance with conventional methods, such as by tumbling the porous particles together with the liquid, active agent formulation or spraying of the liquid, active agent formulation onto the porous particles in a fluidized bed to sorb the liquid, active agent formulation into the porous particles, and then tableting or encapsulating the particles to form a unitary dosage form. Typically, the desired quantity of porous powder is directly contacted with the desired amount of liquid, active agent formulation and mixed in a blender, such as a V-blender or the like. The wet material may be granulated by passing it through sieves of suitable size, e.g., 40-80 mesh. To the extent that some of the liquid, active agent formulation remains on the outside of the granules, the addition of a quickly, dissolving absorbent material, such as sugars, for example, lactose, glucose, fructose, mannitol, maltose, and sorbitol, starches, for example, malodextrin, modified starches and the like, may be added in amounts of 0.1%-10% by weight to improve the ability of the granulated powder to flow without affecting the immediate release characteristics of the invention. Additional excipients can include 0.5-10% by weight of tablet disintegration aids to promote rapid dissintegration of the tablet in the fluid environment of use. Disintegration aids include cross-linked polyvinyl pyrrolidone, starch granules, purified cellulose, alginates, chemically-modified starch such as sodium starch glycolate, cross-linked sodium carboxy methyl cellulose, bentonite, and ion exchange resins. The granulated material may then by compacted in a conventional tableting press, at pressures of 500-3000 kgf/cm². The tablets may be provided in capsule sizes (000), (00), (0), (1), (2), (3), (4), and (5), or other non-conventional sizes as appropriate. The largest number represents the smallest size. The tablets may also be manufactured in various shapes such as round, triangular, oval, square, and the like. Optionally, the tablets can be compressed with score marks providing the option of dividing the unit dose into subunits prior to administration. Tableting methods and equipment are well known in the art, such as described, for example, in Remington's Pharmaceutical Sciences, Eighteenth Edition (1990), Mack Publishing Company, Easton, Pennsylvania. Tablets may be coated with a film coat in conventional manner to provide a smooth surface and also mask objectional flavors that certain active agents may exhibit.

Also, the liquid, active agent formulation absorbed into the particles may by filled into gelatin capsules in the form of a paste or semi-compressed composition (such as by screw filling wherein the pitch of the screw changes to compress the composition as it moves to the capsule feeder) having a hardness less than that of the tableted dosage form to eliminate the compression, tableting step. The gelatin capsule may be made conveniently in two parts, with one part (the "cap") slipping over and capping the other part (the "body"). The two parts completely surround and capsulate the internal lumen that contains the liquid, active agent formulation, which may contain, as described above, useful additives. The two parts are fitted together after the body is filled with a preselected formulation. The assembly is done by slipping or telescoping the cap section over the body section, and optionally sealing the cap and body. Since it may take some time for the gelatin capsule wall to dissolve, the time for delivery of the active agent to the environment of use may be delayed somewhat as compared to the tableted dosage form. However, once the capsule has been at least partially dissolved, the liquid, active agent formulation will begin to be delivered to the environment of use in the liquid state and the advantages of the invention will be forthcoming.

The tableted dosage form of liquid, active agent formulation absorbed into particles or the composition of porous particles containing the liquid, active agent formulation may also be utilized as the active agent formulation in associated delivery technologies, such as for example, the Chronset® drug delivery system of Alza Corporation, Palo Alto, California. Such systems can be programmed to release the active agent formulation, in this case the tableted dosage form or the loaded porous particles at designated times and at targeted absorption sites. That technology is described in US Patents Nos.5,110,597; 5,223,265; 5,312,390; 5,443,459; 5,417,682; 5,498,255; 5,531,736; and 5,800,422.

While the dosage forms of the present invention are considered to be particularly advantageous for the preparation of immediate-release dosage forms, they do allow for the modification of the surface area of the tablet or capsule by methods described in US Patent 5,534,263, which is incorporated herein by reference, to provide controlled release of the active agent. In such an aspect of the invention, a banded dosage form may provide the onset of immediate relief due to the exposed portions of the dosage form that are not banded, and then delayed or sustained release of the active agent from the portions of the dosage form that are banded. The advantages of the delivery of a liquid, active agent formulation are present in this configuration of the dosage form as well.

The expression "active agent" as used herein, comprises any active agent, therapeutic compound, drug or composition that can be delivered as a component of a liquid, active agent formulation. The term active agent includes active agents for veterinary and human applications, such as pharmaceutical drugs. The term drug includes an active substance that produces a desired effect, often beneficial or therapeutic in animals, including warm-blooded mammals, humans and primates; avians; household and farm animals; laboratory animals; fishes; reptiles; and zoo animals. The drug can be in various forms such as unchanged molecules, molecular complexes, pharmacologically acceptable salts such as hydrochloride, hydrobromide, sulfate, laurate, palmitate, phosphate, nitrite, nitrate, borate, acetate, maleate, tartrate, oleate, salicylate, and the like. For acidic drugs, salts of metals, amines, or organic cations, for example quarternary ammonium can be used. Derivatives of drugs, such as bases, ester, ether and amide can be used.

The expression "liquid, active agent formulation" may include neat, liquid active agent, or a solution, suspension, slurry, emulsion, self-emulsifying composition, liposomal solution, or other flowable composition in which the active agent is present. The liquid, active agent formulation may be a "solid" at temperatures lower than the temperature of the environment of use, such as body temperature of humans or animals, but the solid should become a flowable, liquid composition after administration or application. The active agent may be accompanied by a binder, antioxidant, pharmaceutically acceptable carrier, permeation enhancer and the like. The amount of an active agent in a dosage form generally is about 0.05 ng to 5 g or more, with individual dosage forms comprising, for example, 25 ng, 1 mg, 5 mg, 10 mg, 25 mg, 100 mg, 250 mg, 500 mg, 750 mg, 1.0 g, 1.2 g, and the like, of active agent. The system can be administered once, twice or thrice daily, or more or less often as required.

The active drug that can be delivered includes inorganic and organic compounds without limitation, including drugs that act on the peripheral nerves, adrenergic receptors, cholinergic receptors, nervous system, skeletal muscles, cardiovascular system, smooth muscles, blood circulatory system, synoptic sites, neuroeffector junctional sites, endocrine system, hormone systems, immunological system, organ systems, reproductive system, skeletal system, autocoid systems, alimentary and excretory systems, inhibitory of autocoids and histamine systems, and physiological systems. The active drug that can be delivered for acting on these animal systems includes depressants, beta-blockers, hypnotics, sedatives, psychic energizers, tranquilizers, anti-convulsants, muscle relaxants, steroids, antiparkinson agents, analgesics, anti-inflammatories, polypeptides, local anesthetics, muscle contractants, anti-microbials, anti-malarials, hormonal agents, contraceptives, sympathomimetics, diuretics, anti-parasitics, neoplastics, hypoglycemics, ophthalmics, electrolytes, diagnostic agents, cardiovascular drugs, calcium channel blockers, angiotensin-converting enzyme inhibitors, and the like.

Exemplary drugs that can be delivered by the immediate-release system of this invention include prochlorperazine edisylate, ferrous sulfate, aminocaproic acid, potassium chloride, mecamylamine hydrochloride, procainamide hydrochloride, amphetamine sulfate, benzphetamine hydrochloride, isoproternol sulfate, methamphetamine hydrochloride, phenmetrazine hydrochloride, bethanechol chloride, metacholine chloride, pilocarpine hydrochloride, atropine sulfate, methascopolamine bromide, isopropamide iodide, tridihexethyl chloride, phenformin hydrochloride, methylphenidate hydrochloride, oxprenolol hydrochloride, metroprolol tartrate, cimetidine hydrochloride, diphenidol, meclizine hydrochloride, prochlorperazine maleate, phenoxybenzamine, thiethylperazine, maleate, anisindone, diphenadione erythrityl teranitrate, digoxin, isofurophate, reserpine, acetazolamide, methazolamide, bendroflumethiazide, chlorpropamide, tolazamide, chlormadinone acetate, phenaglycodol, allopurinol, aluminum aspirin, methotrexate, acetyl sulfisoxazole, erythromycin, progestins, estrogenic progrestational, corticosteroids, hydrocortisone, hydrocorticosterone acetate, cortisone acetate, triamcinolone, methyltesterone, 17 β-estradiol, ethinyl estradiol, ethinyl estradiol 3-methyl ether, prednisolone, 17-hydroxyprogesterone acetate, 19-nor-progesterone, norgestrel, orethindone, norethiderone, progesterone, norgestrone, norethynodrel, aspirin, indomethacin, naproxen, fenoprofen, sulindac, diclofenac, indoprofen, nitroglycerin, propranolol, metroprolol, valproate, oxprenolol, timolol, atenolol, alprenolol, cimetidine, clonidine, imipramine, levodopa, chloropropmazine, resperine, methyldopa, dihydroxyphenylalanine, pivaloyloxyethyl ester of α-methyldopa hydrochloride, theophylline, calcium gluconate ferrous lactate, ketoprofen, ibuprofen, cephalexin, erythromycin, haloperiodol, zomepirac, sildenafil citrate, vincamine, diazepam, phenoxybenzamine, β-blocking agents, calcium-channel blocking drugs such as nifedipine, diltiazen, verapamil, lisinopril, captopril, ramipril, fosimopril, benazepril, libenzapril, cilazapril cilazaprilat, perindopril, zofenopril, enalapril, indalapril, qumapril, and the like. Other active agents are known to the dispensing art as described in Pharmaceutical Sciences, by Remington, 14th Ed., 1979, published by Mack Publishing Co., Easton, Pa.; The Drug, The Nurse, The Patient, Including Current Drug Handbook, 1976, by Falconer et al., published by Saunder Company, Philadelphia, Pa.; Medical Chemistry, 3rd Ed., Vol. 1 and 2, by Burger, published by Wiley-Interscience, New York; and, Physician's Desk Reference, 55th Ed., 1998, published by Medical Economics Co., New Jersey. Particularly suited for the immediate-release dosage form of this invention are pain relievers which are sparingly soluble in water such as acetaminophen, ibuprofen and ketoprofen.

The pharmaceutically acceptable carriers useful for mixing with a drug to provide a dispensable formulation, in a presently preferred embodiment, are carriers that are compatible with the active agent and which are easily excreted, metabolized, assimilated, or the like by a warm-blooded animal. The carrier medium used for the present purpose can be inorganic, or organic, and of naturally occurring or synthetic origin. Examples of carriers included in the term are substances such as solutions, suspensions, liquids, immiscible liquids, emulsions, sols, colloids, and oils. Representative carriers include citrate esters such as triethyl citrate, acetyl triethyl citrate, tributyl citrate, trihexyl citrate, acetyl trihexyl citrate, trioctyl citrate, acetyl trioctyl citrate, acetin, diacetin, triacetin, glycerin, propylene glycol, Vitamin E, triglycerides, liquid alkylene glycols such as ethylene glycol, diethylene glycol, triethylene glycol, ethylene glycol monomethyl ether, liquid polyethylene glycols having a molecular weight of 200, 300, 400 and higher; oils of plant, animal and marine origin such as corn oil, almond oil, babassu oil, eucalyptus oil, cottonseed oil, palm oil, peanut oil, wheat germ oil, tung oil, mint oil, whale oil, herring oil, mineral oil, and the like: emulsions of castor oil in aqueous solutions of pigskin gelatin: emulsions of gum arabic, water and ethyl cellulose; liquid glyceryl triesters of a low molecular weight fatty acids, particularly medium chain mono-, di-, and tri-gycerides; oils with emulsifiers such as mono-or di-glyceride of a fatty acid; a mixture of from about 70% to about 99.9% propylene glycol and from about 0.1% to 30% of glycerin: a mixture of from about 70% to about 99.9% propylene glycol and from about 0.1 to 30% of ethanol; a mixture by volume of from about 80% to 99.9% of propylene glycol and from about 0.1% to about 20% of a mixture of from about 50% to 99.9% of ethanol or glycerin and from 0.1% to about 50% of sterile water; 5% dextrose in physiological saline; oils mixed with surfactants such as poly-oxyethylene sorbitan monolaurate; a mixture of peanut oil and beeswax; peanut oil containing pectin; glycerine and gelatin, with or without added water; glycerin/castile soap formulation; distilled monoglycerides, distilled propylene glycol monoesters, succinylated monoglycerides, acetylated monoglycerides, glyceryl monostearates, monoglycerides water-in-oil emulsions, hydrogenated palm oil, hydrogenated palm oil stearine, hydrogenated soybean oil, hydrogenated vegetable oil, hydrogenated cottonseed oil, partially hydrogenated oils, cottonseed oil, sunflower oil, grapeseed oil, and the like Preferred liquid carriers generally are those in which the unit dose of active agent is soluble.

In general, the present invention has particular utility in the delivery of liquid, active agent formulations that are in the form of emulsions or self-emusifying compositions. The term emulsion as used in this specification denotes a two-phase system in which one phase is finely dispersed in the other phase. The term emulsifier, as used by this invention, denotes an agent that can reduce and/or eliminate the surface and the interfacial tension in a two-phase system. The emulsifier agent, as used herein, denotes an agent possessing both hydrophilic and lipophilic groups in the emulsifier agent. The term microemulsion, as used herein, denotes a multicomponent system that exhibits a homogenous single phase in which quantities of a drug can be solubilized. Typically, a microemulsion can be recognized and distinguished from ordinary emulsions in that the microemulsion is more stable and usually substantially transparent. The term solution, as used herein, indicates a chemically and physically homogenous mixture of two or more substances.

The emulsion formulations of active agent generally comprise 0.5 wt % to 99 wt % of a surfactant. The surfactant functions to prevent aggregation, reduce interfacial tension between constituents, enhance the free-flow of constituents, and lessen the incidence of constituent retention in the dosage form. The therapeutic emulsion formulations useful in this invention may comprise a surfactant that imparts emulsification comprising a member selected from the group consisting of polyoxyethylenated castor oil comprising 9 moles of ethylene oxide, polyoxyethylenated castor oil comprising 15 moles of ethylene oxide, polyoxyethylenated castor oil comprising 20 moles of ethylene oxide, polyoxyethylenated castor oil comprising 25 moles of ethylene oxide, polyoxyethylenated castor oil comprising 40 moles of ethylene oxide, polyoxylenated castor oil comprising 52 moles of ethylene oxide, polyoxyethylenated sorbitan monopalmitate comprising 20 moles of ethylene oxide, polyoxyethylenated sorbitan mono-oleate comprising 20 moles of ethylene oxide, polyoxyethylenated sorbitan monolaurate comprising 20 moles of ethylene oxide, polyoxyethylenated sorbitan monostearate comprising 20 moles of ethylene oxide, polyoxyethylenated sorbitan monostearate comprising 4 moles of ethylene oxide, polyoxyethylenated sorbitan tristearate comprising 20 moles of ethylene oxide, polyoxyethylenated sorbitan monostearate comprising 20 moles of ethylene oxide, polyoxyethylenated sorbitan trioleate comprising 20 moles of ethylene oxide, polyoxyethylenated stearic acid comprising 8 moles of ethylene oxide, polyoxyethylene lauryl ether, polyoxyethylenated stearic acid comprising 40 moles of ethylene oxide, polyoxyethylenated stearic acid comprising 50 moles of ethylene oxide, polyoxyethylenated stearyl alcohol comprising 2 moles of ethylene oxide, and polyoxyethylenated oleyl alcohol comprising 2 moles of ethylene oxide. The surfactants are available from Atlas Chemical Industries, Wilmington, Delaware; Drew Chemical Corp., Boonton, New Jersey; and GAF Corp., New York, New York.

Typically, an active agent emulsified formulation useful in the invention initially comprises an oil phase. The oil phase of the emulsion comprises any pharmaceutically acceptable oil which is not miscible with water. The oil can be an edible liquid such as a non-polar ester of an unsaturated fatty acid, derivatives of such esters, or mixtures of such esters can be utilized for this purpose. The oil can be vegetable, mineral, animal or marine in origin. Examples of non-toxic oils comprise a member selected from the group consisting of peanut oil, cottonseed oil, sesame oil, olive oil, corn oil, almond oil, mineral oil, castor oil, coconut oil, palm oil, cocoa butter, safflower, a mixture of mono- and diglycerides of 16 to 18 carbon atoms, unsaturated fatty acids, fractionated triglycerides derived from coconut oil, fractionated liquid triglycerides derived from short chain 10 to 15 carbon atoms fatty acids, acetylated monoglycerides, acetylated diglycerides, acetylated triglycerides, olein known also as glyceral trioleate, palmitin known as glyceryl tripalmitate, stearin known also as glyceryl tristearate, lauric acid hexylester, oleic acid oleylester, glycolyzed ethoxylated glycerides of natural oils, branched fatty acids with 13 molecules of ethyleneoxide, and oleic acid decylester. The concentration of oil, or oil derivative in the emulsion formulation is 1 wt % to 40 wt %, with the wt % of all constituents in the emulsion preparation equal to 100 wt %. The oils are disclosed in *Pharmaceutical Sciences* by Remington, 17^{th} Ed., pp. 403-405, (1985) published by Mark Publishing Co., in *Encyclopaedia of Chemistry,* by Van Nostrand Reinhold, 4^{th} Ed., pp. 644-645, (1986) published by Van Nostrand Reinhold Co.; and in U. S. Patent No. 4,259,323 issued to Ranucci.

All dosage forms of the present invention may include an antioxidant to slow or effectively stop the rate of any autoxidizable material present in the dosage form, particularly if it is in the form of a gelatin capsule. Representative antioxidants comprise a member selected from the group of ascorbic acid; alpha tocopherol; ascorbyl palmitate; ascorbates; isoascorbates; butylated hydroxyanisole; butylated hydroxytoluene; nordihydroguiaretic acid; esters of garlic acid comprising at least 3 carbon atoms comprising a member selected from the group consisting of propyl gallate, octyl gallate, decyl gallate , decyl gallate; 6-ethoxy-2,2,4-trimethyl-1,2-dihydro-guinoline; N-acetyl-2,6-dit-butyl-p-aminophenol; butyl tyrosine; 3-tertiarybutyl-4-hydroxyanisole; 2-tertiary-butyl-4-hydroxyanisole; 4-chloro-2,6-ditertiary butyl phenol; 2,6-ditertiary butyl p-methoxy phenol; 2,6-ditertiary butyl-p-cresol: polymeric antioxidants; trihydroxybutyro-phenone physiologically acceptable salts of ascorbic acid, erythorbic acid, and ascorbyl acetate; calcium ascorbate; sodium ascorbate; sodium bisulfite; and the like. The amount of antioxidant used for the present purposes is about 0.001% to 25% of the total weight of the composition present in the dosage form. Antioxidants are known to the prior art in U.S. Pat. Nos. 2,707,154; 3,573,936; 3,637,772; 4,038,434; 4,186,465 and 4,559,237.

All dosage forms of the present invention may also contain a chelating agent to protect the active agent either during storage or when in use. Examples of chelating agents include, for example, polyacrylic acid, citric acid, edetic acid, disodium edetic acid, and the like. The chelating agent may be co-delivered with the active agent in the environment of use to preserve and protect the active agent *in situ.* Protection is provided for active agents which are inactivated by chelation with multivalent metal cations such as calcium, magnesium or aluminum that may be present in some foods and are at natural background levels in the fluids of the gastrointestinal tract. Such chelating agents may be combined with the liquid, active agent formulation in the porous particles.

The liquid formulation of all forms of the present invention may also comprise a surfactant or a mixture of surfactants where the surfactant is selected from the group consisting of nonionic, anionic and cationic surfactants. Exemplary nontoxic, nonionic surfactants suitable for forming a composition comprise alkylated aryl polyether alcohols known as Triton®; polyethylene glycol tertdodecyl throether available as Nonic®; fatty and amide condensate or Alrosol®; aromatic polyglycol ether condensate or Neutronyx®; fatty acid alkanolamine or Ninol® sorbitan monolaurate or Span®; polyoxyethylene sorbitan esters or Tweens®; sorbitan monolaurate polyoxyethylene or Tween 20®; sorbitan mono-oleate polyoxyethylene or Tween 80®; triblock copolymers polyoxyethylene-polyoxypropylene-polyoxyethylene or Pluronics®; polyglycolyzed glycerides such as Labraosol, polyoxyethylated castor oil such as Cremophor and polyoxypropylene-polyoxyethylene-8500 or Pluronic®. By way of example, anionic surfactants comprise sulfonic acids and the salts of sulfonated esters such as sodium lauryl sulfate, sodium sulfoethyl oleate, dioctyl sodium sulfosuccinate, cetyl sulfate sodium, myristyl sulfate sodium; sulated esters; sulfated amides; sulfated alcohols; sulfated ethers; sulfated carboxylic acids; sulfonated aromatic hydrocarbons; sulfonated ethers; and the like. The cationic surface active agents comprise cetyl pyridinium chloride; cetyl trimethyl ammonium bromide; diethylmethyl cetyl ammonium chloride; benzalkonium chloride; benzethonium chloride; primary alkylamonium salts; secondary alkylamonium salts; tertiary alkylamonium salts; quaternary alkylamonium salts; acylated polyamines; salts of heterocyclic amines; palmitoyl carnitine chloride, behentriamonium methosulfate, and the like. Generally, from 0.01 part to 1000 parts by weight of surfactant, per 100 parts of active agent is admixed with the active agent to provide the active agent formulation.

Surfactants are known to the prior art in U.S. Pat. Nos. 2,805,977; and in 4,182,330.

The liquid formulation of all forms of the present invention may also comprise permeation enhancers that facilitate absorption of the active agent in the environment of use. Such enhancers may, for example, open the so-called "tight junctions" in the gastrointestinal tract or modify the effect of cellular components, such a p-glycoprotein and the like. Suitable enhancers include alkali metal salts of salicyclic acid, such as sodium salicylate, caprylic or capric acid, such as sodium caprylate or sodium caprate, and the like. Enhancers may include the bile salts, such as sodium deoxycholate. Various p-glycoprotein modulators are described in US Patents 5,112,817 and 5,643,909. Various other absorption enhancing compounds and materials are described in US Patent 5,824,638. Enhancers may be used either alone or as mixtures in combination with other enhancers.

The liquid, active agent formulation of all dosage forms of the present invention may optionally be formulated with inorganic or organic acids or salts of drugs which promote dissolution and disintegration or swelling of the porous particles upon contact with biological fluids. The acids serve to lower the pH of the microenvironment at the porous particle, and promote rapid dissolution of a particle, such as calcium hydrogen phosphate, that is soluble in low pH environments, thus providing rapid liberation of the liquid, active agent formulation contained in the porous particle. Examples of organic acids include citric acid, tartaric acid, succinic acid, malic acid, fumaric acid and the like. Salts of drugs where the anion of the salt is acidic, such as acetate, hydrochloride, hydrobromide, sulfate, succinate, citrate, and the like, can be utilized to produce immediate disintegration and dissolution of the porous particle. A more complete list of acidic components for this application is provided in Journal of Pharmaceutical Sciences, "Pharmaceutical Salts", Review Articles, January, (1977), Vol. 66, No. 1, pages 1-19. The interaction of an acidic component with a porous particle of, for example, calcium hydrogen phosphate, in the presence of water from gastric fluids accelerates dissolution of the particle at a greater rate than gastric fluid alone, producing a more rapid and complete release of the liquid, active agent formulation into the environment of use. Likewise alkaline components or salts of drugs where the cation of the salt is alkaline such as choline may be incorporated into the liquid, active agent formulation to promote rapid and complete dissolution of a porous particle which is soluble or swells at elevated pH. Such a particle may be formed, for example, of poly(methacrylic acid-methyl methacrylate) 1:2 available commercially as Eudragit S100 (Rohm America, Sommerset, New Jersey.

The following examples are illustrative of the first embodiment of the present invention.

### EXAMPLE 1

A general procedure for the formation of the tableted form of the dosage form of the invention is presented below. Percentages are by weight unless otherwise specified.

An immediate-release dosage form of the anti-impotence drug sildenafil citrate is prepared. 70 Grams of the active agent sildenafil citrate is mixed with 280 grams of the liquid carrier propylene glycol. The active agent/liquid mixture is added to 550 grams of calcium hydrogen phosphate particles, FujiCalin® Type S. The blend is tumble mixed at room temperature in a twin-shell blender for 20 minutes, producing a free-flowing dry mix. Then 100 grams of the disintegrating agent low-substituted hydroxypropyl cellulose, having an average hydroxypropoxyl content of 10-13 weight percent is added to the blend, and the combined mixture is tumble mixed for an additional 5 minutes. The resulting formulation is transferred to a tablet press. Oval tablets having a major axis length of ½ inch (121.7 mm) and a minor axis length of 9/32 inch (7.1mm) and weighing 357 mg are compressed using a force of 1.0 ton. Each tablet contains a unit dose of 25 mg of active agent. The tablets are transferred to a pharmaceutical pan coater where 20 mg of water-soluble film coating is applied to each tablet. The composition of the film coating consists of 75 parts hydroxypropyl methylcellulose and 25 parts polyethylene glycol. The hydroxypropyl methylcellulose has a hydroxyl content of 10 weight percent, a methoxyl content of 29 weight percent, and molecular weight of approximately 11,900 grams per mole. The polyethylene glycol has a molecular weight of 8,000 grams per mole. The resulting tablet disintegrates rapidly when placed in a simulated gastric fluid environment and release active agent immediately.

### EXAMPLE 2

The general procedure of EXAMPLE 1 is followed to prepare dosage forms containing 250 mg of acetaminophen, 50 and 100 mg of ibuprofen and 25, 50 and 75 mg of ketoprofen in a tablet form. The fabricated dosage forms disintegrate rapidly when placed in a simulated gastric fluid environment and release active agent immediately.

### EXAMPLE 3

Blends of 80% calcium hydrogen phosphate particles, FujiCalin® Type S, and 20% magnesium aluminosilicate powder, Neusilin™ grades S₁, SG₁, US₂ and UFL₂, respectively, are substituted for the 100% FujiCalin® Type S particles of EXAMPLE 1, and unit dosage forms containing 25 mg of sildenafil citrate, 250 mg of acetaminophen, 50 and 100 mg of ibuprofen and 25, 50 and 75 mg of ketoprofen in tablet form are prepared. The fabricated dosage forms disintegrate rapidly when placed in a simulated gastric fluid environment and release active agent immediately.

### EXAMPLE 4

Proportional amounts of magnesium aluminosilicate powder, Neusilin™ grades S₁, SG₁, US₂ and UFL₂, respectively, are substituted for the 100% FujiCalin® Type S particles of EXAMPLE 1, and unit dosage forms containing 25 mg of sildenafil citrate, 250 mg of acetaminophen, 50 and 100 mg of ibuprofen and 25, 50 and 75 mg of ketoprofen in tablet form are prepared. The fabricated dosage forms disintegrate rapidly when placed in a simulated gastric fluid environment and release active agent immediately.

### EXAMPLE 5

An equivalent amount of FujiCalin® Type SG particles is substituted for the FujiCalin® Type S particles in EXAMPLE 1, and the procedures of that example are generally followed to prepare unit dosage forms containing 25 mg of sildenafil citrate, 250 mg of acetaminophen, 50 and 100 mg of ibuprofen and 25, 50 and 75 mg of ketoprofen in tablet form. The fabricated dosage forms disintegrate rapidly when placed in a simulated gastric fluid environment and release active agent immediately.

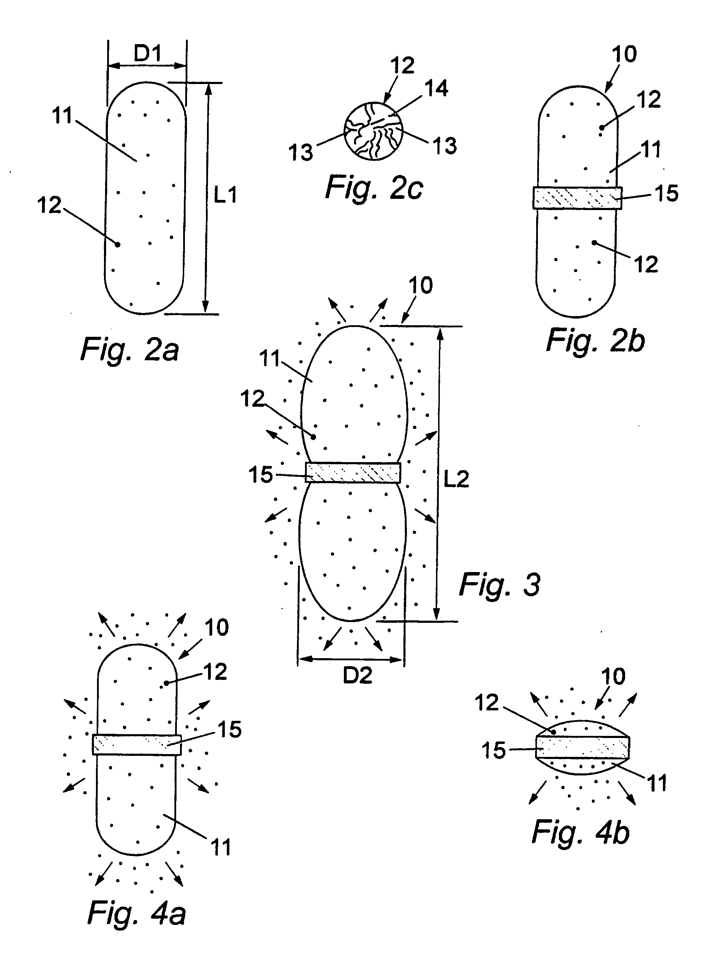

Figures 2-5 depict forms of a delivery device according to the second embodiment of the present invention using a bioerodible carrier. The delivery device or active agent dosage form 10 comprises a polymer matrix 11 having a plurality of porous particles 12 having pores 13 in which the liquid, active agent 14 is absorbed (illustrated by the multitude of dots) dissolved or dispersed therein. Polymer matrix 11 typically is formed of combination of a swellable, high molecular weight, water-soluble polymer and a hydroattractant. Materials useful for sorbing the liquid active agent formulations have already been described herein. Generally, the polymer/liquid, active agent formulation matrix will contain at least 10% of the polymer component to form a gel when in the environment of use.

Particular suitable porous particle is exemplified by the particular forms of calcium hydrogen phosphate and magnesium aluminometasilicate as previously described. Other absorptive materials may be substituted for the foregoing. For example, powders of microcrystalline cellulose sold under the tradenames Avicel (FMC Corporation) and Elcema (Degussa) and porous agglomerated silicon dioxide, sold under the tradenames Cab-O-Sil (Cabot) and Aerosil (Degussa), may be used.

The method of this form of the invention may be applied generally to liquid formulations such as those sold commercially as liquid formulations or those prepared as described herein. Examples of commercially available encapsulated liquid formulations that may be utilized include, inter alia, Placidyl® brand of ethchlorvynol, Adalat® brand of nifedipine, VePesid® brand of etoposide, Lanoxicaps® brand of digoxin, Zantac® brand of ranitidine hydrochloride, Sandimmune® and Neoral® brands of cyclosporin, Calderol® brand of calcifediol, Zarontin® brand of ethosuximide, Procardia® brand of nifedipine, Rocaltrol® brand of calcitriol and Vescenoid® brand of tretinoin.

Representative examples of the swellable polymer comprising high molecular weight, water-soluble polymers are polyethylene oxide and cellulosic polymer derivatives including hydroxypropyl cellulose, hydroxypropyl methyl cellulose, hydroxyethyl cellulose, sodium carboxy methylcellulose, calcium carboxymethyl cellulose, methyl cellulose, as well as noncellulosics such as maltodextrin, polyvinyls, polyvinyl alcohol, polyacrylic acids, alginates, gelatin, natural gums, including guar, lightly crosslinked versions of these polymers,starches, starch graft copolymers and the like. The polymers generally have number average molecular weights over 50,000 grams per mole, such as between 50,000 and 10,000,000 grams per mole and representative viscosities, e.g. for polyethylene oxide in the range of 12-20,000 cps (5% aq, 25°C, MW 100,000-900,000), 400-4000 cps (2% aq, 25°C, MW 1,000,000 - 2,000,000) and 1500-15,000 cps (1% aq, 25°C, MW 4,000,000 - 8,000,000) [Brookfield viscometer, rotational spindle]; for methylcellulose in the range of 1,500-18,000 cps (2% aq, 20°C, MW 62,000-134,000) [Ubbelohde tube viscometer]; for hydroxypropyl methylcellulose in the range of 4,000-100,000 cps (2% aq, 20°C, MW 88,000-242,000) [Ubbelohde tube viscometer]; for hydroxyethyl cellulose in the range of 75-400 cps (5% aq, 25°C, MW 90,000-200,000), 400-6500 cps (2% aq, 25°C, MW 300,000 - 720,000) and 1500-5,000 cps (1% aq, 25°C, MW 1,000,000 - 1,300,000) [Brookfield viscometer, rotational spindle]; for guar about 5100 cps (1%) [Brookfield viscometer, rotational spindle]; for poly(methyl vinyl ether/maleic anhydride) in the range of 15 to greater than 200 cps (5% aq., MW 20,000-80,000) [Brookfield viscometer, rotational spindle]; for polyvinyl alcohol in the range 27-65 cps (4%aq, 20°C [Hoeppler falling ball method and 1100-1500 cps (10%aq, 25°C) [Brookfield viscometer, rotational spindle; for sodium carboxymethyl cellulose in the range of 25-50 cps (2% aq, 25°C) (MW 90,000) to about 2,500-6,000 cps (1% aq, 25°C) (MW 700,000) [Brookfield viscometer, rotational spindle]; and for sodium polyacrylic acid 5000-80,000 (0.5% aq) (MW 750,000 - 4,000,000) [Brookfield viscometer, rotational spindle]. Polymers having molecular weights between 300,000 and 8,000 000 grams per mole are preferred, and those having molecular weights between about 2,000,000 to 8,000,000 grams per mole are especially preferred. Polyethylene oxide having a number average molecular weight between about 5,000,000 to 8,000,000 grams per mole is most especially preferred, e.g. Polyox 303 and Polyox 308. Also, especially preferred are methylcellulose type/grade A15C, A4M, A18M and hydroxypropyl methylcellulose type/grade K4M, K15M, K100M, E4M and F4M (Dow Chemical Company); hydroxyethyl cellulose such as Natrosol® HEC; hydroxypropyl cellulose such as Klucel (Grades H, M, G, J, L, E - Aqualon Company); guar such as Supercol® Guar U (Aqualon Company); pectin such as GENU Pectin (Aqualon Company); carrageenan such as GENU Carrageenan (Aqualon Company); poly(methyl vinyl ether/maleic anhydride) such as Gantrez® AN Copolymer (AN-119, -139, -149, -169, -179, GAF Corporation); polyvinyl alcohol such as Elvanol® 71-30, Elvanol® 85-30, Elvanol® 50-42 and Elvanol® HV (DuPont); sodium carboxymethyl cellulose such as Aqualon cellulose gum grade 7H4; polyacrylic acids such as Carpobol® resin grades 971P, 974P, 980, 981, 1382, 2984, 5984, ETD 2001, ETD 2050, calcium polyacrylic acids such as Noveon® resin grades AA-1, CA-1 and CY-2, and sodium polyacrylic acid (BF Goodrich, Cleveland, Ohio).

Representative examples of hydroattractants are water-insoluble polymers such as low substituted hydroxypropyl cellulose, microcrystalline cellulose (Avicel), cross-linked sodium or calcium carboxymethyl cellulose, cellulose fiber (Solka-Floc or Elcema), cross-linked polyvinyl pyrrolidone (Polyplasdone XL), cross-linked Amberlite resin, alginates (Satialgine), colloidal magnesium-aluminum silicate (Veegum), corn starch granules, rice starch granules, potato starch granules, wheat starch granules, sodium carboxymethyl starch (Expotab, Primojel), corn starch/acrylamide/sodium acrylate copolymer, acrylamide/sodium acrylate copolymer and the like. A particularly suitable hydroattractant is hydroxypropyl cellulose having a hydroxypropyl content of between about 8-15 weight percent , and preferably about 10-13 weight percent, such as that supplied as Low Substituted Hydroxypropyl Cellulose grade 11 as manufactured by Shin-Etsu Chemical Company, Ltd., Tokyo, Japan.

Typically, the water soluble, high molecular weight polymer in the polymer matrix is present in from about 5% to about 90% by weight based on the total weight of the active agent formulation matrix, and the hydroattractant is present in from about 5% to about 70% by weight based on the total weight of the active agent formulation matrix. The particular percentages may be chosen to provide the desired retention time in the stomach and the desired release profile of active agent. However, it is presently preferred to have the polymer matrix contain from about 10 weight percent to about 50 weight percent of the water soluble, high molecular weight polymer and from about 10 weight percent to about 60 weight percent of the hydroattractant, with weight percentages of water soluble, high molecular weight polymer in the range of 10 to 40 weight percent and hydroattractant in the range of 25 to 35 being especially preferred.

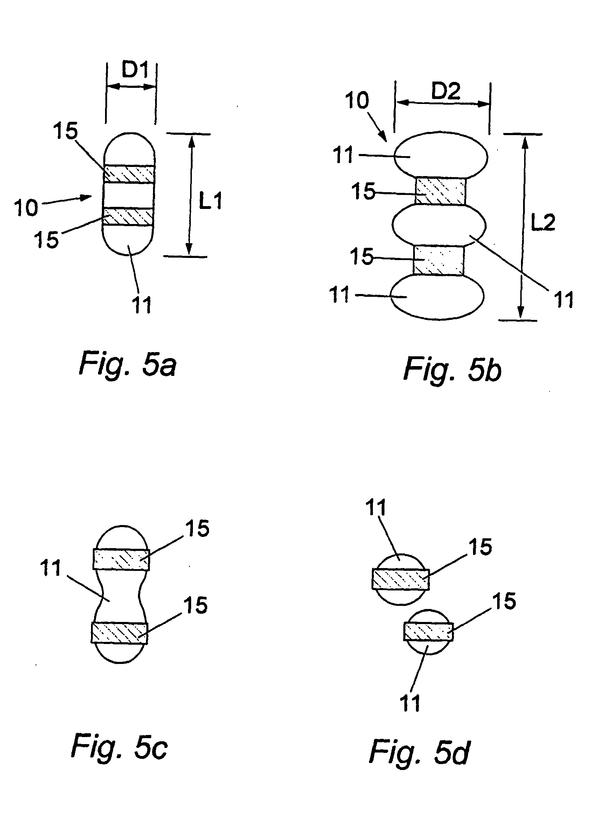

Dosage form 10 is conveniently cylindrically shaped with rounded ends that facilitate administration of the dosage form in its non-swelled state. In FIG. 2A, the device 10 is shown in preparation prior to application of the insoluble material or band 15 shown in FIG. 2B. The insoluble material exemplified as band 15, circumscribes a portion of the outer surface of the polymer matrix 11. While a single band is illustrated in FIG. 2, additional bands such as illustrated in FIG. 5 can be utilized depending on the particular application for which the device is being used.

The band of insoluble material 15 is applied to the outer surface of the polymer matrix. The insoluble material imparts rigidity to the gel-forming polymer matrix to manage gastric retention time and further control the delivery profile of the active agent of interest. Band 15 typically exhibits low water permeability and will prevent that portion of the polymer matrix which it surrounds from imbibing fluid, thus substantially limiting any swelling of polymer matrix 11 at that location. The number, size, and placement of the insoluble bands that are applied onto the surface of the active agent formulation matrix may be varied to adjust the active agent delivery profile and the retention time in the stomach. For example, bands 0.1 mm to about 12 mm in width, preferably between about 0.5 and 8 mm, may be applied onto the active agent formulation matrix surface. Further, between about 1 and 10 bands may be used, but generally between about 1 and 3 are affixed to the matrix. The bands may be placed close together (i.e., within about 0.5 mm of each other) or may be placed about 8 to 12 mm apart.

With reference to Figs. 5A-5D, dosage form 10 is formed with two bands 15, each circumscribing a portion of the surface of polymer matrix 11 in which active agent (not shown) is dispersed. FIG. 5A illustrates dosage form 10 in its initial configuration before it has imbibed any fluid. Upon administration to a subject, dosage form 10 swells as shown in FIG. 5B in those segments of polymer matrix 11 that are not surrounded by bands 15. Because of the low fluid impermeability of bands 15, those portions of polymer matrix 11 surrounded by bands 15 do not appreciably imbibe fluid and the polymer in such segments of the polymer matrix does not swell to any significant extent. FIGs. 5C and 5D illustrate sequential states of dosage form 10 after it is substantially eroded by gastric fluid and contractions of the stomach. Eventually, dosage form 10 will separate into two pieces and be expelled from the stomach.

The insoluble material comprising band(s) 15 may be any material that is nontoxic, biologically inert, nonallergenic and nonirritating to body tissue, that exhibits little impermeability to liquids, and that maintains its physical and chemical integrity in the environment of use for at least a portion of the dispensing period. The low liquid permeability of the insoluble material serves to limit swelling of the polymer matrix in that section of the polymer matrix that is surrounded by the band.

Insoluble materials from which the bands may be prepared include, for example, polyethylene, polystyrene, ethylene-vinyl acetate copolymers, polycaprolactone and Hytrel® polyester elastomers (Du Pont). Additional banding materials include but are not limited to polysaccharides, cellulosics, cellulose acetate, cellulose acetate propionate, cellulose acetate butyrate, cellulose acetate pseudolatex (such as described in U.S. Patents 4,931,285 and 5,024,842), cellulose acetate propionate, cellulose acetate butyrate, ethyl cellulose, ethyl cellulose pseudolatex (such as Surelease® as supplied by Colorcon, West Point, PA or Aquacoat™ as supplied by FMC Corporation, Philadelphia, PA), nitrocellulose, polylactic acid, poly- glycolic acid, polylactide glycolide copolymers, polycaprolactone, polyvinyl alcohol, polyvinyl acetate, polyethylene vinylacetate, polyethylene teraphthalate, polybutadiene styrene, polyisobutylene, polyisobutylene isoprene copolymer, polyvinyl chloride, polyvinylidene chloride-vinyl chloride copolymer, copolymers of acrylic acid and methacrylic acid esters, copolymers of methylmethacrylate and ethylacrylate, latex of acrylate esters (such as Eudragit® supplied by RöhmPharma, Weiterstadt, Germany), polypropylene, copolymers of propylene oxide and ethylene oxide, propylene oxide ethylene oxide block copolymers, ethylenevinyl alcohol copolymer, poly sulfone, ethylene vinylalcohol copolymer, polyxylylenes, polyamides, rubbers, such as styrenebutadiene, polyisobutylene and the like, natural and synthetic waxes, paraffin, carnauba wax, petroleum wax, white or yellow bees wax, castor wax, candelilla wax, rice bran wax, microcrystalline wax, stearyl alcohol, cetyl alcohol, bleached shellac, esterified shellac, chitin, chitosan, silicas, polyalkoxysilanes, polydimethyl siloxane, polyethylene glycol-silicone elastomers, crosslinked gelatin, zein, electromagnetic irradiation crosslinked acrylics, silicones, or polyesters, thermally crosslinked acrylics, silicones, or polyesters, butadiene-styrene rubber, glycerol ester of partially dimerized rosin, glycerol ester of partially hydrogenated wood rosin, glycerol ester of tall oil rosin, glycerol ester of wood rosin, pentaerythritol ester of partially hydrogenated wood rosin, pentaerythritol ester of wood rosin, natural or synthetic terpene resin and blends of the above.

The banding materials often are also formulated with plasticizers, and optionally with wetting agents, surfactants, opacifiers, colorants, flavorants, taste-masking agents, and the like. Examples of typical plasticizers are as follows: triacetin, polyhydric alcohols, polyethylene glycol, glycerol, propylene glycol, acetate esters, glycerol triacetate, triethyl citrate, acetyl triethyl citrate, glycerides, acetylated monoglycerides, oils, mineral oil, castor oil and the like. Referring again to the embodiment of the invention depicted in FIG. 2A, the polymer matrix 11 in its non-swelled state has a length L1 and a maximum diameter D1 intermediate the ends. FIG. 3 shows dispensing device 10 after having been placed in the stomach. The active agent formulation matrix 11 on each side of the band 15 has swelled from imbibing fluid from the stomach and begun to erode, thereby releasing porous particles 12 to the stomach environment. In contrast to the exposed segments of the swollen polymer matrix 11, band 15 and the portion of the polymer matrix beneath it have not swelled to such an extent. Accordingly, that segment of the polymer matrix surrounded by band 15 is maintained in a constrained and more compressed, non-swollen state than the unhanded portion of the matrix. Since band 15 does not take up an appreciable amount of fluid from the stomach and swell, band 15 retains its substantially rigid or semi-rigid form, and provides an element of rigidity to the dosage form as a whole. While it is not entirely clear how band 15 and the constrained segment of polymer matrix 11 facilitate retention of the dosage form in the stomach through housekeeping waves, it is thought that the band reduces the rate of erosion of the polymer matrix, thus maintaining a larger effective size of the dosage form and reducing the chance for its expulsion from the stomach, for a longer period of time than would otherwise occur if the band was not present. Additionally, the presence of the band on the polymer matrix provides a semi-rigid segment of the dosage form that appears to cause the dosage form to be retropelled into the main area of the stomach as a reaction to the stomach contractions rather than being expelled by the housekeeping wave, as a less rigid gel would be inclined to be.

After swelling, the dosage form 10 has a length L2 and a maximum diameter D2 measured at the widest part of the swollen polymer matrix. Generally, for human applications the largest dimension of the device in the swollen state equivalent to the diameter D2 should be greater than 7 mm, preferably 10 mm or greater, and most preferably 13 mm or greater during the period of residence in the stomach when active agent is being dispensed. Since the dosage form is intended to remain in the stomach for a prolonged retention period, the effective diameter of the active agent dosage form in when in its swollen state in the stomach may have to be significantly larger than 13 mm, and may extend to more that 50 mm or greater. Larger dosage forms may be appropriate particularly when the polymer matrix is designed to erode relatively rapidly over time in order to provide the required delivery of active agent for therapeutic effect. For applications in animals other than humans, for example in dogs, the maximum diameter should be greater than about 2 mm.

The maximum dimension for any particular dosage form will depend on the particular application and animal in which the device is being used. Such dimensions can be determined by those skilled in the art in accordance with the teaching herein and the various patents and publications noted herein and existing in the related art. A practical consideration, particularly for oral administration to humans, is that the initial size of the device be such that it can be reasonably, comfortably swallowed. For human oral applications, a preferred size of the device in its form prior to administration to the stomach would be on the order of a size 000 capsule to a size 5 capsule. However, it is understood that smaller or larger sizes could be used for particular applications where necessary.

Since the dosage forms of the invention may be gel-forming, it may be desirable to wet the outer surface of the dosage form immediately prior to the subject swallowing the dosage form in order to provide a more slippery outer surface and promote ease of swallowing. Alternatively, the matrix core can be inserted into a hard gelatin capsule prior to application of the band in order to facilitate swallowing and also promote ease of manufacture in applying and forming the bands. Upon entering the stomach, that portion of the hard gelatin capsule that is not covered by the band will dissolve, exposing the polymer matrix to fluid in the stomach. As the polymer matrix imbibes fluid, the dosage form will swell in the exposed segments as previously described. The dosage form typically is prepared to allow for swelling at a controlled rate, particularly at a limited initial rate, so that the dosage form does not swell inordinately during the swallowing process and result in obstruction of the esophagus.

It is preferred that the dosage forms of certain embodiments be administered when the subject is in the fed state to allow time for maximum swelling of the polymer matrix prior to the housekeeping wave being initiated. Generally a meal size that results in a delay of the housekeeping wave of from about 1 to 3 hours is satisfactory. It may be preferable to administer one or more of the dosage forms at the start of each dosing period, depending on the size of the dosage form, to facilitate swallowing and yet provide sufficient dose of active agent. Particularly in those instances where the dosage form is near the lower end of the size range, i.e., the maximum diameter along the longitudinal axis is on the order of 7-13 mm, it is preferable that the dosage form be administered to the subject in the fed state to allow for significant swelling of the dosage form prior to the housekeeping wave occurring. Typically, administration will occur with the meal or within two hours thereafter, and preferably within one hour of completion of the meal. Depending on the half-life of an active agent, once-a-day dosing could conveniently occur with or after dinner. For b.i.d. (i.e., twice-a-day) dosing to a human subject, the dosage form can conveniently be administered with or after breakfast and dinner, but, if after, preferably within one or two hours after conclusion of the meal. For more frequent administration, such as t.i.d., the dosage form may be administered after breakfast, lunch and dinner. For administration within usual meal patterns, it is desirable that the subject consume small amounts of food or liquids prior to administration of the dosage form. The dosage form may be administered prior to the taking of food if administered with a sufficient quantity of liquid so as to delay onset of the housekeeping wave, until consumption of food is initiated.

To facilitate retention of the dosage forms of the invention, particularly if the dosage form is to be administered to a subject in the fasted state, it may be desirable to combine one or more gastric-emptying delaying agents with the active agent composition or coat the dosage form with a composition containing a gastric-emptying delaying agent, i.e., a substance that delays onset of the housekeeping wave of the IMMC. Examples of agents for delaying onset of the housekeeping wave, preferably locally delivered by the dosage form in amounts not resulting in any substantial systemic effect to the subject, as for example, anticholenergic agents such as propantheline, and other agents including, but not limited to, methylcellulose, guar gum, fats such as triglyceride esters, e.g., triethanol myristate, fatty acids of 10-15 carbon atoms, and the like.

Figs. 4A and 4B show dosage form 10 after a length of time in the fluid environment of the stomach. Polymer matrix 11 has eroded at the exposed surface of the matrix, i.e., those portions of the matrix not covered by the insoluble material 15 to such an extent that the device 10 is smaller than its initial swollen configuration. Erosion of the matrix permits release of the porous particles from the matrix. After the porous particles are released, the liquid, active agent may elute by diffusion or convection from the pores into the environment of use. Additionally, as the released porous particles disintegrate in the gastric environment, the liquid, active agent formulation will be directly released into the environment of use. At some point, the matrix may erode to such an extent that the remainder of the dosage form is expelled from the stomach. Band 15 will be expelled from the stomach either alone, if it has separated from the dosage form at some time near the end of the delivery period, or as part of the remainder of the dosage form expelled from the stomach. In some applications, it may be desirable to form band 15 with weakened portions so that band 15 splits and falls away from the polymer matrix after some predetermined time in the stomach to permit a particular release pattern of active agent from the dosage form over the delivery period.

The dosage forms in this embodiment of the invention can be prepared by standard methods from the materials previously described. Typically, the liquid, active agent formulation will be prepared independently of the porous particle; although in some circumstances, it may be desirable to combine the formation of the liquid, active agent formulation with the mixing of the formulation components and the porous particles. As described previously, the liquid formulation may be a solution, suspension, dispersion, emulsion, etc. depending on the particular application for which the dosage form is intended.

After, the liquid, formulation is prepared, the desired quantity of liquid, active agent formulation and porous particles may be mixed in a blender to sorb the liquid into the porous particles. That mixture may be milled by passing it through mesh screens to insure intimate mixing and complete absorption of the liquid, active agent formulation into the porous particles. The wet granulation may then be dried at ambient conditions to facilitate handling. However, the drying conditions are not so severe as to evaporate a significant amount of the liquid of the liquid, active formulation. Also, to facilitate handling, it may be desirable to add a small amount of another absorbent , such as a soluble sugar, e.g., maltose or the like, that will readily dissolve in the environment of use when the porous particle is released from the dosage form, but not subsequently change the desired release characteristics of the dosage form. Small amounts of inert absorbents, such as mircocrystalline cellulose or silicon dioxide, may be substituted for the soluble material, but, again, the quantities should not be so great that the desired release characteristics of the liquid, active agent formulation from the absorbent particles is significantly affected. Another method of manufacture would be to sorb the liquid, active agent formulation into the particles in a fluidized bed of the particles. Those and other methods are conventional and will be apparent to those skilled in the art.

An appropriate quantity of porous particles, containing the liquid, active agent formulation, and the polymer ingredients are separately passed through a screen, such as a screen having a mesh of about 40 wires per inch, to reduce any larger sized materials, and dry mixed. Then, a pharmaceutically-acceptable liquid, having a sufficient vapor pressure to allow subsequent drying over a reasonable period of time, for example 24 hours, is added to the dry mixture and the damp mass is extruded through a mesh screen (e.g. 20 wires per inch) to further mix the materials. Examples of suitable liquids are water, methanol, ethanol, isopropanol, acetone, and the like. The liquid will need to be compatible with the liquid of the liquid, formulation.

After the extrusion process, the mixture is allowed to dry, for example in air overnight at room temperature, if the active agent does not require any special handling. After drying, the resulting material is granulated, for example by passing the dried material through a mesh screen (e.g., 20 wires per inch). The granules are combined with a suitable tableting lubricant which has been previously passed through a mesh screen (e.g., 60 wires per inch). The resulting material is tumbled to produce the finished granulation for the tableting process. Tablets are produced using well known methodologies associated with horizontal and vertical compression units using dies and punches of appropriate dimensions. Alternate granulation methods, for example, fluid bed granulation or direct compression granulation can be used as well and such method will be chosen by one skilled in the art depending on the particular nature of the materials being used and the convenience and preference of the fabricator.

In order to prepare a preferred device of the present invention, the active agent formulation is first prepared and formed into a matrix of the desired size and shape typically by tableting, e.g. by conventional tableting methods. The matrix in its initial prepared form is about the size and dimensions of a size "000" to size 5 hard gelatin capsule. The cross-sectional shape of the matrix may be generally circular or may be oval, triangular, square, hexagonal or other shapes that are easily handled, especially by patients with limited dexterity. Presently preferred shapes are those in which the cross-section is circular or oval. The ring or bands are then placed onto the surface of active agent formulation matrix or printed onto the surface using conventional banding or printing techniques, such as disclosed herein or in U.S. Patent 5,534,263, which is incorporated herein by reference.

The terms "active agent" and "drug" are used interchangeably herein and refer to an agent, active agent, compound, composition of matter or mixture thereof which provides some pharmacologic, often beneficial, effect. This includes foods, food supplements, nutrients, drugs, antiacids, vitamins such as, for example, Vitamin C, Vitamin E, microorganism attenuators and other agents that benefit the environment of use. As used herein, the terms include any physiologically or pharmacologically active substance that produces a localized or systemic effect or effects in animals, including warm blooded mammals, humans and primates; domestic household or farm animals such as cats, dogs, sheep, goats, cattle, horses and pigs; laboratory animals such as mice, rats and guinea pigs; zoo and wild animals; and the like. The active agent that can be delivered includes inorganic and organic compounds, including, without limitation, active agents which act on the peripheral nerves, adrenergic receptors, cholinergic receptors, the skeletal muscles, the cardiovascular system, smooth muscles, the blood circulatory system, synoptic sites, neuroeffector junctional sites, endocrine and hormone systems, the immunological system, the reproductive system, the skeletal system, autacoid systems, the alimentary and excretory systems, the histamine system and the central nervous system.

Suitable active agents may be selected from, for example, proteins, enzymes, enzyme inhibitors, hormones, polynucleotides, nucleoproteins, polysaccharides, glycoproteins, lipoproteins, peptides, polypeptides, steroids, hypnotics and sedatives, psychic energizers, tranquilizers, anticonvulsants, antidepressants, muscle relaxants, antiparkinson agents, analgesics, immunosuppressants, anti-inflammatories, antihistamines, local anesthetics, muscle contractants, antimicrobials, antimalarials, antivirals, antibiotics, antiobesity agents, antidiabetic agents, hormonal agents including contraceptives, sympathomimetics, polypeptides and proteins capable of eliciting physiological effects, diuretics, lipid regulating agents, antiandrogenic agents, antiparasitics, neoplastics, antineoplastics, antidiabetics, immunosuppressives, antidepressants, antiobesity agents, antihyperglycemics, hypoglycemics, nutritional agents and supplements, growth supplements, fats, ophthalmics, antienteritis agents, electrolytes and diagnostic agents.

Examples of active agents useful in this form of the invention include prochlorperazine edisylate, ferrous sulfate, albuterol, aminocaproic acid, mecamylamine hydrochloride, procainamide hydrochloride, amphetamine sulfate, methamphetamine hydrochloride, benzphetamine hydrochloride, isoproterenol sulfate, phenmetrazine hydrochloride, bethanechol chloride, methacholine chloride, pilocarpine hydrochloride, atropine sulfate, scopolamine bromide, isopropamide iodide, tridihexethyl chloride, phenformin hydrochloride, metformin, methylphenidate hydrochloride, theophylline cholinate, cephalexin hydrochloride, diphenidol, meclizine hydrochloride, prochlorperazine maleate, phenoxybenzamine, thiethylperazine maleate, anisindione, diphenadione erythrityl tetranitrate, digoxin, isoflurophate, acetazolamide, nifedipine, methazolamide, bendroflumethiazide, chlorpropamide, glipizide, glyburide, gliclazide, tobutamide, chlorproamide, tolazamide, acetohexamide, troglitazone, orlistat, bupropion, nefazodone, tolazamide, chlormadinone acetate, phenaglycodol, allopurinol, aluminum aspirin, methotrexate, acetyl sulfisoxazole, hydrocortisone, hydrocorticosterone acetate, cortisone acetate, dexamethasone and its derivatives such as betamethasone, triamcinolone, methyltestosterone, 17-β-estradiol, ethinyl estradiol, ethinyl estradiol 3-methyl ether, prednisolone, 17-β-hydroxyprogesterone acetate, 19-nor-progesterone, norgestrel, norethindrone, norethisterone, norethiederone, progesterone, norgesterone, norethynodrel, terfandine, fexofenadine, aspirin, acetaminophen, indomethacin, naproxen, fenoprofen, sulindac, indoprofen, nitroglycerin, isosorbide dinitrate, propranolol, timolol, atenolol, alprenolol, cimetidine, clonidine, imipramine, levodopa, selegiline, chlorpromazine, methyldopa, dihydroxyphenylalanine, calcium gluconate, ketoprofen, ibuprofen, cephalexin, erythromycin, haloperidol, zomepirac, ferrous lactate, vincamine, phenoxybenzamine, diltiazem, milrinone, captropril, mandol, quanbenz, hydrochlorothiazide, ranitidine, flurbiprofen, fenbufen, fluprofen, tolmetin, alclofenac, mefenamic, flufenamic, difuninal, nimodipine, nitrendipine, nisoldipine, nicardipine, felodipine, lidoflazine, tiapamil, gallopamil, amlodipine, mioflazine, lisinopril, enalapril, captopril, ramipril, enalaprilat, famotidine, nizatidine, sucralfate, etintidine, tetratolol, minoxidil, chlordiazepoxide, diazepam, amitriptyline, and imipramine, and pharmaceutical salts of these active agents. Further examples are proteins and peptides which include, but are not limited to, cyclosporins such as cyclosporine A, insulin, colchicine, glucagon, thyroid stimulating hormone, parathyroid and pituitary hormones, calcitonin, renin, prolactin, corticotrophin, thyrotropic hormone, follicle stimulating hormone, chorionic gonadotropin, gonadotropin releasing hormone, bovine somatotropin, porcine somatropin, oxytocin, vasopressin, prolactin, somatostatin, lypressin, pancreozymin, luteinizing hormone, LHRH, interferons, interleukins, growth hormones such as human growth hormone, bovine growth hormone and porcine growth hormone, fertility inhibitors such as the prostaglandins, fertility promoters, growth factors, and human pancreas hormone releasing factor.

In this form, the present invention is particularly useful to deliver active agents that are poorly absorbed in the lower gastrointestinal tract, but well absorbed in the upper gastrointestinal tract (i.e., the small intestine) or active agents that exhibit poor solubility such that the increased retention time in the stomach allows for a greater quantity of active agent to dissolve from the dosage form than would otherwise be dissolved. Typically, antiviral, antifungal and antibiotic agents, e.g. sulfonamides, quinolones, penicillins, cephalosporins, aminoglycosides, and tetracyclines, are representative classes of agents for which the invention is particularly useful. Such antibiotic agents may include, for example, β-lactam antibiotics, vancomycin, clidamycin, erthromycin, trimethoprimsulfamethoxaazole, rifampin, ciprofloxacin, amoxicillin, clindamycin, ceftriaxone, cefotaxime, chloramphenicol, clindamycin, cefoxitin, doxyclycline, spectinomycin, ofloxacin, rifampin, minocycline, doxycycline, aztreonam, imipenem, meropenem, nitrofurantoin, azithromycin, atovaquone, trimetrexate, dapsone, primaquin, trimetrexate, ketoconazole, floconazole, amphotericin B, itraconazole, trifluridine, foscarnet, zidovudine amantadine, interferon alfa, sulfonamides such as sulfisoxazole, sulfadiazine, and sulfasalazine, quinolones and fluoroquinolones such as, for example, cinoxacin, forfloxacin, diprofloxacin, ofloxacin, spardlosxacin, lomefloxacin, fleroxacin, pefloxacin and amifloxacin, gentamicin, tobramycin, amikacin,netilmicin, kanamycin,and neomycin. Representative antiviral agents include acyclovir, famciclovir, foscarnet, ganciclovir, idoxuridine, sorivudine, trifluridine, valacylcovir, vidarabine, didanosine, stavudine, zalcitabine, zidovudine, amantadine, interferons, e.g., interfon alpha, ribavirin, rimantadine, nucleoside RT inhibitors, such as lamivudine and adeforvir, non-nucleoside inhibitors such as nevrapine, delavairidine, lviride, saquinavir and indinavir, nucleoside DNAp inhibitors such as famciclovir, fialuridine, cidofovir and lobucavir, antisense oligonucleotides such as afovirsen, receptor decoys such as sICAM-1, capsid binding agents such as pirodavir, and neuraminidase inhibitors such as GG167.

The dosage form of the embodiment of the invention is useful for the delivery of oral, hypoglycermic agents, such as the sulfonylureas, e.g., tolbutamide, glyburide, glipizide and gliclazide, the biguamides, e.g., metformin and phenformin, and the thiazolidinediones, e.g. ciglitazone and pioglitazone. Also, immunosupressives, such as, for example, cyclosporine, tacrolimus (Fk506) and micophenolate mofetil.

Specific examples of active agents that are readily absorbed in the upper gastrointestinal tract relative to the lower gastrointestinal tract are acyclovir, ganciclovir, cimetidine, ranitidine, captopril, methyldopa, selegiline and the like. Specific examples of active agents that exhibit poor solubility in water are diphenidol, meclizine hydrochloride, prochloperazine maleate, phenoxybenzamine, triethylperazine maleate, anisindone, diphenadione erythrityl tetranitrate, digoxin, isofllurophate, acetazolamide, methazolamide, bendroflumethiazide, chlorpropamide, tolazamide, chlormadionone acetate, phenaglycodol, allopurinol, alluminum aspirin, methotrexate, acetyl sulfisoxazole, erythromyciin, progestins, esterogenic, progestational corticosteroids, hydrocortisone, hydrocorticosterone acetate, cortisone acetate, tramcinolone, methyltesterone, 17-beta-estradiol, ethinyl estradiol, prazosin hydrochloride, ethinyl estradiool 3-methyl ether, pednisolone, 17-alpha-hydroxyprogesterone acetate, 19-norprogesterone, norgestrel, norethindrone, progesterone, norgesterone, norethlynodrel, and the like.

Retention of the dosage form of the present invention in the stomach for a prolonged period of time makes it especially useful for the localized treatment of gastric acidity, gastrointestinal disorders, such as duodenal ulcers, peptic ulcers and chronic gastritis, and the eradication of Helicobacter pylori. Representative active agents for such uses include cimetidine, rantitidine, famotidine, nizatidine, zolentine, omeprazole, lansoprazole and active agents useful for the treatment of Helicobacter pylori, such as metronidazole, timidazole, amoxicillin, clarithromycin, minocycline and tetracycline.

While for reasons of efficacy, safety, economy, convenience and/or efficiency it may be desirable to utilize a single active agent in the active agent formulation, it is to be understood that more than one active agent may be incorporated into the active agent formulation in a device of this form of the invention, and that the use of the term "agent" or "active agent" in no way excludes the use of two or more such agents or active agents. The agents can be in various forms, such as uncharged molecules, components of molecular complexes or nonirritating, pharmacologically acceptable salts. Also, simple derivatives of the agents (such as ethers, esters, amides, etc) which are easily hydrolyzed by body pH, enzymes, etc, can be employed. Combinations of two or more active agents can optionally be co-delivered, simultaneously or sequentially from the dosage form of this invention.

The dosage form of this form of the invention may include additional ingredients, such as, for example, a buffer or other agents for controlling pH in the stomach or elsewhere in the gastrointestinal tract, an agent or agents for delaying onset of the housekeeping wave, preferably locally delivered by the dosage form in amounts not resulting in any substantial systemic effect to the subject, as for example, anticholenergic agents such as propantheline, and other agents including, but not limited to, methylcellulose, guar gum, fats such as triglyceride esters, e.g., triethanol myristate, fatty acids of 10-15 carbon atoms, and the like, a viscosity regulating vehicle, a surfactant, a dye, a permeation enhancer, a proteinase inhibitor, or other formulation ingredients and additives, as are known in the art.

The present dosage form may also include minor amounts of low molecular weight polymers, which serve useful functions in tablet formation, for example, to improve the tablet cohesiveness after compression or to improve the physical or chemical stability of the dosage form. These polymers are added at quantities less than 10% by weight and preferably less that 5% by weight of the tablet. Examples of such polymers include hydroxypropyl methyl cellulose having molecular weights of less that 20,000 grams per mole, methycellulose having a molecular weight of less than 20,000 grams per mole, polyvinyl pyrrolidone having a molecular weight of less than 50,000 grams per mole, and the like.

The amount of active agent employed in the present dosage form will be that amount necessary to deliver a therapeutically effective amount of the agent to achieve the desired therapeutic result. In practice, this will vary widely depending upon the particular agent, the degree of active agent absorption, the severity of the condition, and the desired therapeutic effect. Thus, it is not practical to define.a particular range for the therapeutically effective amount of each active agent incorporated into the device. Such ranges can easily be determined by one skilled in the art using conventional methods, for example from dose ranging and plasma level studies. Any references to specific quantities of active agent or specific dose ranges of active agent herein are intended to include the amount or amounts of active agent specified and bioequivalents thereof.

When the delivery device of this form of the invention is being used to substitute for one or more doses of an active agent presented in a conventional dosage form that is usually prescribed for multiple dosing during a predetermined period, the sum of the amounts of active agent present in the multiple doses of the conventional dosage form for use in the period may be used to determine an upper limit on the of the amount of active agent to be included in the device of this invention. For example, if the conventional dosage form contains 200 mg of active agent and is to be administered every 3 hours, a dosage form of this invention may be prepared for administration every 6 hours, and that dosage form may contain 400 mg of active agent which will be delivered over the 6 hour period.

However, when compliance with multiple dosing is a problem, the advantage of administering the dosage forms of the invention at fewer times throughout a twenty-four hour period may provide incentive to incorporate greater amounts of active agent, where such greater amounts do not have any deleterious effects. The specific amount of active agent to be included in the dosage form of the invention can easily be determined by routine dosage studies that compare the blood plasma active agent levels of subjects with conventional dosing and the dosage form of this invention.

The dosage forms of this form of the invention can conveniently release active agent in a controlled and sustained manner over a prolonged period. Typically, active agent will be released from the dosage form at a rate that releases a therapeutically effective amount of active agent to the subject over a substantial portion of the period between administration of the dosage forms. Typically, release will occur over 40% of the period between repeated administration of the dosage form, more preferably at least over 60% of the period, and most preferably over 80% of the period.

In an especially preferred embodiment, the invention comprises porous particles in which is sorbed liquid, active agent formulation dispersed in a polymer composition having from about 10 weight percent to about 50 weight percent of a water-soluble, high molecular weight polyethylene oxide polymer and from about 10 weight percent to about 60 weight percent of a water-insoluble hydroxypropyl cellulose polymer. The polyethylene oxide polymer has a molecular weight of between about 100,000 and 10,000,000 grams per mole. The hydroxypropyl cellulose polymer preferably has a hydroxypropyl content of between about 8-15 weight percent, and most preferably between about 10-13 weight percent.

The following examples are illustrative of the gastric retentive embodiment of the present invention.

### PREPARATION 1

A general procedure for preparing the dosage forms of the present invention is described below with the exemplary active agent being cyclosporin. Various other materials or additives as described herein may be used in place of or in addition to the specific materials provided in this description in the same or other proportions based on the desired final characteristics of the dosage forms to be fabricated.

A self-emulsifying drug solution comprising, in weight percent, 2% cyclosporin, 49% polyoxyl 35 castor oil (Cremophor EL, BASF Corporation) and 49% distilled acetylated monoglyceride (Myvacet 9-45) is prepared. Then, 15 g of the solution is blended with 35 g of porous calcium hydrogen phosphate (FujiCalin) in a mixing vessel. 3.6 Grams of the gel-forming polymer polyethylene oxide, having a number average molecular weight of approximately 7 million grams per mole, is separately screened through a mesh having 40 wires per inch. The polyethylene oxide is supplied under the trade name Polyox® grade 303 as manufactured by Union Carbide Corporation, Danbury, Connecticut. The sized active agent and polymer are mixed. Then, 8.25 grams of a hydroattractant water-insoluble polymer, hydroxypropyl cellulose having a hydroxypropyl content of 10-13 weight percent and an average fiber particle size of 50 microns, is sieved through the 40-mesh screen and blended into the mixture. The hydroxypropyl cellulose is supplied as Low-Substituted Hydroxypropyl Cellulose grade 11 as manufactured by Shin-Etsu Chemical Company, Ltd., Tokyo, Japan. Anhydrous ethyl alcohol, specially denatured formula 3A, i.e., ethanol denatured with 5 volume percent methanol, is added to the mixture with stirring until a uniformly damp mass formed. The damp mass is extruded through a screen having 20 wires per inch. The extrudate is then allowed to air dry at room temperature overnight. After drying, the resulting extrudate is passed again through a 20-mesh sieve, forming granules. 0.15 Grams of the tableting lubricant, magnesium stearate, is passed through a sieve having 60 wires per inch. The sized 60-mesh lubricant is then tumbled into the granules to produce the finished granulation.

Portions of the resulting granulation are weighed and compacted with caplet-shaped tooling on a Carver press at pressure head of 1.5 tons. Each tablet will contain a target weight of active agent and be of a suitable size to be orally administered. The shape of the tablet may have approximately cylindrical proportions, and the diameter may be approximately 7.5 millimeters (mm) and the length approximately 22 mm.

A tube of polyolefin material having an outside diameter of about 0.1 mm larger than the diameter of the tablet and having a wall thickness of 0.25 mm is sliced with a razor to produce rings. The width of each ring is approximately 3 mm. One ring is then press fitted onto each caplet such that the ring, or band, is located approximately at the midpoint of the length of the caplet. This step completes the fabrication procedure for the dosage form.

### ASSAY

The dosage forms fabricated in Preparation 1 may be placed in a beaker of simulated gastric fluid, as specified in U.S. Pharmacopedia/National Formulary 23/18, having a pH of approximately 1.2 and a maintained temperature of 37° C to determine release of active agent over time. Additionally, the swollen size of the dosage form may be removed and measured for dimensional change. A swollen device has the general appearance of the dosage form shown in Figure 3.

### EXAMPLE 6

Equivalent amounts of the following polymers may be substituted for the polyethylene oxide in Preparation 1 (all molecular weights are number average molecular weights in grams per mole): hydroxypropyl cellulose (MW: 1,000,000), hydroxypropyl methyl cellulose (MW: 254,000), hydroxyethyl cellulose (MW: 1,300,000), sodium carboxy methylcellulose (MW: 700,000), calcium carboxymethyl cellulose (MW: 700,000), methyl cellulose (MW: 135,000), and polyvinyl alcohol (Elvanol® HV), and dosage forms with a polyethylene band are fabricated to the same dimensions as described in Preparation 1 with equivalent quantities of the active agents acyclovir, ganciclovir, minocycline metformin and cyclosporin. The prepared dosage forms are retained in the stomach of a dog for a prolonged retention period and deliver the active agents over a prolonged period of time.

### EXAMPLE 7

Dosage forms containing equivalent quantities of the active agents of EXAMPLE 7 are prepared according to the procedures in Preparation 1, except that the nonwater soluble hydroattractant used is, respectively, microcrystalline cellulose (Avicel), cross-linked sodium or calcium carboxymethyl cellulose, cellulose fiber (Solka-Floc, Elcema, Arbocel), cross-linked polyvinyl pyrrolidone (Polyplasdone XL), cross-linked Amberlite resin, alginates (Satialgine), colloidal magnesium-aluminum silicate (Veegum), corn starch granules, rice starch granules, potato starch granules, and sodium carboxymethyl starch (Expotab, Primojel). The prepared dosage forms are retained in the stomach of a subject and deliver active agent over a prolonged period of time.

### EXAMPLE 8

The following active agents are substituted, in the quantities indicated in the parentheses following each active agent listed, for the quantity of active agent in Example 6: cimetidine (400 mg; 800 mg, 1200 mg, 1600 mg), ranitidine (150 mg; 200 mg, 300 mg), captopril (12.5 mg; 25 mg; 50 mg; 100 mg, 150 mg), methyldopa (125; 250; 500 mg), and selegiline (5 mg, 10 mg) and the dosage forms are prepared in the same manner as described in Example 6. The prepared dosage forms are retained in the stomach of a subject and deliver active agent over a prolonged period of time.

### EXAMPLE 9

Dosage forms of this invention containing metformin are fabricated according to the procedures of Preparation 1, except that the tablet is inserted into a size "00" hard gelatin capsule before banding. The band is applied by a printing process using the methods and compositions described in U.S. Patent 5,534,263, incorporated herein by reference, where the band material is ethyl acrylate/methyl methacrylate 70:30 copolymer (Eudragit NE 30 D, Rohm Tech). The resulting dosage form is smooth and easy to swallow.

### EXAMPLE 10

A gastric platform dosage form for an insoluble drug, metformin free base, is prepared in accordance with the procedures of Preparation 1 by substituting a drug/particle/Polyox mass consisting of 2% metformin base, 38% corn oil, 40% Neusilin and 20% Polyox 303 for the sized active agent-polymer mixture. Then the hydroattractant is added to the mixture and the subsequent steps repeated for this formulation, forming a tableted core.

A solution for use in film coating the tablets is prepared by stirring 40 grams of methyl cellulose (Methocel A15 LV Premium supplied by Dow Chemical, Midland Michigan) and 10 grams of sorbitol 950 grams of purified water at room temperature. The mixture is then chilled overnight at 9° centigrade to complete dissolution. The tablets from above are transferred to a pharmaceutical coating pan spray coated with the solution in a current of warmed air until a dry film coating is deposited onto each tablet.

An aqueous dispersion for use in banding the tablets is prepared by dissolving 30 grams of triacetin in 174.75 grams of ethyl acrylate methylmethacrylate 70:30 copolymer aqueous dispersion (Eudragit® NE40D supplied by Rohm Corporation, Darmstadt, West Germany). Then, 0.1 grams of anti-foam agent (Simethicone Q7-2587, Dow Chemical, Midland, Michigan) is blended into the mixture. This formed the final composition of the banding dispersion.

The film coated tablets from above are then banded by applying a the above banding dispersion in a transfer printing process using a printing wheel having a width of approximately 100 mils (2.54 mm). The banded system is then dried in warm air to remove the water from the aqueous dispersion, leaving a single band located in the center of the caplet having a width of approximately 120 mils (3.05 mm) and a weight of approximately 21 mg. The entire banded system is then overcoated with more of the aqueous-based film coat solution using the formulation and process as described above until a film coat weight of approximately 30 mg is applied. The dosage forms so prepared are retained in the stomach of a dog and deliver active agent over a prolonged period of time.

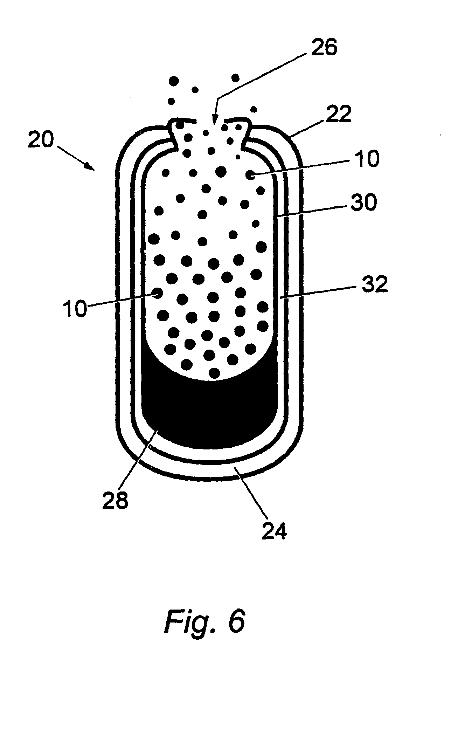

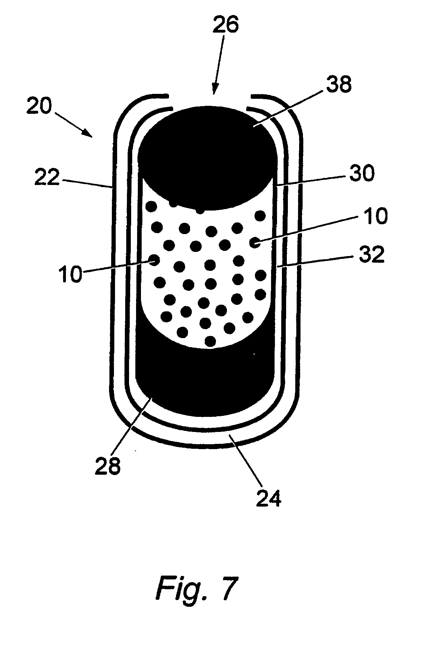

In relation to the third embodiment of the present invention exemplified by Figures 6 and 7, they are best understood by reference to the following definitions, the drawings and exemplary disclosure provided herein.

### Definitions

By "active agent", "drug", or "compound", which are used interchangeably herein, is meant an agent , drug, compound, composition of matter or mixture thereof which provides some physiological, psychological, biological, or pharmacological, and often beneficial, effect when in the environment of use.

By "uniform rate of release" or "uniform release rate" is meant a rate of release of the active agent from a dosage form that does not vary positively or negatively by more than 30% from the mean rate of release of the active agent over a prolonged period of time, as determined in a USP Type 7 Interval Release Apparatus. Preferred uniform rates of release will vary by not more than 25% (positively or negatively) from the mean rate of release determined over a prolonged period of time.

By "prolonged period of time" or "prolonged period" is meant a continuous period of time of 4 hours or more, more typically 6 hours or more.

By "dosage form" is meant a pharmaceutical composition or device comprising an active pharmaceutical agent, the composition or device optionally containing inactive ingredients, such as pharmaceutically-acceptable carriers, excipients, suspension agents, surfactants, disintegrants, binders, diluents, lubricants, stabilizers, antioxidants, osmotic agents, colorants, plasticizers, and the like, that are used to manufacture and deliver active pharmaceutical agents.

By "pharmaceutically-acceptable acid addition salt" or "pharmaceutically-acceptable salt", which are used interchangeably herein, are meant those salts in which the anion does not contribute significantly to the toxicity or pharmacological activity of the salt, and, as such, they are the pharmacological equivalents of the bases of the compounds to which they refer. Examples of pharmaceutically acceptable acids that are useful for the purposes of salt formation include but are not limited to hydrochloric, hydrobromic, hydroiodic, citric, acetic, benzoic, mandelic, phosphoric, nitric, mucic, isethionic, palmitic, and others.

By "sustained release " is meant continuous release of active agent to an environment of use over a prolonged period.

By "pulsatile release" is meant release of an active agent to an environment of use for one or more discrete periods of time preceded or followed by (i) at least one discrete period of time in which the active agent is not released, or (ii) at least one period of time in which another, different active agent is released. Pulsatile release is meant to include delayed release of active agent following administration of the dosage form and release in which one or more pulses of active agent are released over a period of time.

By "steady state" is meant the condition in which the amount of drug present in the blood plasma of a subject does not vary significantly over a prolonged period of time.

By "release rate assay" is meant a standardized assay for the determination of a compound using a USP Type 7 interval release apparatus substantially in accordance with the description of the assay contained herein. It is understood that reagents of equivalent grade may be substituted in the assay in accordance with generally-accepted procedures. Also, different fluids such as artificial gastric fluid or artificial intestinal fluid may be used to evaluate release characteristics in environments characterized by different pH values.

By "liquid, active agent formulation" is meant that the active agent is present in a composition that is miscible with or dispersible in the fluids of the environment of use, or is able to flow or diffuse from the pores of the particles into the environment of use.

The formulation may be neat, liquid active agent, or a solution, suspension, slurry, emulsion, self-emulsifying composition, colloidal dispersion or other flowable composition in which the active agent is present.

The active agent may be accompanied by a suspension agent, antioxidant, emulsion former, protecting agent, permeation enhancer and the like. The amount of an active agent in a dosage form generally is about 0.05 ng to 5 g or more, with individual dosage forms comprising, for example, 25 ng, 1 mg, 5 mg, 10 mg, 25 mg, 100 mg, 250 mg, 500 mg, 750 mg, 1.0 g, 1.2 g, and the like, of active agent. The system typically can be administered once, twice or thrice daily for pharmaceutical applications, or more or less as required by the particular application. In agricultural applications, systems typically will be applied at longer intervals, such as weekly, monthly, seasonally or the like.

One of the most suitable devices for the controlled release of liquid active agent formulations in accordance with this form of the invention is that having a semipermeable wall defining a compartment, an expandable push layer and a drug layer in the compartment, and an exit orifice formed in the dosage form to permit the drug layer to be dispensed. Within the drug layer is a carrier in which is dispersed a plurality of porous particles in which the liquid, active agent has been sorbed. As the push layer expands, the carrier comprising the drug layer will be forced from the dosage form substantially in the dry state where it will erode and release the porous particles containing the liquid, active agent formulation. After release, the liquid active agent formulation will be immediately available the environment of use in the liquid state, and the porous particles will themselves disintegrate or erode and further release the active agent formulation.

When manufacturing such dosage forms, a common practice is to fabricate a compressed tablet comprising the drug layer and the push layer. Typically, the drug layer composition, conveniently in granulated or powdered form, is compressed in a die cavity of a vertical tabletting press. Then the push layer composition, also conveniently in granular or powdered form, is placed in the die cavity above the drug layer and compressed as well to form a bilayer tablet. During the compression or compacting step of the drug layer, the porous particles should be sufficiently resistant to the compressive forces so as not to be crushed or pulverized to any significant extent and prematurely release the liquid, active agent formulation from the porous particles.

Materials useful for sorbing the liquid active agent formulations have already been described herein. Other absorptive materials may be substituted for the foregoing. For example, powders of microcrystalline cellulose sold under the tradenames Avicel (FMC Corporation) and Elcema (Degussa) and porous agglomerated silicon dioxide, sold under the tradenames Cab-O-Sol (Cabot) and Aerosil (Degussa), may be used.

The liquid, active agent formulation may be in any form that can be dispensed from the inside of the pores as the drug layer disintegrates in the environment of use. The formulation, for example, may be neat, liquid active agent, liquid active agent in a solution, suspension, emulsion or self-emulsifying composition, or the like, or a liposomal solution or solid formulation, or solid active agent in solution, suspension or slurry. Optionally other dosage-forming ingredients, such as an anti-oxidant, a suspending agent, a surface active agent, and the like may be present in the liquid, active agent formulation. The liquid, active agent formulation will be released in a form most suitable to provide active agent to the site of delivery in a state in which it may be rapidly absorbed in the environment of use to provide its beneficial action with minimum delay once delivered to the absorption site.

It often is desirable to provide the dosage form with a flow-promoting layer or lubricant that facilitates complete release of the drug layer from the compartment formed by the semipermeable wall since the formed bilayer tablet may be formed with surface irregularities that impede the release of the drug layer from the dosage form and sometimes results in incomplete release of the drug layer.

Dosage forms of this form of the invention release effective amounts of active agent to the patient over a prolonged period of time and often provide the opportunity for less frequent dosing, including once-a-day dosing, than previously required for immediate release compositions. The dosage forms of this invention comprise a composition containing a liquid, active agent formulation contained in porous particles dispersed in a bioerodible carrier.

Active agents include, inter alia, foods, food supplements, nutrients, drugs, antiacids, vitamins, microorganism attenuators and other agents that provide a benefit in the environment of use and may be dissolved, suspended or otherwise dispersed in a liquid to form a liquid, active agent formulation. Active agents include any physiologically or pharmacologically active substance that produces a localized or systemic effect or effects in animals, including warm blooded mammals, humans and primates; domestic household or farm animals such as cats, dogs, sheep, goats, cattle, horses and pigs; laboratory animals such as mice, rats and guinea pigs; zoo and wild animals; and the like. Active agents that can be delivered include inorganic and organic compounds as previously discussed which act on the peripheral nerves, etc.

Suitable active agents and examples of particular useful active agents are as previously discussed for the second embodiment exemplified hereinbefore.

The method of this invention may be applied generally to liquid formulations such as contained in commercially-available dosage forms as previously described and exemplified herein.

The dosage form may also contain a chelating agent as previously discussed, either combined with the liquid, active agent formulation in the porous particles, or incorporated into the drug layer in which the porous particles are dispersed.

A dosage form 20 intended for continuous, zero order release of the active agent is illustrated in Figure 6. As can be seen therein, the dosage form 20 comprises a wall 22 defining a cavity 24. Wall 22 is provided with an exit orifice 26. Within cavity 24 and remote from the exit orifice 26 is a push layer 28. A drug layer 30 is located within cavity 24 adjacent exit orifice 26. A plurality of porous particles 10 is dispersed in carrier 18 within the cavity 24 to form the drug layer 30. An optional, flow-promoting layer 32, the function of which will be described and which may be formed as a secondary wall, extends between drug layer 30 and the inner surface of wall 22. An orifice 26 is provided at one end of dosage form 20 to permit expression of the drug layer 30 from the dosage form upon expansion of push layer 28.

The wall 22 is formed to be permeable to the passage of an external fluid, such as water and biological fluids, and it is substantially impermeable to the passage of active agent, osmagent, osmopolymer and the like. As such, it is semipermeable. The selectively semipermeable compositions used for forming the wall are essentially nonerodible and they are insoluble in biological fluids during the life of the dosage form. Wall 22 need not be semipermeable in its entirety, but at least a portion of wall 22 should be semipermeable to allow fluid to contact or communicate with push layer 28 such that push layer 28 imbibes fluid during use. Specific materials for the fabrication of semipermeable wall 22 are well known in the art, and representative examples of such materials are described later herein.

Secondary wall 32, which functions as the flow-promoting layer or lubricant, is in contacting position with the inner surface of the semipermeable wall 22 and at least the external surface of the drug layer that is opposite wall 22; although the secondary wall 32 may, and preferably will, extend to, surround and contact the external surface of the push layer. Wall 32 typically will surround at least that portion of the external surface of the drug layer that is opposite the internal surface of wall 22. Secondary wall 32 may be formed as a coating applied over the compressed core comprising the drug layer and the push layer. The outer semipermeable wall 22 surrounds and encases the inner, secondary wall 32. Secondary wall 32 is preferably formed as a subcoat of at least the surface of the drug layer 30, and optionally the entire external surface of the compacted drug layer 30 and the push layer 28. When the semipermeable wall 22 is formed as a coat of the composite formed from the drug layer 30, the push layer 28 and the secondary wall 32, contact of the semipermeable wall 22 with the inner coat is assured.

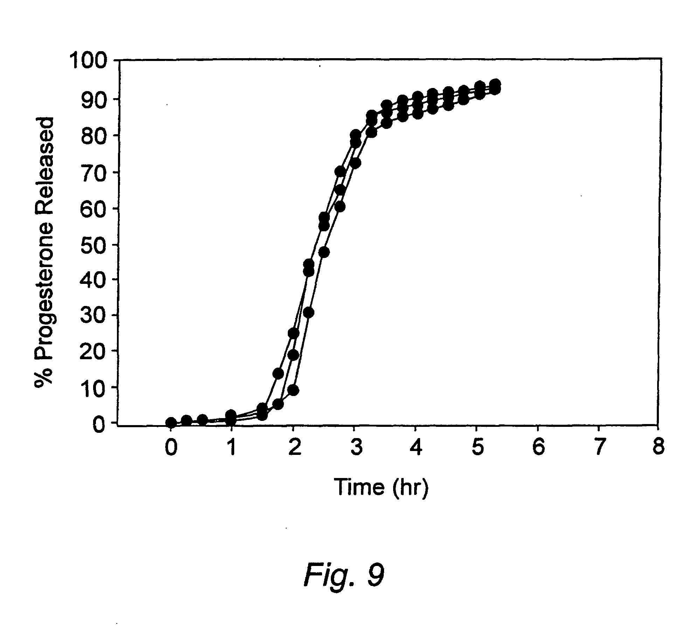

Figure 7 illustrates another form of the invention wherein the dosage form 20 includes a placebo layer 38 which serves to delay release of particles 10 the environment of use. The other components of the dosage form 20 are substantially the same as those described with reference to Figure 6, and like components are designated with the same reference numerals. The extent of the delay that may be afforded by the placebo layer will in part depend on the volume of the placebo layer 38 which has to be displaced by the push layer 28 as it imbibes fluid and expands. Figures 9-13 illustrate different periods of delay that may be obtained by varying the placebo layer 38 when delivering a representative compound progesterone. The dosage forms for which the results in Figures 9-13 are illustrated correspond to those described in Examples 12-16, respectively. Delays of 2 hours to 10 hours are illustrated.

Representative polymers for forming wall 22 comprise semipermeable homopolymers, semipermeable copolymers, and the like. Such materials comprise cellulose esters, cellulose ethers and cellulose ester-ethers. The cellulosic polymers have a degree of substitution (DS) of their anhydroglucose unit of from greater than 0 up to 3, inclusive. Degree of substitution (DS) means the average number of hydroxyl groups originally present on the anhydroglucose unit that are replaced by a substituting group or converted into another group. The anhydroglucose unit can be partially or completely substituted with groups such as acyl, alkanoyl, alkenoyl, aroyl, alkyl, alkoxy, halogen, carboalkyl, alkylcarbamate, alkylcarbonate, alkylsulfonate, alkysulfamate, semipermeable polymer forming groups, and the like, wherein the organic moieties contain from one to twelve carbon atoms, and preferably from one to eight carbon atoms.

The semipermeable compositions typically include a member selected from the group consisting of cellulose acylate, cellulose diacylate, cellulose triacylate, cellulose acetate, cellulose diacetate, cellulose triacetate, mono-, di- and tri-cellulose alkanylates, mono-, di-, and tri-alkenylates, mono-, di-, and tri-aroylates, and the like. Exemplary polymers include cellulose acetate having a DS of 1.8 to 2.3 and an acetyl content of 32 to 39.9%; cellulose diacetate having a DS of 1 to 2 and an acetyl content of 21 to 35%; cellulose triacetate having a DS of 2 to 3 and an acetyl content of 34 to 44.8%; and the like. More specific cellulosic polymers include cellulose propionate having a DS of 1.8 and a propionyl content of 38.5%; cellulose acetate propionate having an acetyl content of 1.5 to 7% and an acetyl content of 39 to 42%; cellulose acetate propionate having an acetyl content of 2.5 to 3%, an average propionyl content of 39.2 to 45%, and a hydroxyl content of 2.8 to 5.4%; cellulose acetate butyrate having a DS of 1.8, an acetyl content of 13 to 15%, and a butyryl content of 34 to 39%; cellulose acetate butyrate having an acetyl content of 2 to 29%, a butyryl content of 17 to 53%, and a hydroxyl content of 0.5 to 4.7%; cellulose triacylates having a DS of 2.6 to 3, such as cellulose trivalerate, cellulose trilamate, cellulose tripalmitate, cellulose trioctanoate and cellulose tripropionate; cellulose diesters having a DS of 2.2 to 2.6, such as cellulose disuccinate, cellulose dipalmitate, cellulose dioctanoate, cellulose dicaprylate, and the like; and mixed cellulose esters, such as cellulose acetate valerate, cellulose acetate succinate, cellulose propionate succinate, cellulose acetate octanoate, cellulose valerate palmitate, cellulose acetate heptanoate, and the like. Semipermeable polymers are known in U.S. Patent No. 4,077,407, and they can be synthesized by procedures described in Encyclopedia of Polymer Science and Technology, Vol. 3, pp. 325-354 (1964), Interscience Publishers Inc., New York, NY.

Additional semipermeable polymers for forming the outer wall 22 comprise cellulose acetaldehyde dimethyl acetate; cellulose acetate ethylcarbamate; cellulose acetate methyl carbamate; cellulose dimethylaminoacetate; semipermeable polyamide; semipermeable polyurethanes; semipermeable sulfonated polystyrenes; cross-linked selectively semipermeable polymers formed by the coprecipitation of an anion and a cation, as disclosed in U.S. Patents Nos. 3,173,876; 3,276,586; 3,541,005; 3,541,006 and 3,546,142; semipermeable polymers, as disclosed by Loeb, et al. in U.S. Patent No. 3,133,132; semipermeable polystyrene derivatives; semipermeable poly(sodium styrenesulfonate); semipermeable poly(vinylbenzyltrimethylammonium chloride); and semipermeable polymers exhibiting a fluid permeability of 10⁻⁵ to 10⁻² (cc. mil/cm hr.atm), expressed as per atmosphere of hydrostatic or osmotic pressure differences across a semipermeable wall. The polymers are known to the art in U.S. Patents Nos. 3,845,770; 3,916,899 and 4,160,020; and in Handbook of Common Polymers, Scott and Roff (1971) CRC Press, Cleveland, OH.

Wall 22 also can comprise a flux regulating agent. The flux regulating agent is a compound added to assist in regulating the fluid permeability or flux through wall 22. The flux regulating agent can be a flux enhancing agent or a decreasing agent. The agent can be preselected to increase or decrease the liquid flux. Agents that produce a marked increase in permeability to fluid such as water, are often essentially hydrophilic, while those that produce a marked decrease to fluids such as water, are essentially hydrophobic. The amount of regulator in the wall when incorporated therein generally is from about 0.01% to 20% by weight or more. The flux regulator agents in one embodiment that increase flux include polyhydric alcohols, polyalkylene glycols, poilyalkylenediols, polyesters of alkylene glycols, and the like. Typical flux enhancers include polyethylene glycol 300, 400, 600, 1500, 4000, 6000 and the like; low molecular weight gylcols such as polypropylene glycol, polybutylene glycol and polyamylene glycol: the polyalkylenediols such as poly(1,3-propanediol), poly(1,4-butanediol), poly(1,6-hexanediol), and the like; aliphatic diols such as 1,3-butylene glycol, 1,4-pentamethylene glycol, 1,4-hexamethylene glycol, and the like; alkylene triols such as glycerine, 1,2,3-butanetriol, 1,2,4-hexanetriol, 1,3,6-hexanetriol and the like; esters such as ethylene glycol dipropionate, ethylene glycol butyrate, butylene glucol dipropionate, glycerol acetate esters, and the like. Representative flux decreasing agents include phthalates substituted with an alkyl or alkoxy or with both an alkyl and alkoxy group such as diethyl phthalate, dimethoxyethyl phthalate, dimethyl phthalate, and [di(2-ethylhexyl) phthalate], aryl phthalates such as triphenyl phthalate, and butyl benzyl phthalate; insoluble salts such as calcium sulphate, barium sulphate, calcium phosphate, and the like; insoluble oxides such as titanium oxide; polymers in powder, granule and like form such as polystyrene, polymethylmethacrylate, polycarbonate, and polysulfone; esters such as citric acid esters esterfied with long chain alkyl groups; inert and substantially water impermeable fillers; resins compatible with cellulose based wall forming materials, and the like.

Other materials that can be used to form the wall 22 for imparting flexibility and elongation properties to the wall, for making wall 22 less-to-nonbrittle and to render tear strength, include phthalate plasticizers such as dibenzyl phthalate, dihexyl phthalate, butyl octyl phthalate, straight chain phthalates of six to eleven carbons, di-isononyl phthalte, di-isodecyl phthalate, and the like. The plasticizers include nonphthalates such as triacetin, dioctyl azelate, epoxidized tallate, tri-isoctyl trimellitate, tri-isononyl trimellitate, sucrose acetate isobutyrate, epoxidized soybean oil, and the like. The amount of plasticizer in a wall when incorporated therein is about 0.01% to 20% weight, or higher.

The drug layer 30 comprises a composition formed of a liquid active agent formulation absorbed in porous particles, the preferred characteristics of the particles being described elsewhere herein, and a carrier, such as a hydrophilic polymer. The hydrophilic polymer provides a hydrophilic polymer composition in the drug layer that may contribute to the uniform release rate of active agent and controlled delivery pattern by controlling the rate of release of the porous particles containing the liquid, active agent formulation from the dosage form. Representative examples of these polymers are poly(alkylene oxide) of 100,000 to 750,000 number-average molecular weight, including poly(ethylene oxide), poly(methylene oxide), poly(butylene oxide) and poly(hexylene oxide); and a poly(carboxymethylcellulose) of 40,000 to 400,000 number-average molecular weight, represented by poly(alkali carboxymethylcellulose), poly(sodium carboxymethylcellulose), poly(potassium carboxymethylcellulose) and poly(lithium carboxymethylcellulose). The drug composition can comprise a hydroxypropylalkylcellulose of 9,200 to 125,000 number-average molecular weight for enhancing the delivery properties of the dosage form as represented by hydroxypropylethylcellulose, hydroxypropyl methylcellulose, hydroxypropylbutylcellulose and hydroxypropylpentylcellulose; and a poly(vinylpyrrolidone) of 7,000 to 75,000 number-average molecular weight for enhancing the flow properties of the dosage form. Preferred among those polymers are the poly(ethylene oxide) of 100,000 - 300,000 number average molecular weight. Carriers that erode in the gastric environment, i.e., bioerodible carriers, are especially preferred.

Surfactants and disintegrants may be utilized in the carrier as well. Exemplary of the surfactants are those having an HLB value of between about 10 - 25, such as polyethylene glycol 400 monostearate, polyoxyethylene-4-sorbitan monolaurate, polyoxyethylene-20-sorbitan monooleate, polyoxyethylene-20-sorbitan monopalmitate, polyoxyethylene-20-monolaurate, polyoxyethylene-40 - stearate, sodium oleate and the like. Disintegrants may be selected from starches, clays, celluloses, algins and gums and crosslinked starches, celluloses and polymers. Representative disintegrants include corn starch, potato starch, croscarmelose, crospovidone, sodium starch glycolate, Veegum HV, methylcellulose, agar, bentonite, carboxymethylcellulose, alginic acid, guar gum and the like.

The drug layer 30 is formed as a mixture containing the porous particles and the carrier. The carrier portion of the drug layer may be formed from particles by comminution that produces the desired size of the carrier particle used in the fabrication of the drug layer. The means for producing carrier particles include granulation, spray drying, sieving, lyophilization, crushing, grinding, jet milling, micronizing and chopping to produce the intended micron particle size. The process can be performed by size reduction equipment, such as a micropulverizer mill, a fluid energy grinding mill, a grinding mill, a roller mill, a hammer mill, an attrition mill, a chaser mill, a ball mill, a vibrating ball mill, an impact pulverizer mill, a centrifugal pulverizer, a coarse crusher and a fine crusher. The size of the particle can be ascertained by screening, including a grizzly screen, a flat screen, a vibrating screen, a revolving screen, a shaking screen, an oscillating screen and a reciprocating screen. The processes and equipment for preparing drug and carrier particles are disclosed in Pharmaceutical Sciences, Remington, 17th Ed., pp. 1585-1594 (1985); Chemical Engineers Handbook, Perry, 6th Ed., pp. 21-13 to 21-19 (1984); Journal of Pharmaceutical Sciences, Parrot, Vol. 61, No. 6, pp. 813-829 (1974); and Chemical Engineer, Hixon, pp. 94-103 (1990).

The active compound may be provided in the liquid active agent formulation in amounts of from 1 microgram to 5000 mg per dosage form, depending upon the required dosing level that must be maintained over the delivery period, i.e., the time between consecutive administrations of the dosage forms. More typically, loading of compound in the dosage forms will provide doses of compound to the subject ranging from 1 microgram to 2500 mg per day, more usually 1 mg to 2500 mg per day. The drug layer typically will be a substantially dry composition formed by compression of the carrier and the porous particles, with the understanding that the porous particles will have contained therein the liquid, active agent formulation. The push layer will push the drug layer from the exit orifice as the push layer imbibes fluid from the environment of use, and the exposed drug layer will be eroded to release the porous particles into the environment of use. This may be seen with reference to Figure 6.

The push layer 28 is an expandable layer having a push-displacement composition in direct or indirect contacting layered arrangement with the drug layer 30. When in indirect contacting layered arrangement, an inert element (not shown), such as a spacer layer or disk, may be placed between the drug layer and the push layer. If several pulses of active agent are to be delivered from a single dosage form, similar inert layers may be interposed between discrete portions of drug layer. The inert layer(s) may be sized to provide appropriate time delay(s) between pulses of active agent and the volume of each discrete drug layer will provide control of the time period over which the pulse of active agent is delivered. Inert layers may be formed of materials utilized to form the push layer 28, or if desired, formed of materials that are easily compacted but do not swell in the fluid environment of use.

Push layer 28 comprises a polymer that imbibes an aqueous or biological fluid and swells to push the drug composition through the exit means of the device. Representatives of fluid-imbibing displacement polymers comprise members selected from poly(alkylene oxide) of 1 million to 15 million number-average molecular weight, as represented by poly(ethylene oxide) and poly(alkali carboxymethylcellulose) of 500,000 to 3,500,000 number-average molecular weight, wherein the alkali is sodium, potassium or lithium. Examples of additional polymers for the formulation of the push-displacement composition comprise osmopolymers comprising polymers that form hydrogels, such as Carbopol® acidic carboxypolymer, a polymer of acrylic cross-linked with a polyallyl sucrose, also known as carboxypolymethylene, and carboxyvinyl polymer having a molecular weight of 250,000 to 4,000,000; Cyanamer® polyacrylamides; cross-linked water swellable indenemaleic anhydride polymers; Good-rite® polyacrylic acid having a molecular weight of 80,000 to 200,000; Aqua-Keeps® acrylate polymer polysaccharides composed of condensed glucose units, such as diester cross-linked polygluran; and the like. Representative polymers that form hydrogels are known to the prior art in U.S. Patent No. 3,865,108, issued to Hartop; U.S. Patent No. 4,002,173, issued to Manning; U.S. Patent No. 4,207,893, issued to Michaels; and in Handbook of Common Polymers, Scott and Roff, Chemical Rubber Co., Cleveland, OH.

The osmagent, also known as osmotic solute and osmotically effective agent, which exhibits an osmotic pressure gradient across the outer wall and subcoat, comprises a member selected from the group consisting of sodium chloride, potassium chloride, lithium chloride, magnesium sulfate, magnesium chloride, potassium sulfate, sodium sulfate, lithium sulfate, potassium acid phosphate, mannitol, urea, inositol, magnesium succinate, tartaric acid raffinose, sucrose, glucose, lactose, sorbitol, inorganic salts, organic salts and carbohydrates.

Use of the inner wall or subcoat 32 is optional, but presently preferred. The inner subcoat 32 typically may be 0.01 to 5 mm thick, more typically 0.025-0.25 mm thick, although a thicker subcoat, for example 0.5 to 5mm thick, may be used in certain applications. The inner subcoat 32 comprises a member selected from hydrogels, gelatin, low molecular weight polyethylene oxides, e.g., less than 100,000 MW, hydroxyalkylcelluloses, e.g., hydroxyethylcellulose, hydroxypropylcellulose, hydroxyisopropylcelluose, hydroxybutylcellulose and hydroxyphenylcellulose, and hydroxyalkyl alkylcelluloses, e.g., hydroxypropyl methylcellulose, and mixtures thereof. The hydroxyalkylcelluloses comprises polymers having a 9,500 to 1,250,000 number-average molecular weight. For example, hydroxypropyl celluloses having number average molecular weights of between 80,000 to 850,000 are useful. The flow promoting layer may be prepared from conventional solutions or suspensions of the aforementioned materials in aqueous solvents or inert organic solvents. Prefered materials for the subcoat or flow promoting layer include hydroxypropyl cellulose, hydroxyethyl cellulose, hydroxypropyl methyl cellulose, povidone [poly(vinylpyrrolidone)], polyethylene glycol, and mixtures thereof. More prefered are mixtures of hydroxypropyl cellulose and povidone, prepared in organic solvents, particularly organic polar solvents such as lower alkanols having 1-8 carbon atoms, preferably ethanol, mixtures of hydroxyethyl cellolose and hydroxypropyl methyl cellulose prepared in aqueous solution, and mixtures of hydroxyetyyl cellulose and polyethylene glycol prepared in aqueous solution. Most preferably, the subcoat consists of a mixture of hydroxypropyl cellulose and povidone prepared in ethanol. Conveniently, the weight of the subcoat applied to the bilayer core may be correlated with the thickness of the subcoat and residual drug remaining in a dosage form in a release rate assay such as described herein. During manufacturing operations, the thickness of the subcoat may be controlled by controlling the weight of the subcoat taken up in the coating operation. When wall 32 is fabricated of a gel-forming material, contact with water in the environment of use facilitates formation of a gel or gel-like inner coat having a viscosity that may promote and enhance slippage between outer wall 22 and drug layer 30.

Exemplary solvents suitable for manufacturing the respective walls, layers, coatings and subcoatings utilized in the dosage forms of the invention comprise aqueous and inert organic solvents that do not adversely harm the materials utilized to fabricate the dosage forms. The solvents broadly include members selected from the group consisting of aqueous solvents, alcohols, ketones, esters, ethers, aliphatic hydrocarbons, halogenated solvents, cycloaliphatics, aromatics, heterocyclic solvents and mixtures thereof. Typical solvents include acetone, diacetone alcohol, methanol, ethanol, isopropyl alcohol, butyl alcohol, methyl acetate, ethyl acetate, isopropyl acetate, n-butyl acetate, methyl isobutyl ketone, methyl propyl ketone, n-hexane, n-heptane, ethylene glycol monoethyl ether, ethylene glycol monoethyl acetate, methylene dichloride, ethylene dichloride, propylene dichloride, carbon tetrachloride nitroethane, nitropropane tetrachloroethane, ethyl ether, isopropyl ether, cyclohexane, cyclooctane, benzene, toluene, naphtha, 1,4-dioxane, tetrahydrofuran, diglyme, water, aqueous solvents containing inorganic salts such as sodium chloride, calcium chloride, and the like, and mixtures thereof such as acetone and water, acetone and methanol, acetone and ethyl alcohol, methylene dichloride and methanol, and ethylene dichloride and methanol.

Pan coating may be conveniently used to provide the completed dosage form, except for the exit orifice. In the pan coating system, the subcoat on the wall-forming compositions is deposited by successive spraying of the respective composition on the bilayered core comprising the drug layer and the push layer accompanied by tumbling in a rotating pan. A pan coater is used because of its availability at commercial scale. Other techniques can be used for coating the drug core. Finally, the wall or coated dosage form are dried in a forced-air oven, or in a temperature and humidity controlled oven to free the dosage form of solvent. Drying conditions will be conventionally chosen on the basis of available equipment, ambient conditions, solvents, coatings, coating thickness,and the like.

Other coating techniques can also be employed. For example, the semipermeable wall and the subcoat of the dosage form can be formed in one technique using the air-suspension procedure. This procedure consists of suspending and tumbling the bilayer core in a current of air, an inner subcoat composition and an outer semipermeable wall forming composition, until, in either operation, the subcoat and the outer wall coat is applied to the bilayer core. The air-suspension procedure is well suited for independently,forming the wall of the dosage form. The air-suspension procedure is described in U.S. Patent No. 2,799,241; in J. Am. Pharm. Assoc., Vol. 48, pp. 451-459 (1959); and, ibid., Vol. 49, pp. 82-84 (1960). The dosage form also can be coated with a Wurster® air-suspension coater using, for example, methylene dichloride methanol as a cosolvent. An Aeromatic® air-suspension coater can be used employing a cosolvent.

The dosage form of the invention may be manufactured by standard techniques. For example, the dosage form may be manufactured by the wet granulation technique. In the wet granulation technique a solution, suspension or dispersion of the active agent in a liquid is mixed with the porous particles to allow the liquid, active agent formulation to sorb into the pores of the porous particles. Then the carrier is blended with the porous particles using an organic solvent, such as denatured anhydrous ethanol, as the granulation fluid. After a wet blend is produced, the wet mass blend is forced through a predetermined screen onto trays. The blend is dried under ambient conditions until the desired moisture level is obtained. The drying conditions are not so severe, however, that the liquid of the liquid, active agent formulation is allowed to evaporate to any significant extent. Next, a lubricant such as magnesium stearate or agglomerated silicon dioxide (Cab-O-Sil) for example, is added to the blend, which is then put into milling jars and mixed on a jar mill for several minutes. The composition is pressed into a layer, for example, in a Manesty® press. The first compressed layer is typically the drug layer, and then the push layer may be pressed against the composition forming the drug layer, and the bilayer tablets are fed to the Kilian® Dry Coater and surrounded with the drug-free coat, followed by the exterior wall solvent coating. In those instances where a trilayer dosage form for pulsatile release having a placebo layer is to be fabicated, the placebo layer is usually formed first, then the drug layer is pressed onto the placebo layer to form a bilayer composition, and then the push layer is compressed onto the bilayer core to form the trilayer composition. The trilayer tablet is then provided with the optional subcoat and the membrane coat for the rate controlling membrane. It is apparent, however, that the order in which the respective layers are compressed may be different, but the foregoing is preferred.

In another manufacture the porous particles containing the liquid, active agent formulation and other ingredients comprising the drug layer are blended and pressed into a solid layer. The layer possesses dimensions that correspond to the internal dimensions of the area the layer is to occupy in the dosage form, and it also possesses dimensions corresponding to the second layer for forming a contacting arrangement therewith. The drug layer components can also be blended with a solvent and mixed into a solid or semisolid form by conventional methods, such as ballmilling, calendering, stirring or rollmilling, and then pressed into a preselected shape. Next, the expandable layer, e.g., a layer of osmopolymer composition, is placed in contact with the layer of drug in a like manner. The layering of the drug formulation and the osmopolymer layer can be fabricated by conventional two-layer press techniques. The two contacted layers are first coated with the flow-promoting subcoat and then an outer semipermeable wall. The air-suspension and air-tumbling procedures comprise in suspending and tumbling the pressed, contacting first and second layers in a current of air containing the delayed-forming composition until the first and second layers are surrounded by the wall composition.

The dosage form of the invention is provided with at least one exit orifice. The exit orifice cooperates with the drug core for the uniform release of drug from the dosage form. The exit orifice can be provided during the manufacture of the dosage form or during drug delivery by the dosage form in a fluid environment of use. The expression "exit orifice" as used for the purpose of this invention includes a member selected from the group consisting of a passageway; an aperture; an orifice; and a bore. The expression also includes an orifice that is formed from a substance or polymer that erodes, dissolves or is leached from the outer coat or wall or inner coat to form an exit orifice.
The substance or polymer may include an erodible poly(glycolic) acid or poly(lactic) acid in the outer or inner coats; a gelatinous filament; a water-removable poly(vinyl alcohol); a leachable compound, such as a fluid removable pore-former selected from the group consisting of inorganic and organic salt, oxide and carbohydrate. An exit, or a plurality of exits, can be formed by leaching a member selected from the group consisting of sorbitol, lactose, fructose, glucose, mannose, galactose, talose, sodium chloride, potassium chloride, sodium citrate and mannitol to provide a uniform-release dimensioned pore-exit orifice. The exit orifice can have any shape, such as round, triangular, square, elliptical and the like for the uniform metered dose release of a drug from the dosage form. The dosage form can be constructed with one or more exits in spaced apart relation or one or more surfaces of the dosage form. The exit orifice can be performed by drilling, including mechanical and laser drilling, through the outer coat, the inner coat, or both. Exits and equipment for forming exits are disclosed in U.S. Patents Nos. 3,845,770 and 3,916,899, by Theeuwes and Higuchi; in U.S. Patent No. 4,063,064, by Saunders, et al.; and in U.S. Patent No. 4,088,864, by Theeuwes, et al. The exit orifice may be from 10% to 100% of the inner diameter of the compartment formed by wall 22, preferably from 30% to 100%, and most preferably from 50% to 100%.

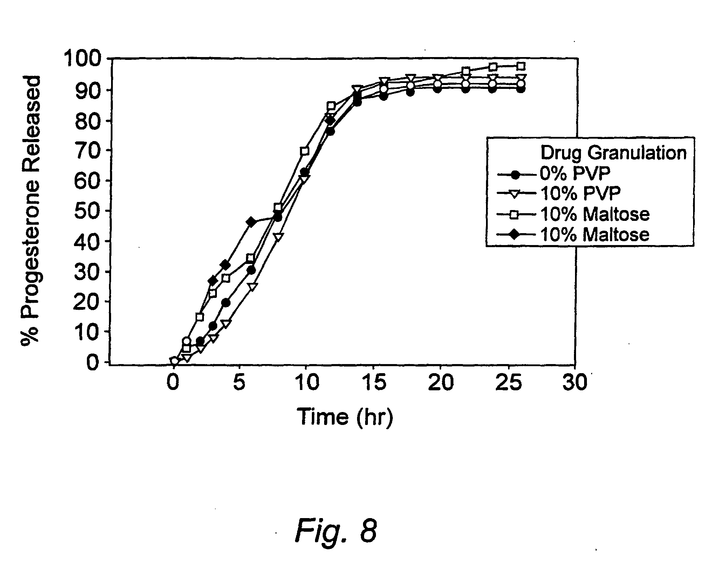

The continuous release dosage forms provide a uniform rate of release of compound over a prolonged period of time, typically from about zero hours, the time of administration, to about 4 hours to 20 hours or more, often for 4 hours to 16 hours, and more usually for a time period of 4 hours to 10 hours. At the end of a prolonged period of uniform release, the rate of release of drug from the dosage form may decline somewhat over a period of time, such as several hours. The dosage forms provide therapeutically effective amounts of drug for a broad range of applications and individual subject needs. The results of the release of progesterone from a representative, continuous release dosage form of this invention is provided in Figure 8. As can be seen therefrom, progesterone is released over a period of time extending up to about 15 hours. In Figure 8, the filled circles represent a drug granulation that does not contain any PVP (polyvinylpyrollidone), the empty triangles represent a drug granulation containing 10% PVP, and the filled squares and filled triangles represent drug granulations containing 10% maltose. In each case, the dosage forms were formed as trilayer, continuous system with (1) a mannitol layer adjacent the exit orifice that quickly dissolves in the release bath, (2) a drug layer containing progesterone dispersed in Cremophor EL/Myvacet in calcium hydrogen phosphate in a ratio of 45/55 % by weight as described in greater detail in Example 11, and (3) a push layer.

The dosage forms may also provide active agent in a pulsatile release profile. With reference to Figures 9-13, varying delays in the onset of the release of active agent are illustrated. Those results are achieved with the dosage forms described in Examples 12-16, respectively. By varying the volume of the placebo layer, it was possible to control the initial period before active agent is released from the dosage form.

With zero order release, upon initial administration, the dosage forms may provide a drug concentration in the plasma of the subject that increases over an initial period of time, typically several hours or less, and then provide a relatively constant concentration of drug in the plasma over a prolonged period of time, typically 4 hours to 24 hours or more. The release profiles of the dosage forms of this invention provide release of drug over the entire 24-hour period corresponding to once-a-day administration, such that steady state concentration of drug in blood plasma of a subject may be maintained at therapeutically effective levels over a 24 hour period after administration the sustained release dosage form. Steady state plasma levels of drug may typically be achieved after twenty-four hours or, in some cases, several days, e.g., 2-5 days, in most subjects.

Dosage forms of this invention release drug at a uniform rate of release over a prolonged period of time as determined in a standard release rate assay such as that described herein. When administered to a subject, the dosage forms of the invention provide blood plasma levels of drug in the subject that are less variable over a prolonged period of time than those obtained with immediate release dosage forms. When the dosage forms of this invention are administered on a regular, once-a-day basis, the dosage forms of the invention provide steady state plasma levels of drug such that the difference between Cₘₐₓ and Cₘᵢₙ over the 24-hour period is substantially reduced over that obtained from administration of an immediate release product that is intended to release the same amount of drug in the 24-hour period as is provided from the dosage forms of the invention

The dosage forms of this invention may be adapted to release active agent at a uniform rate of release rate over a prolonged period of time, preferably 4-6 hours or more. Measurements of release rate are typically made *in vitro,* in acidified water, simulated gastric fluid or simulated intestinal fluid to provide a simulation of conditions in specific biological locations, and are made over finite, incremental time periods to provide an approximation of instantaneous release rate. Information of such *in vitro* release rates with respect to a particular dosage form may be used to assist in selection of dosage form that will provide desired *in vivo* results. Such results may be determined by present methods, such as blood plasma assays and clinical observation, utilized by practitioners for prescribing available immediate release dosage forms.

Dosage forms of the present invention having zero order release rate profiles as described herein may provide to a patient a substantially constant blood plasma concentration and a sustained therapeutic effect of active agent, after administration of the dosage form, over a prolonged period of time. The sustained release dosage forms of this invention demonstrate less variability in drug plasma concentration over a 24-hour period than do immediate release formulations, which characteristically create significant peaks in drug concentration shortly or soon after administration to the subject.

The dosage forms of the invention may have a delayed onset of action incorporated directly into the dosage form by means of the placebo layer that has been described. For particular applications, it may be desirable to deliver a plurality of the dosage forms, with or without a placebo layer or other drug layer design, at a single location in the gastrointestinal tract. This may effected conveniently by combining the dosage forms of the invention with associated technology, such as for example, the Chronset® drug delivery system of Alza Corporation, Palo Alto, California. Such systems can be programmed to release the dosage forms at designated times and at targeted absorption sites. That technology is described in US Patents Nos.5,110,597; 5,223,265; 5,312,390; 5,443,459; 5,417,682; 5,498,255; 5,531,736; and 5,800,422. The composite delivery system may be manufactured by loading the osmotic dosage forms described herein into the Chronset® systems, and provide for the controlled release of active agent in a variety of formats.

An illustrative general method of manufacturing dosage forms of this form of the invention is described below in PREPARATION 2. Percentages are percentages by weight unless noted otherwise. Variations in the methods and substitution of materials may be made and will be apparent from the earlier description. Equivalent or proportional amounts of such materials may be substituted for those used in the PREPARATION 2. More specific descriptions are provided in the Examples and alternative materials and procedures are illustrated therein.

### PREPARATION 2

### Preparation of the Drug Layer.

A binder solution is prepared by adding hydroxypropyl cellulose (Klucel MF, Aqualon Company), "HPC", to water to form a solution containing 5 mg of HPC per 0.995 grams of water. The solution is mixed until the hydroxypropyl cellulose is dissolved. For a particular batch size, a fluid bed granulator ("FBG") bowl is charged with the required amounts of liquid, active agent formulation and the corresponding amount of porous particles, such as exemplified by the calcium hydrogen phosphate particles sold under the trademark FujiCalin.

After the liquid is absorbed by the particles, the blend is mixed with, polyethylene oxide (MW 200,000) (Polyox® N-80, Union Carbide Corporation) (20.3%), hydroxypropyl cellulose (Klucel MF) (5%), polyoxyl 40 stearate (3%) and crospovidone (2%). After mixing the semi-dry materials in the bowl, the binder solution prepared as above is added. Then the granulation is dried in the FBG to a dough-like consistency suitable for milling, and the granulation is milled through a 7 or a 10 mesh screen.

The granulation is transferred to a tote blender or a V-blender. The required amounts of antioxidant, butylated hydroxytoluene ("BHT") (0.01%), and lubricant, stearic acid (1%), are sized through a 40 mesh screen and both are blended into the granulation using the tote or V-blender until uniformly dispersed (about 1 minute of blending for stearic acid and about 10 minutes of blending for BHT.

### Preparation of the Osmotic Push Layer Granulation.

A binder solution is prepared by adding hydroxypropyl methylcellulose 2910 ("HPMC") to water in a ratio of 5 mg of HPMC to 1 g of water. The solution is mixed until the HPMC is dissolved. Sodium chloride powder (30%) and red ferric oxide (1.0%) are milled and screened. A fluid bed granulator ("FBG") bowl is charged with the required amounts of polyethylene oxide (MW 7,000,000) (Polyox® 303) (63.7%), HPMC (5.0%), the sodium chloride and the red ferric oxide. After mixing the dry materials in the bowl, the binder solution prepared above is added. The granulation is dried in the FBG until the target moisture content (< 1% by weight water) is reached. The granulation is milled through a 7 mesh screen and transferred to a tote blender or a V-blender. The required amount of antioxidant, butylated hydroxytoluene (0.08%), is sized through a 60 mesh screen. The required amount of lubricant, stearic acid (0.25%), is sized through a 40 mesh screen and both materials are blended into the granulation using the tote or V-blender until uniformly dispersed (about 1 minute for stearic acid and about 10 minutes for BHT).

### Bilayer Core Compression.

A longitudinal tablet press (Korsch press) is set up with round, deep concave punches and dies. Two feed hoppers are placed on the press. The drug layer prepared as above is placed in one of the hoppers while the osmotic push layer prepared as above is placed in the remaining hopper.

The initial adjustment of the tableting parameters (drug layer) is performed to produce cores with a uniform target drug layer weight. The second layer adjustment (osmotic push layer) of the tableting parameters is performed which bonds the drug layer to the osmotic layer to produce cores with a uniform final core weight, thickness, hardness, and friability. The foregoing parameters can be adjusted by varying the fill space and/or the force setting. A typical tablet containing a target amount of drug may be approximately 0.465 inches long and approximately 0.188 inches in diameter.

### Preparation of the Subcoat Solution and Subcoated System.

The subcoat solution is prepared in a covered stainless steel vessel. The appropriate amounts of povidone (K29-32) (2.4%) and hydroxypropyl cellulose (MW 80,000) (Klucel EF, Aqualon Company) (5.6%) are mixed into anhydrous ethyl alcohol (92%) until the resulting solution is clear. The bilayer cores prepared above are placed into a rotating, perforated pan coating unit. The coater is started and after the coating temperature of 28 -36 °C is attained, the subcoating solution prepared above is uniformly applied to the rotating tablet bed. When a sufficient amount of solution has been applied to provide the desired subcoat weight gain, the subcoat process is stopped. The desired subcoat weight will be selected to provide acceptable residuals of drug remaining in the dosage form as determined in the release rate assay for a 24-hour period. Generally, it is desirable to have less than 10%, more preferably less than 5%, and most preferably less than 3% of residual drug remaining after 24 hours of testing in a standard release rate assay as described herein, based on the initial drug loading. This may be determined from the correlation between subcoat weight and the residual drug for a number of dosage forms having the same bilayer core but different subcoat weights in the standard release rate assay.

### Preparation of the Rate Controlling Membrane and Membrane Coated System.

Subcoated bilayer cores prepared as above are placed into a rotating, perforated pan coating unit. The coater is started, and after the coating temperature (28 - 38 °C) is attained, a coating solution such as illustrated in A, B or C below is uniformly applied to the rotating tablet bed until the desired membrane weight gain is obtained. At regular intervals throughout the coating process, the weight gain is determined and sample membrane coated units may be tested in the release rate assay to determine a T₉₀ for the coated units. Weight gain may be correlated with T₉₀ for membranes of varying thickness in the release rate assay. When sufficient amount of solution has been applied, conveniently determined by attainment of the desired membrane weight gain for a desired T₉₀, the membrane coating process is stopped. Illustrative rate controlling membrane compositions:
A. A coating solution is prepared in a covered stainless steel vessel. The appropriate amounts of acetone (565 mg) and water (29.7 mg) are mixed with the poloxamer 188 (1.6 mg) and cellulose acetate (29.7 mg) until the solids are completely dissolved. The coating solution has about 5% solids upon application.
B. Acetone (505.4 mg) is mixed with cellulose acetate (27.72 mg) until the cellulose acetate is completely dissolved. Polyethylene glycol 3350 (0.28 mg) and water (26.6 mg) are mixed in separate container. The two solutions are mixed together until the resulting solution is clear. The coating solution has about 5% solids upon application.
C. Acetone (776.2 mg) is mixed with cellulose acetate (42.57 mg) until the cellulose acetate is completely dissolved. Polyethylene glycol 3350 (0.43 mg) and water (40.9 mg) are mixed in separate container. The two solutions are mixed together until the resulting solution is clear. The coating solution has about 5% solids upon application.

### Drilling of Membrane Coated Systems.

One exit port is drilled into the drug layer end of the membrane coated system. During the drilling process, samples are checked at regular intervals for orifice size, location, and number of exit ports.

### Drying of Drilled Coated Systems.

Drilled coated systems prepared as above are placed on perforated oven trays which are placed on a rack in a relative humidity oven (43-45 % relative humidity) and dried to remove the remaining solvents from the coating layers.

### Color and Clear Overcoats.

Optional color or clear coats solutions are prepared in a covered stainless steel vessel. For the color coat 88 parts of purified water is mixed with 12 parts of Opadry II [color not critical] until the solution is homogeneous. For the clear coat 90 parts of purified water is mixed with 10 parts of Opadry Clear until the solution is homogeneous. The dried cores prepared as above are placed into a rotating, perforated pan coating unit. The coater is started and after the coating temperature is attained (35-45 °C), the color coat solution is uniformly applied to the rotating tablet bed. When sufficient amount of solution has been applied, as conveniently determined when the desired color overcoat weight gain has been achieved, the color coat process is stopped. Next, the clear coat solution is uniformly applied to the rotating tablet bed. When sufficient amount of solution has been applied, or the desired clear coat weight gain has been achieved, the clear coat process is stopped. A flow agent (e.g., Carnu-bo wax) is applied to the tablet bed after clear coat application.

Variations in the foregoing procedure will be apparent to one skilled in the art. The examples are provided to illustrate representative dosage forms of the invention prepared by analogous methods.

### ASSAY

The release rate of drug from devices containing the dosage forms of the invention may be determined in standardized assays such as the following. The method involves releasing systems into a release liquid medium, such as acidified water (pH 3), artificial gastric fluid or artificial intestinal fluid. Aliquots of sample release rate solutions are injected onto a chromatographic system to quantify the amount of drug released during specified test intervals. Drug is resolved on a C₁₈ column and detected by UV absorption at the appropriate wavelength for the drug in question. Quantitation is performed by linear regression analysis of peak areas from a standard curve containing at least five standard points.

Samples are prepared with the use of a USP Type 7 Interval Release Apparatus. Each system (invention device) to be tested is weighed. Then, each system is glued to a plastic rod having a sharpened end, and each rod is attached to a release rate dipper arm. Each release rate dipper arm is affixed to an up/down reciprocating shaker (USP Type 7 Interval Release Apparatus), operating at an amplitude of about 3 cm and 2 to 4 seconds per cycle. The rod ends with the attached systems are continually immersed in 50 ml calibrated test tubes containing 50 ml of the release medium, equilibrated in a constant temperature water bath controlled at 37°C ± 0.5°C. At the end of each time interval specified, typically one hour or two hours, the systems are transferred to the next row of test tubes containing fresh release medium. The process is repeated for the desired number of intervals until release is complete. Then the solution tubes containing released drug are removed and allowed to cool to room temperature. After cooling, each tube is filled to the 50 ml mark, each of the solutions is mixed thoroughly, and then transferred to sample vials for analysis by high pressure liquid chromatography ("HPLC"). Standard solutions of drug are prepared in concentration increments encompassing the range of 5 micrograms to about 400 micrograms and analyzed by HPLC. A standard concentration curve is constructed using linear regression analysis. Samples of drug obtained from the release test are analyzed by HPLC and concentration of drug is determined by linear regression analysis. The amount of drug released in each release interval is calculated.

### EXAMPLE 11

A delivery system (Fig. 6) is manufactured for dispensing a beneficial drug progesterone in a controlled manner over a prolonged period of time. A self-emulsifying drug solution comprising, in weight percent, 2% progesterone, 49% polyoxyl 35 castor oil (Cremophor EL, BASF Corporation) and 49% distilled acetylated monoglyceride (Myvacet 9-45) is prepared. Then, 38 % of the solution is blended with 47% of porous calcium hydrogen phosphate (FujiCalin SG) in a mixing vessel. Four percent of hydroxypropyl methylcellulose (HPMC E5) dissolved in ethanol is slowly added into the mixing vessel containing the blend, and is mixed with the blend until even consistency of wet mass is attained. The wet mass is passed through a screen and then dried at ambient conditions until the granulation reaches the specified moisture level. The mass is rescreened, and then 10 % of maltose and 1 % magnesium stearate is added to the granules and blended.

Next, an osmotic-layer forming composition comprising, in weight percent, 58.75% sodium carboxymethyl cellulose (7H4F), 30.0% sodium chloride, 5.0% hydroxpropyl methylcellulose (E5), 1.0% red ferric oxide is prepared by passing each component a 40-mesh stainless steel screen and then blending in a Galtt fluid-bed granulator and sprayed with 5.0% hydroxypropyl cellulose (EF) solution in purified water until homogeneous granules form. These granules are passed through an 8-mesh stainless steel screen and mixed with 0.25% magnesium stearate.

376 Mg of the drug-layer granules and 169 mg of the osmotic(push)-layer granules are compressed into bilayer longitudinal caplets using 0.265" round punch and Carver press. The tablets are coated with a subcoat composition comprising 5% of Klucel JF and 95% of ethanol using a Freud Hi-coater . The weight of the subcoat is about 3 mg. Then, the subcoated tablets are coated with a rate-controlling membrane composition. The membrane-forming composition comprises, in weight percent, 85% cellulose acetate having an acetyl content of 39.8% and 15% Pluronic F68. The membrane-forming composition is dissolved in acetone to make a 5% solid solution. The membrane-forming composition is sprayed onto the tablets in a Freud Hi-coater. The membrane weight is about 22 mg. Finally, an exit orifice (230 mil) is cut mechanically on the drug-layer side of the system. The final system delivers progesterone in-vitro with a zero order delivery as shown in Figure 5.

### EXAMPLE 12

A delivery system (Fig. 7) is manufactured for dispensing a beneficial drug such as progesterone as a delayed pulse. First, a self-emulsifying drug solution comprising, in weight percent, 2% progesterone, 49% Cremophor EL and 49% Myvacet 9-45 is prepared. Then, 38 % of the solution is blended with 47% of porous calcium hydrogen phosphate (FujiCalin SG)in a mixing vessel. Four percent of HPMC E5 dissolved in ethanol is slowly added into the mixing vessel containing the blend, and is mixed with the blend until even consistency of wet mass is attained. The wet mass is passed through a screen and then dried at ambient conditions until the granulation reaches the specified moisture level. The mass is rescreened, and then 10 % of maltose and 1 % magnesium stearate is added to the granules and blended.

Next, an osmotic (push)-layer forming composition comprising, in weight percent, 58.75% sodium carboxymethyl cellulose (7H4F), 30.0% sodium chloride, 5.0% hydroxpropyl methylcellulose (E5), 1.0% red ferric oxide is prepared by passing each component a 40-mesh stainless steel screen and then blending in a Galtt fluid-bed granulator and sprayed with 5.0% hydroxypropyl cellulose (EF) solution in purified water until homogeneous granules form. These granules are passed through an 8-mesh stainless steel screen and mixed with 0.25% magnesium stearate.

Then, 50 mg of placebo-layer granules (having the same composition as the osmotic-layer), 195 mg of the drug-layer granules and 165 mg of the osmotic-layer granules are compressed into tri-layer longitudinal caplets using 0.265" round punch and Carver press. The tablets are coated with a subcoat composition comprising 5% of Klucel JF and 95% of ethanol using a Freud Hi-coater . The weight of the subcoat is about 3 mg. Then, the subcoated tablets are coated with a rate-controlling membrane composition. The membrane-forming composition comprises, in weight percent, 85% cellulose acetate having an acetyl content of 39.8% and 15% Pluronic F68. The membrane-forming composition is dissolved in acetone to make a 5% solid solution. The membrane-forming composition is sprayed onto the tablets in a Freud Hi-coater. The membrane weight is about 22 mg. Finally, an exit orifice (230 mil) is cut mechanically on the ist placebo-layer side of the system. The final system delivers progesterone in-vitro with a 2 hour delayed pulse as shown in Figure 9.

### EXAMPLE 13

The procedure of Example 12 is repeated in this example for providing the following dosage form:

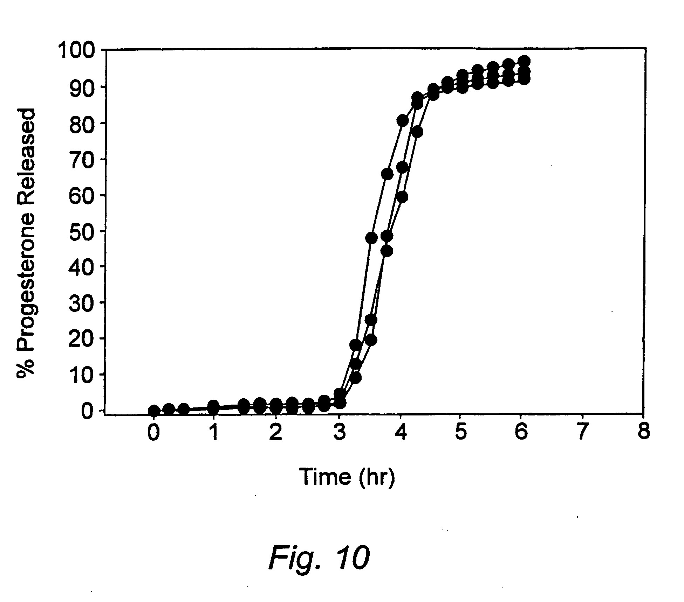

A dosage form composed of the drug-layer, osmotic-layer and the membrane, the compositions of which are all identical to those in Example 12 is prepared, except that the placebo-layer weight is 100 mg. The final dosage form delivers progesterone in-vitro with a 3 hour delayed pulse as shown in Figure 10.

### EXAMPLE 14

The procedure of Example 12 is repeated in this example for providing the following dosage form:

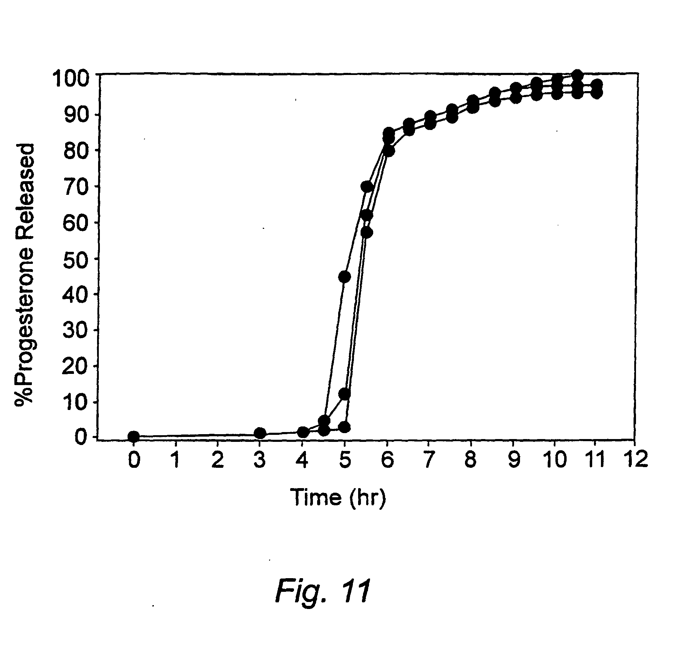

A dosage form composed of the drug-layer, osmotic-layer and the membrane , the compositions of which are all identical to those in Example 12 is prepared, except that the placebo-layer weight is 155 mg. The final dosage form delivers progesterone in-vitro with a 5 hour delayed pulse as shown in Figure 11.

### EXAMPLE 15

The procedure of Example 12 is repeated in this example for providing the following dosage form:

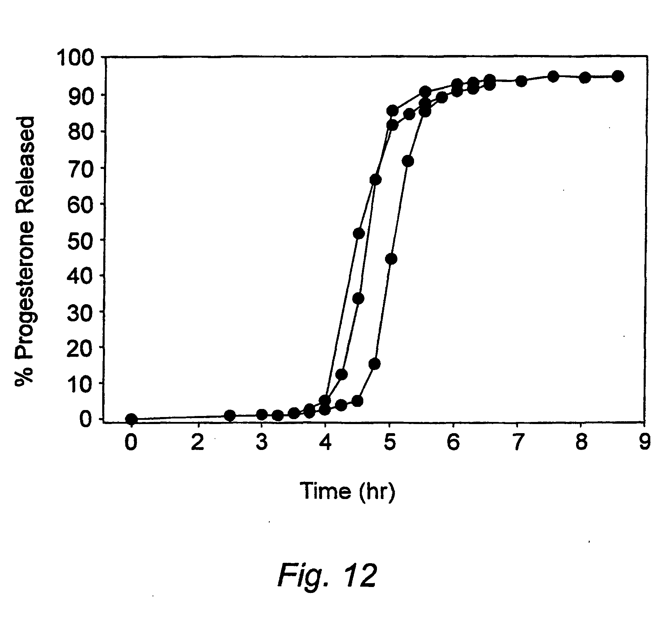

A dosage form composed of the drug-layer, osmotic-layer and the membrane, the compositions of which are all identical to those in Example 12 is prepared, except that the placebo-layer weight is 250 mg. The final dosage form delivers progesterone in-vitro with a 6-7 hour delayed pulse as shown in Figure 12.

### EXAMPLE 16

The procedure of Example 12 is repeated in this example for providing the following dosage form:

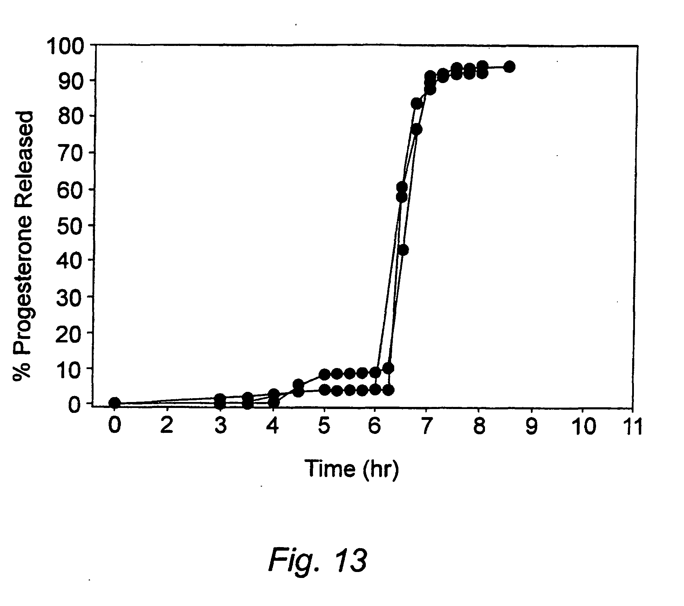

A dosage form composed of the osmotic-layer and the membrane layer compositions which are identical to those in Example 12 is prepared, except that the placebo-layer weight is 155 mg, the drug-layer granulation is composed of 36% of the drug solution described in Example 12, 44% calcium phosphate, 4% HPMC E5, 1% Mg stearate and 15% maltose, and the weight of the rate-controlling membrane is 105 mg. The final dosage form delivers progesterone in-vitro with a 10-h delayed pulse as shown in Figure 13.

### EXAMPLE 17

The following formulations are prepared for the incorporation into the dosage forms illustrated in Figure 6 and Figure 7 in accordance with the general procedures described. All percentages are by weight unless otherwise noted. The Polyox 303 push layer is used as the barrier or delay layer (sometimes denoted as a placebo layer) for those dosage forms illustrated in Figure 7 and as the expandable or push layer in both dosage forms illustrated in Figures 6 and 7. Tableting is done on a Carver press at one-quarter ton pressure.

| Polyox 303 push and delay layer formulation | |
|---|---|
| Polyox 303 | 63.68% |
| Sodium Chloride | 30% |
| HPMC E5 | 5% |
| Red Ferric Oxide | 1% |
| Mg Stearate | 0.25% |
| BHT | |
| | 0.08% |

### Polyox 303 preparation:

The polyox, NaCl, and oxide are blended in a Giatt fluid-bed granulator and sprayed with a 5% HPMC E5 solution in purified water until homogeneous granules are formed. These granules are passed through 16-mesh stainless steel screen and mixed with magnesium stearate and BHT.

### FujiCalin formulations for drug tablet dissolution

| Formulation | A | B | C | D | E |
|---|---|---|---|---|---|
| FujiCalin SG | 52% | 52% | 47% | 47% | 44% |
| Cremophor EL | -- | 20.6% | 18.6% | 18.6% | 17.6% |
| Cremophor RH | 20.6% | -- | -- | -- | -- |
| Myvacet 9-45 | 20.6% | 20.6% | 18.6% | 18.6% | 17.6% |
| Progesterone | 0.84% | 0.84% | 0.76% | 0.76% | 0.72% |
| HPMC E5 | 4% | 4% | 4% | 4% | 4% |
| PVP XL | -- | -- | 10% | -- | 15% |
| Maltose | -- | -- | -- | 10% | -- |
| Mg Stearate | 1% | 1% | 1% | 1% | 1% |

### FujiCalin tablet preparation:

The progesterone, Cremophor and Myvacet are dissolved by combining the materials in a mixing bowl and mixing with a magnetic stir bar in a 40C water bath for 3 hours. The resulting solution is slowly added to the FujiCalin granules in a mechanical mixing bowl (KitchenAid mixer) while mixing. Mixing is continued for 10 minutes and the HPMC E5, wet granulated with ethanol, is added. The resulting mass is passed through a 20-mesh screen and allowed to dry overnight under ambient conditions. The dried material is again screened through a 20-mesh screen, and the dried granules are blended with the PVP XL on a roller mixer for 10 minutes. Then, the magnesium stearate is added, and the mixture is blended on the roller mixer for an additional 2 minutes. The resulting material is suitable for tableting. To facilitate release of the tablets from the die components, a small amount of mannitol may be applied to the outside surface of the drug formulation being tableted. Tableting is done on a Carver press at one-quarter ton pressure.

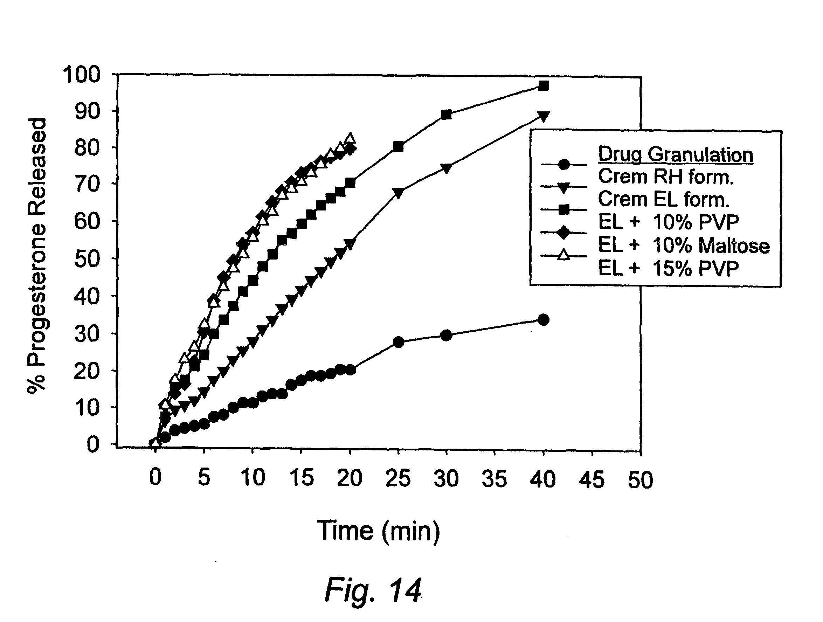

The dissolution profiles for tablets containing the various drug formalities described above in artificial gastric fluid developed in a USP bath are represented in Figure 14, in which circles represent the formulation A, inverted triangles represent formulation B, squares represent formulation C, diamonds represent formulation D, and triangles represent formulation E.

### Pulse System Tableting:

Tri-layer tablets containing the foregoing formulations and completed dosage forms are prepared according to the general procedures described in Example 11. The dosage forms provide pulsed delivery of progesterone having varying delay periods depending on the amount of the material in the placebo/barrier layer.

| Neusilin formulations for drug tablet | | | | |
|---|---|---|---|---|
| Formulation | G/K | H/L | I/M | J |
| Neusilin US2 | 34% | 36% | 38% | 40% |
| Cremophor EL | 24.99% | 26.46% | 27.93% | 29.4% |
| Myvacet 9-45 | 24.99% | 26.46% | 27.93% | 29.4% |
| Progesterone | 1.02% | 1.08% | 1.14% | 1.2% |
| Acdisol or PVP XL | 15% | 10% | 5% | 0% |

### Neusilin tablet preparation:

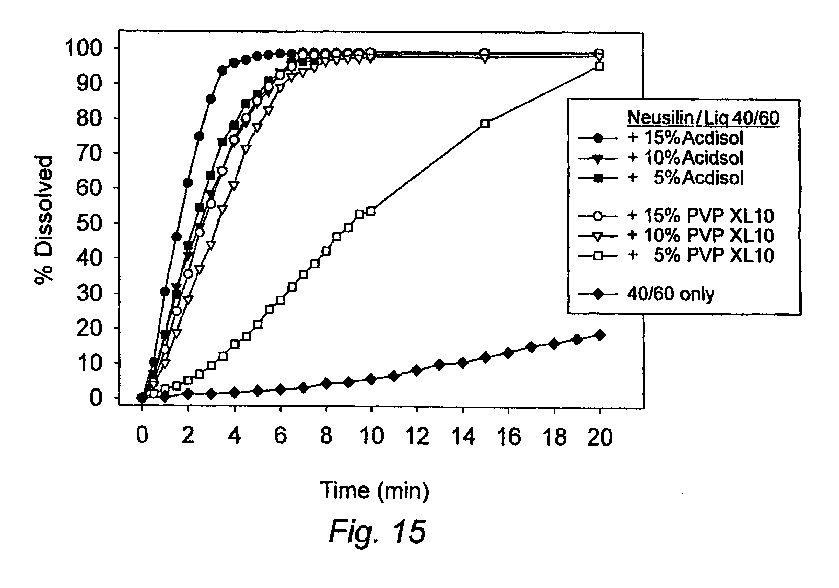

Neusilin tablets having formulations as set forth above are prepared in a similar manner to that described for FujiCalin above except that the magnesium stearate and its mixing step are eliminated. Formulations G, H and I are formed with Acdisol. Formulations K, L and M are formed with PVP XL. Tableting is done on a Carver press at one-quarter ton pressure. Tablets are readily ejected from the die without the use of mannitol. The dissolution profiles for the various formulations are represented in Figure 15. The filled circles represent formulation G, filled, inverted triangles represent formulation H, and filled squares represent formulation I. The open circles represent formulation K, open, inverted triangles represent formulation L, and open squares represent formulation M. The filled diamonds represent formulation J.

### Pulse System Tableting:

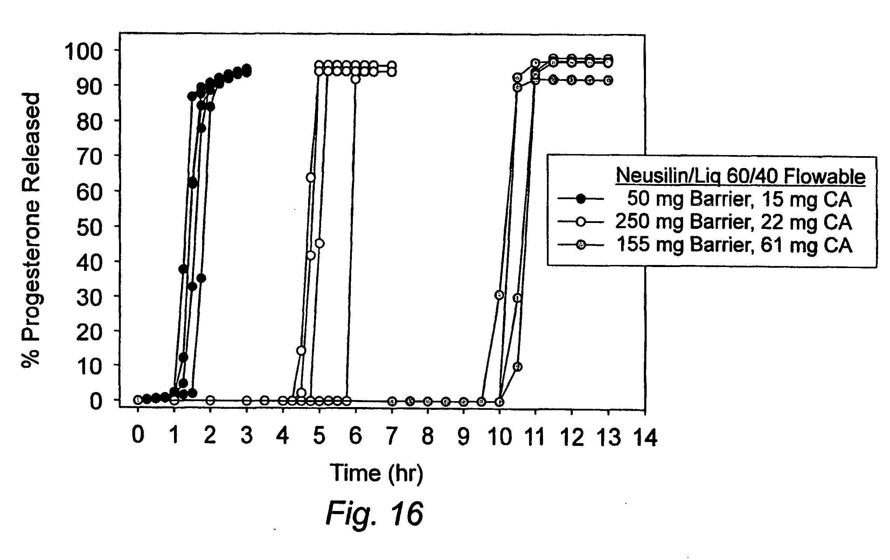

Tri-layer tablets are prepared by the general procedures described in Example 11, and coated with a semipermable membrane of cellulose acetate/Pluronics F68 at a weight ratio 85/15 as described. Representative release profiles for the tri-layer, pulse dosage forms are illustrated in Figure 16 for formulations as described above with 5% Acdisol, and barrier/membrane layer weights of 50/15 mg, 250/22 mg and 155/16 mg, providing delay periods of about 1, 5 and 10 hours, respectively.

### EXAMPLE 18

This example illustrates that the various layers of the dosage forms may be tableted with conventional tableting equipment. The practice formulation, without drug, is prepared as a 10 kg batch for use in a tri-layer dosage form as illustrated in Figure 7. The tri-layer tablets are formed on a multi-station tri-layer tablet press having 11 stations. The press is operated at 5 rpm and the compression forces utilized for the first layer (osmotic push layer), second layer (placebo/particles) and a third layer (barrier) are 100, 100, and 4,000 N respectively. The weights in each tablet of the osmotic/placebo (particles)/barrier layers are 175/160/125 mg, respectively. Tablets prepared are expelled from the tableting cavity without sticking to the cavity walls or the punch.

| | |
|---|---|
| Neusilin US2 | 55.8% |
| Cremophor EL | 18.6% |
| Myvacet 9-45 | 18.6% |
| Acdisol | 4.5% |
| Stearic Acid | 2.0% |
| Mg stearate | 0.5% |

### Particle layer preparation:

In this example drug was not included in the particle layer. The Cremophor and Myvacet are mixed in a large steel pot with a mechanical mixer for 20 minutes. In a large Hobart mixer Neusilin powder is added to the bowl, and the Cremophor/Myvacet blend is slowly added through a funnel to the powder over a 5 minute period while stirring is maintained. Material on the sides of the bowl is scraped down and the blend is mixed for 2 minutes more. Then the material is transferred to a Gerrico V-blender, and the Acdisol and stearic acid are added. The resulting mass is mixed for 5 minutes, after which the magnesium stearate is added and the mass mixed for 1 minute more. The blend material flows easily and may be directly loaded into the hoppers of the tableting press.

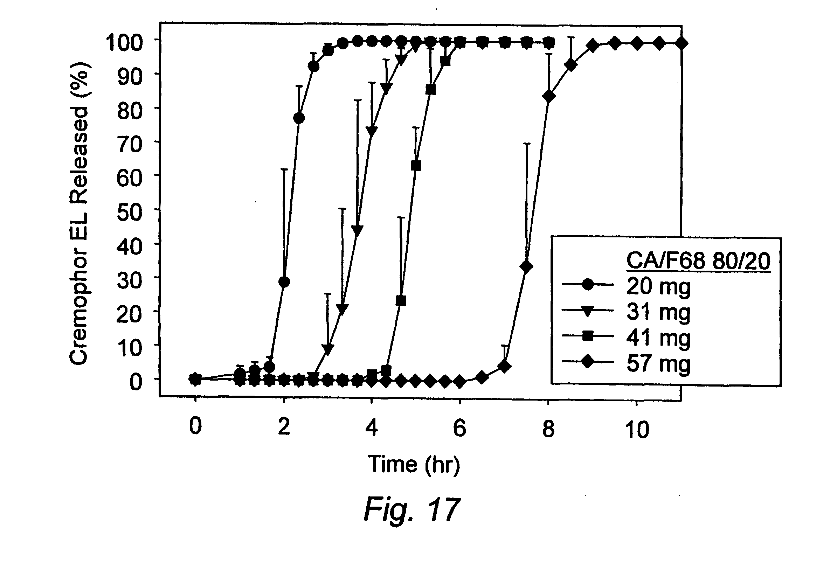

Tri-layer tablets prepared from the above formulation as the (drug)/particle layer and the Polyox formulation for the barrier and push layers described above were prepared as described with semipermeable membrane coats formed from 80/20 cellulose acetate/Pluronics F68 of 20 mg, 31 mg, 41 mg, and 57 mg and 190 mil exit orifice. The release profiles (measured in terms of Cremophor released since no drug was present) of those systems are presented in Figure 17. The filled circles correspond to a 20 mg membrane coat, filled inverted triangles correspond to a 31 mg membrane coat, filled squares correspond to a 41 mg membrane coat, and filled diamonds correspond to a 57 mg membrane coat.

## Claims

1. A dosage form for an active agent comprising a wall defining a cavity, the wall having an exit orifice formed or formable therein and at least a portion of the wall being semipermeable; an expandable layer located within the cavity remote from the exit orifice and in fluid communication with the semipermeable portion of the wall; a drug compacted layer located within the cavity adjacent the exit orifice and in direct or indirect contacting relationship with the expandable layer,**characterised in that** the compacted drug layer comprises a plurality of particles having interior pores and a liquid, active agent formulation in the pores, the particles being compactable and adapted to retain substantially all of the liquid active agent formulation within the pores during the compacting process.

2. A dosage form as claimed in Claim 1 wherein the particles are formed from calcium hydrogen phosphate, magnesium aluminometasilicate, microcrystalline cellulose or silicon dioxide.

3. A dosage form as claimed in Claim 2 wherein the particles are formed from calcium hydrogen phosphate of the following general formula
CaHPO₄•mH₂O
wherein m satisfies the relationship 0≦m≦2.0.

4. A dosage form as claimed in Claim 3 wherein the particles are formed by spray drying a scale-like calcium hydrogen phosphate with a specific surface area of 20 m²/g to 60 m²/g, an apparent specific volume of 1.5ml/g or more, an oil absorption capacity of 0.7 ml/g or more, a primary particle size of 0.1µ to 5µ, and an average particle size of 2µ to 10µ among secondary particles that are aggregates of the primary particles.

5. A dosage form as claimed in Claim 3 or 4 wherein the particles are calcium hydrogen phosphate having a specific volume of at least 1.5 ml/g, a BET specific area of at least 20 m²/g, and a water absorption capacity of at least 0.7 ml/g.

6. A dosage form as claimed in any one of Claims 3 to 5 wherein the particles having a size distribution of 100% less than 40 mesh, 50%-100% less than 100 mesh and 10%-60% less than 200 mesh.

7. A dosage form as claimed in Claim 6 wherein the particles have a size distribution of which 100% are less than 40 mesh, 60%-90% are less than 100 mesh and 20%-60% are less than 200 mesh.

8. A dosage form as claimed in any one of the preceding Claims wherein the particles have a bulk density of 0.4-0.6 g/ml, a BET surface area of 30-50 m²/g, a specific volume of greater than 1.5 ml/g, and a mean pore size of at least 50 Angstroms.

9. A dosage form as claimed in Claim 3 wherein the particles are calcium hydrogen phosphate having a bulk specific volume of 1.5 ml/g-5 ml/g, a BET specific area of 20 m²/g-60 m²/g, a water absorption capacity of at least 0.7 ml/g, and a mean particle size of at least 70 microns.

10. A dosage form as claimed in Claim 2 wherein the particles are magnesium aluminometasilicate represented by the general formula
Al₂O₃MgO•2SiO₂•nH₂O
wherein n satisfies the relationship 0≦n≦10.

11. A dosage form as claimed in Claim 10 wherein the silicate has a specific surface area (arca) of 100-300m²/g, an oil absorption capacity of 1.3-3.4ml/g, a mean particle size of 1-2 microns, an angle of repose 25° -45°, a specific gravity of 2 g/ml and a specific volume of 2.1-12 ml/g.

12. A dosage form as claimed in Claim 10 wherein the particles comprise magnesium aluminometasilicate powder.

13. A dosage form as claimed in any one of Claims 1 to 11 wherein the particles are formed from a silcate as defined in Claim 11 or Claim 12, a calcium hydrogen phosphate having a mean pore size of the order of 70 Angstroms, a mean particle size of 110 microns, a specific volume of 2ml/g, a BET specific surface area of 30-40m²/g, and an oil and water absorption capacity of 0.8ml/g, or a combination thereof.

14. A dosage form as claimed in any one of the preceding Claims wherein the dosage form includes a pH regulating agent selected from one or more of the group comprising organic acids, inorganic acids and bases.

15. A dosage form as claimed in any one of the preceding Claims wherein the dosage form includes a chelating agent.

16. A dosage form as claimed in any one of the preceding Claims wherein the particles are formed from calcium hydrogen phosphate and the dosage form includes an organic acid, chelating agent, or a combination thereof.

17. A dosage form as claimed in any one of the preceding Claims wherein the weight percent of liquid, active agent formulation is at least 5% of the total weight of the dosage form.

18. A dosage form as claimed in any one of the preceding Claims wherein the active agent is sildenafil citrate, acetaminophen, ibuprofen or ketoprofen.

19. A dosage form as claimed in any one of the preceding Claims wherein the particles are adapted to be dispersed in a bioerodible carrier.

20. A dosage form as claimed in any one of the preceding Claims having a placebo layer between the exit orifice and the drug layer.

21. A dosage form as claimed in any one of the preceding Claims having a flow-promoting layer interposed between the inner surface of the wall and at least the external surface of the drug layer located within the cavity.

22. A dosage form as claimed in any one of the preceding Claims, having at least two drug layers separated by at least one inert layer.

23. A dosage form as claimed in any one of the preceding Claims having at least two drug layers, each of said drug layers containing a different active agent.

24. A dosage form as claimed in any one of the preceding Claims wherein the liquid, active agent formulation of the drug layer comprises a self-emulsifying formulation.

25. A dosage form as claimed in any one of the preceding Claims wherein the liquid active agent of the drug layer comprises an absorption enhancer.

26. A dosage form as claimed in any one of the preceding Claims wherein the liquid, active agent formulation comprises at least 30% by weight of the drug layer.

27. A dosage form as claimed in any one of the preceding Claims adapted for sustained release of the liquid, active agent formulation upon administration to a subject.

28. A dosage form as claimed in any one of Claims 1 to 26 adapted for pulsatile release of the liquid, active agent formulation upon administration to a subject.

29. A dosage form as claimed in any one of the preceding Claims wherein the dosage form is a unitary and oral dosage form.

30. A composition comprising a liquid formulation of the active agent sorbed into a plurality of porous particles, the particles being as defined in any one of Claims 2 to 19, and dispersed throughout a bioerodible carrier, the particles being released in the environment of use over a time period between several hours to 24 hours.

31. A composition as claimed in Claim 30 wherein the particles are formed by spray drying scale-like calcium hydrogen phosphate with a specific surface area of 20m²/g to 60m²/g, an apparent specific volume of 1.5ml/g or more, an oil absorption capacity of 0.7ml/g or more, a primary particle size of 0.1µ to 5µ, and an average particle size of 2 µ to 10 µ among secondary particles that are aggregates of the primary particles, the scale-like calcium hydrogen phosphate being represented by the following general formula:
CaHPO₄•mH₂O
wherein m satisfies the relationship 0≦m≦2.0.

32. A method of facilitating the release of an active agent from a dosage form comprising sorbing a liquid formulation of the active agent into a plurality of porous particles, the particles being defined in any one of Claims 2 to 19 and dispersing the particles throughout a bioerodible carrier.

## Patentansprüche

1. Eine Darreichungsform für einen aktiven Wirkstoff, die eine einen Hohlraum definierende Wand, in der eine Ausgangsöffnung gebildet ist oder darin gebildet werden kann und von der mindestens ein Abschnitt semipermeabel ist, eine ausdehnbare Schicht, die sich in Entfernung zu der Ausgangsöffnung in dem Hohlraum befindet und in Fluidverbindung mit dem semipermeablen Abschnitt der Wand steht, eine verdichtete Medikamentschicht, die sich neben der Ausgangsöffnung in dem Hohlraum befindet und in direkter oder indirekter Kontaktbeziehung mit der ausdehnbaren Schicht steht, beinhaltet, **dadurch gekennzeichnet, dass** die verdichtete Medikamentschicht eine Vielzahl von Partikeln, die innere Poren aufweisen, sowie eine flüssige Formulierung eines aktiven Wirkstoffs in den Poren beinhaltet, wobei die Partikel verdichtbar sind und angepasst sind, um die flüssige Formulierung des aktiven Wirkstoffs während des Verdichtungsprozesses im Wesentlichen vollständig in den Poren zu halten.

2. Darreichungsform gemäß Anspruch 1, wobei die Partikel aus Kalziumhydrogenphosphat, Magnesiumaluminometasilikat, mikrokristalliner Zellulose oder Siliziumdioxid gebildet sind.

3. Darreichungsform gemäß Anspruch 2, wobei die Partikel aus Kalziumhydrogenphosphat der folgenden allgemeinen Formel gebildet sind:
CaHPO₄•mH₂O,
wobei m die Beziehung 0 ≤ m ≤ 2,0 erfüllt.

4. Darreichungsform gemäß Anspruch 3, wobei die Partikel durch Sprühtrocknen eines schuppenartigen Kalziumhydrogenphosphates mit einer spezifischen Oberfläche von 20 m²/g bis 60 m²/g, einem scheinbaren spezifischen Volumen von 1,5 ml/g oder größer, einem Ölabsorptionsvermögen von 0,7 ml/g oder höher, einer Primärpartikelgröße von 0,1 µm bis 5 µm und einer durchschnittlichen Partikelgröße von 2 µm bis 10 µm bei Sekundärpartikeln, die Aggregate der Primärpartikel sind, gebildet werden.

5. Darreichungsform gemäß Anspruch 3 oder 4, wobei die Partikel Kalziumhydrogenphosphat mit einem spezifischen Volumen von mindestens 1,5 ml/g, einer spezifischen BET-Fläche von mindestens 20 m²/g und einem Wasserabsorptionsvermögen von mindestens 0,7 ml/g sind.

6. Darreichungsform gemäß einem der Ansprüche 3 bis 5, wobei die Partikel eine Größenverteilung von 100 % kleiner als 40 Mesh, 50 % -100 % kleiner als 100 Mesh und 10 % - 60 % kleiner als 200 Mesh aufweisen.

7. Darreichungsform gemäß Anspruch 6, wobei die Partikel eine Größenverteilung aufweisen, bei der 100 % kleiner als 40 Mesh, 60 % - 90 % kleiner als 100 Mesh und 20 % - 60 % kleiner 200 Mesh sind.

8. Darreichungsform gemäß einem der vorhergehenden Ansprüche, wobei die Partikel eine Rohdichte von 0,4-0,6 g/ml, eine BET-Oberfläche von 30-50 m²/g, ein spezifisches Volumen von über 1,5 ml/g und eine mittlere Porengröße von mindestens 50 Angstroms aufweisen.

9. Darreichungsform gemäß Anspruch 3, wobei die Partikel Kalziumhydrogenphosphat mit einem spezifischen Rohvolumen von 1,5 ml/g - 5 ml/g, einer spezifischen BET-Fläche von 20 m²/g - 60 m²/g, einem Wasserabsorptionsvermögen von mindestens 0,7 ml/g und einer mittleren Porengröße von mindestens 70 Mikrometer sind.

10. Darreichungsform gemäß Anspruch 2, wobei die Partikel Magnesiumaluminometasilikat sind, dargestellt durch die allgemeine Formel
Al₂O₃MgO•2SiO₂•nH₂O,
wobei n die Beziehung 0 ≤ n ≤ 10 erfüllt.

11. Darreichungsform gemäß Anspruch 10, wobei das Silikat eine spezifische Oberfläche (Arca) von 100-300 m²/g, ein Ölabsorptionsvermögen von 1,3-3,4 ml/g, eine mittlere Porengröße von 1-2 Mikrometer, einen Schüttwinkel von 25° - 45°, eine Dichtezahl von 2 g/ml und ein spezifisches Volumen von 2,1-12 ml/g aufweist.

12. Darreichungsform gemäß Anspruch 10, wobei die Partikel Magnesiumaluminometasilikatpulver beinhalten.

13. Darreichungsform gemäß einem der Ansprüche 1 bis 11, wobei die Partikel aus einem Silikat wie in Anspruch 11 oder Anspruch 12 definiert, einem Kalziumhydrogenphosphat mit einer mittleren Porengröße in der Größenordnung von 70 Angstroms, einer mittleren Partikelgröße von 110 Mikrometer, einem spezifisches Volumen von 2 ml/g, einer spezifische BET-Oberfläche von 30-40 m²/g und einem Öl- und Wasserabsorptionsvermögen von 0,8 ml/g oder einer Kombination davon gebildet sind.

14. Darreichungsform gemäß einem der vorhergehenden Ansprüche, wobei die Darreichungsform einen pH-regulierenden Wirkstoff umfasst, der aus einem oder mehreren der Gruppe, die organische Säuren, anorganische Säuren und Basen beinhaltet, ausgewählt ist.

15. Darreichungsform gemäß einem der vorhergehenden Ansprüche, wobei die Darreichungsform einen Chelatbildner umfasst.

16. Darreichungsform gemäß einem der vorhergehenden Ansprüche, wobei die Partikel aus Kalziumhydrogenphosphat gebildet sind und die Darreichungsform eine organische Säure, einen Chelatbildner oder eine Kombination davon umfasst.

17. Darreichungsform gemäß einem der vorhergehenden Ansprüche, wobei das Gewichtsprozent einer flüssigen Formulierung eines aktiven Wirkstoffs mindestens 5 % des Gesamtgewichts der Darreichungsform ausmacht.

18. Darreichungsform gemäß einem der vorhergehenden Ansprüche, wobei der aktive Wirkstoff Sildenafilzitrat, Acetaminophen, Ibuprofen oder Ketoprofen ist.

19. Darreichungsform gemäß einem der vorhergehenden Ansprüche, wobei die Partikel angepasst sind, um in einem bioerodierbaren Träger dispergiert zu werden.

20. Darreichungsform gemäß einem der vorhergehenden Ansprüche, die eine Placeboschicht zwischen der Ausgangsöffnung und der Medikamentschicht aufweist.

21. Darreichungsform gemäß einem der vorhergehenden Ansprüche, die eine zwischen der inneren Oberfläche der Wand und mindestens der äußeren Oberfläche der Medikamentschicht, die sich in dem Hohlraum befindet, liegende durchflussfördernde Schicht aufweist.

22. Darreichungsform gemäß einem der vorhergehenden Ansprüche, die mindestens zwei Medikamentschichten aufweist, die durch mindestens eine inerte Schicht voneinander getrennt sind.

23. Darreichungsform gemäß einem der vorhergehenden Ansprüche, die mindestens zwei Medikamentschichten aufweist, wobei jede der Medikamentschichten einen unterschiedlichen aktiven Wirkstoff enthält.

24. Darreichungsform gemäß einem der vorhergehenden Ansprüche, wobei die flüssige Formulierung des aktiven Wirkstoffes der Medikamentschicht eine selbstemulgierende Formulierung beinhaltet.

25. Darreichungsform gemäß einem der vorhergehenden Ansprüche, wobei der flüssige aktive Wirkstoff der Medikamentschicht einen Absorptionsverstärker beinhaltet.

26. Darreichungsform gemäß einem der vorhergehenden Ansprüche, wobei die flüssige Formulierung des aktiven Wirkstoffes mindestens 30 Gewichts-% der Medikamentschicht beinhaltet.

27. Darreichungsform gemäß einem der vorhergehenden Ansprüche, die angepasst ist, um die flüssige Formulierung des aktiven Wirkstoffes bei Verabreichung an einen Patienten verzögert freizugeben.

28. Darreichungsform gemäß einem der Ansprüche 1 bis 26, die angepasst ist, um die flüssige Formulierung des aktiven Wirkstoffes bei Verabreichung an einen Patienten pulsatil freizugeben.

29. Darreichungsform gemäß einem der vorhergehenden Ansprüche, wobei die Darreichungsform eine einheitliche und orale Darreichungsform ist.

30. Eine Zusammensetzung, die eine flüssige Formulierung des aktiven Wirkstoffes in einer Vielzahl von porösen Partikeln sorbiert beinhaltet, wobei die Partikel so sind, wie in einem der Ansprüche 2 bis 19 definiert, und in einem bioerodierbaren Träger dispergiert sind, wobei die Partikel über einen Zeitraum von einigen Stunden bis zu 24 Stunden in die Anwendungsumgebung freigegeben werden.

31. Zusammensetzung gemäß Anspruch 30, wobei die Partikel durch Sprühtrocknen eines schuppenartigen Kalziumhydrogenphosphates mit einer spezifischen Oberfläche von 20 m²/g bis 60 m²/g, einem scheinbaren spezifischen Volumen von 1,5 ml/g oder größer, einem Ölabsorptionsvermögen von 0,7 ml/g oder höher, einer Primärpartikelgröße von 0,1 µm bis 5 µm und einer durchschnittlichen Partikelgröße von 2 µm bis 10 µm bei Sekundärpartikeln, die Aggregate der Primärpartikel sind, gebildet werden, wobei das schuppenartige Kalziumhydrogenphosphat durch die folgende allgemeine Formel dargestellt ist:
CaHPO₄•mH₂O,
wobei m die Beziehung 0 ≤ m ≤ 2,0 erfüllt.

32. Ein Verfahren zum Erleichtern der Freigabe eines aktiven Wirkstoffes aus einer Darreichungsform, das das Sorbieren einer flüssigen Formulierung des aktiven Wirkstoffes in eine Vielzahl poröser Partikel, wobei die Partikel in einem der Ansprüche 2 bis 19 definiert sind, und das Dispergieren der Partikel in einem bioerodierbaren Träger beinhaltet.

## Revendications

1. Une forme posologique pour un agent actif comportant une paroi définissant une cavité, la paroi ayant un orifice de sortie formé ou pouvant être formé à l'intérieur de celle-ci et au moins une portion de la paroi étant hémiperméable ; une couche dilatable située au sein de la cavité à distance de l'orifice de sortie et en communication de fluide avec la portion hémiperméable de la paroi ; une couche compactée médicamenteuse située au sein de la cavité adjacente à l'orifice de sortie et en relation de contact direct ou indirect avec la couche dilatable, **caractérisée en ce que** la couche médicamenteuse compactée comporte une pluralité de particules ayant des pores intérieurs et une formulation d'agent actif liquide dans les pores, les particules pouvant être compactées et étant adaptées pour retenir substantiellement toute la formulation d'agent actif liquide au sein des pores durant le processus de compactage.

2. Une forme posologique telle que revendiquée dans la revendication 1 dans laquelle les particules sont formées à partir de phosphate dicalcique, de méta aluminosilicate de magnésium, de cellulose microcristalline ou de dioxyde de silicium.

3. Une forme posologique telle que revendiquée dans la revendication 2 dans laquelle les particules sont formées à partir de phosphate dicalcique de formule générale suivante :
CaHPO₄•mH₂O
dans laquelle m satisfait la relation 0 ≤ m ≤ 2,0.

4. Une forme posologique telle que revendiquée dans la revendication 3 dans laquelle les particules sont formées par séchage par pulvérisation d'un phosphate dicalcique écailleux ayant une surface spécifique de 20 m²/g à 60 m²/g, un volume spécifique apparent de 1,5 ml/g ou plus, une capacité d'absorption d'huile de 0,7 ml/g ou plus, une taille de particule primaire de 0,1 µ à 5 µ, et une taille de particule moyenne de 2 µ à 10 µ parmi les particules secondaires qui sont des agrégats des particules primaires.

5. Une forme posologique telle que revendiquée dans la revendication 3 ou la revendication 4 dans laquelle les particules sont du phosphate dicalcique ayant un volume spécifique d'au moins 1,5 ml/g, une surface spécifique BET d'au moins 20 m²/g, et une capacité d'absorption d'eau d'au moins 0,7 ml/g.

6. Une forme posologique telle que revendiquée dans n'importe laquelle des revendications 3 à 5 dans laquelle les particules ayant une répartition de taille 100 % inférieures à une maille 40, de 50 % à 100 % inférieures à une maille 100 et de 10 % à 60 % inférieures à une maille 200.

7. Une forme posologique telle que revendiquée dans la revendication 6 dans laquelle les particules ont une répartition de taille où 100 % sont inférieures à une maille 40, de 60 % à 90 % sont inférieures à une maille 100 et de 20 % à 60 % sont inférieures à une maille 200.

8. Une forme posologique telle que revendiquée dans n'importe laquelle des revendications précédentes dans laquelle les particules ont une densité apparente de 0,4 à 0,6 g/ml, une surface BET de 30 à 50 m²/g, un volume spécifique de plus de 1,5 ml/g, et une taille de pore moyenne d'au moins 50 Angströms.

9. Une forme posologique telle que revendiquée dans la revendication 3 dans laquelle les particules sont du phosphate dicalcique ayant un volume spécifique apparent de 1,5 ml/g à 5 ml/g, une surface spécifique BET de 20 m²/g à 60 m²/g, une capacité d'absorption d'eau d'au moins 0,7 ml/g, et une taille de particule moyenne d'au moins 70 microns.

10. Une forme posologique telle que revendiquée dans la revendication 2 dans laquelle les particules sont du méta aluminosilicate de magnésium représenté par la formule générale
Al₂O₃MgO•2SiO₂•nH₂O
dans laquelle n satisfait la relation 0 ≤ n ≤ 10.

11. Une forme posologique telle que revendiquée dans la revendication 10 dans laquelle le silicate a une surface spécifique (arca) de 100 à 300 m²/g, une capacité d'absorption d'huile de 1,3 à 3,4 ml/g, une taille de particule moyenne de 1 à 2 microns, un angle de repos de 25° à 45°, un poids spécifique de 2 g/ml et un volume spécifique de 2,1 à 12 ml/g.

12. Une forme posologique telle que revendiquée dans la revendication 10 dans laquelle les particules comportent de la poudre de méta aluminosilicate de magnésium.

13. Une forme posologique telle que revendiquée dans n'importe laquelle des revendications 1 à 11 dans laquelle les particules sont formées à partir d'un silicate tel que défini dans la revendication 11 ou la revendication 12, un phosphate dicalcique ayant une taille de pore moyenne de l'ordre de 70 Angströms, une taille de particule moyenne de 110 microns, un volume spécifique de 2 ml/g, une surface spécifique BET de 30 à 40 m²/g, et une capacité d'absorption d'huile et d'eau de 0,8 ml/g, ou une combinaison de ceux-ci.

14. Une forme posologique telle que revendiquée dans n'importe laquelle des revendications précédentes dans laquelle la forme posologique comprend un agent de régulation du pH sélectionné parmi un ou plusieurs éléments du groupe comportant des acides organiques, des acides inorganiques et des bases.

15. Une forme posologique telle que revendiquée dans n'importe laquelle des revendications précédentes dans laquelle la forme posologique comprend un agent de chélation.

16. Une forme posologique telle que revendiquée dans n'importe laquelle des revendications précédentes dans laquelle les particules sont formées à partir de phosphate dicalcique et la forme posologique comprend un acide organique, un agent de chélation, ou une combinaison de ceux-ci.

17. Une forme posologique telle que revendiquée dans n'importe laquelle des revendications précédentes dans laquelle le pourcentage en poids de la formulation d'agent actif liquide est au moins 5 % du poids total de la forme posologique.

18. Une forme posologique telle que revendiquée dans n'importe laquelle des revendications précédentes dans laquelle l'agent actif est du citrate de sildénafil, du paracétamol, de l'ibuprofène ou du kétoprofène.

19. Une forme posologique telle que revendiquée dans n'importe laquelle des revendications précédentes dans laquelle les particules sont adaptées pour être dispersées dans un support érodable d'un point de vue biologique.

20. Une forme posologique telle que revendiquée dans n'importe laquelle des revendications précédentes ayant une couche placebo entre l'orifice de sortie et la couche médicamenteuse.

21. Une forme posologique telle que revendiquée dans n'importe laquelle des revendications précédentes ayant une couche favorisant l'écoulement interposée entre la surface interne de la paroi et au moins la surface externe de la couche médicamenteuse située au sein de la cavité.

22. Une forme posologique telle que revendiquée dans n'importe laquelle des revendications précédentes, ayant au moins deux couches médicamenteuses séparées par au moins une couche inerte.

23. Une forme posologique telle que revendiquée dans n'importe laquelle des revendications précédentes ayant au moins deux couches médicamenteuses, chacune desdites couches médicamenteuses contenant un agent actif différent.

24. Une forme posologique telle que revendiquée dans n'importe laquelle des revendications précédentes dans laquelle la formulation d'agent actif liquide de la couche médicamenteuse comporte une formulation auto-émulsifiante.

25. Une forme posologique telle que revendiquée dans n'importe laquelle des revendications précédentes dans laquelle l'agent actif liquide de la couche médicamenteuse comporte un stimulateur d'absorption.

26. Une forme posologique telle que revendiquée dans n'importe laquelle des revendications précédentes dans laquelle la formulation d'agent actif liquide constitue au moins 30 % en poids de la couche médicamenteuse.

27. Une forme posologique telle que revendiquée dans n'importe laquelle des revendications précédentes adaptée pour la libération prolongée de la formulation d'agent actif liquide lorsqu'elle est administrée à un sujet.

28. Une forme posologique telle que revendiquée dans n'importe laquelle des revendications 1 à 26 adaptée pour la libération pulsatile de la formulation d'agent actif liquide lorsqu'elle est administrée à un sujet.

29. Une forme posologique telle que revendiquée dans n'importe laquelle des revendications précédentes dans laquelle la forme posologique est une forme posologique orale et unitaire.

30. Une composition comportant une formulation liquide de l'agent actif sorbée dans une pluralité de particules poreuses, les particules étant tel que défini dans n'importe laquelle des revendications 2 à 19, et dispersée dans tout un support érodable d'un point de vue biologique, les particules étant libérées dans l'environnement d'utilisation sur une période de temps allant de quelques heures à 24 heures.

31. Une composition telle que revendiquée dans la revendication 30 dans laquelle les particules sont formées par séchage par pulvérisation de phosphate dicalcique écailleux ayant une surface spécifique de 20 m²/g à 60 m²/g, un volume spécifique apparent de 1,5 ml/g ou plus, une capacité d'absorption d'huile de 0,7 ml/g ou plus, une taille de particule primaire de 0,1 µ à 5 µ, et une taille de particule moyenne de 2 µ à 10 µ parmi les particules secondaires qui sont des agrégats des particules primaires, le phosphate dicalcique écailleux étant représenté par la formule générale suivante :
CaHPO₄•mH₂O
dans laquelle m satisfait la relation 0 ≤ m ≤ 2,0.

32. Un procédé pour faciliter la libération d'un agent actif d'une forme posologique comportant la sorption d'une formulation liquide de l'agent actif dans une pluralité de particules poreuses, les particules étant définies dans n'importe laquelle des revendications 2 à 19, et la dispersion des particules dans tout un support érodable d'un point de vue biologique.
